# EUROPEAN PATENT SPECIFICATION

(11) **EP 4 442 692 B1**
(45) Date of publication and mention of the grant of the patent: **29.07.2026**
(21) Application number: 22897783.1
(22) Date of filing: 22.11.2022
(51) Int. Cl.: C07D 513/04, A61K 31/429, A61K 31/433, A61P 1/16, A61P 3/04, A61P 3/06, A61P 3/10, A61P 35/00

(54) **IMIDAZOTHIAZOLE DERIVATIVE, PREPARATION METHOD THEREFOR, AND APPLICATION THEREOF**
IMIDAZOTHIAZOLDERIVAT, HERSTELLUNGSVERFAHREN DAFÜR UND ANWENDUNG DAVON
DÉRIVÉ D'IMIDAZOTHIAZOLE, SON PROCÉDÉ DE PRÉPARATION ET SON UTILISATION

(30) Priority: 29.11.2021 CN 202111435801
(43) Date of publication of application: 09.10.2024
(73) Proprietor: Ocean University of China, Qingdao, Shandong 266003 (CN)
(72) Inventor: JIANG, Tao, Qingdao, Shandong 266003 (CN); JIN, Xin, Qingdao, Shandong 266003 (CN); WEI, Xianfeng, Qingdao, Shandong 266003 (CN); QIU, Tingting, Qingdao, Shandong 266003 (CN)
(74) Representative: Lavoix
(86) International application number: PCT/CN2022/133420
(87) International publication number: WO 2023/093700

(56) References cited:
- WO-A1-2011/121317
- WO-A1-2017/189613
- WO-A1-2022/053838
- CN-A- 1 052 117
- CN-A- 101 878 219
- CN-A- 104 350 055
- CN-A- 105 492 008
- CN-A- 109 020 957
- CN-A- 109 415 359
- CN-A- 111 978 317
- US-A- 4 265 898

## Description

### TECHNICAL FIELD

The invention belongs to the field of medicinal chemistry, in particular to Imidazothiazole derivatives and a preparation method and application thereof.

### BACKGROUND

Metabolic syndrome is a group of clinical syndromes determined by genetic and environmental factors and characterized by the co-occurrence of multiple metabolic diseases such as obesity and type 2 diabetes mellitus (T2D), which can further lead to dyslipidemia, hypertension, non-alcoholic fatty liver disease, and gout. With the development of social economy and the change of people's lifestyle (increase in energy intake and decrease in exercise, etc.), the incidence of metabolic syndrome has shown an increasing trend globally year by year, and has become a global public health challenge.

Nonalcoholic fatty liver disease (NAFLD) is a genetic, environmental, metabolic and stress related liver disease characterized by hepatic parenchymal cell steatosis and fat storage. Clinically, there are three types: simple fatty liver, steatohepatitis (NASH) and fatty cirrhosis.

MNK (Human mitogenic protein kinase interacting enzyme), including two subtypes MNK1 and MNK2. MNK has been shown to phosphorylate elF4E in vivo, thereby regulating protein synthesis in organisms. Previous studies have found that regulating MNK activity can regulate body weight, glucose tolerance, enhance insulin sensitivity, energy consumption capacity, liver fat accumulation and inflammation in animal fat, suggesting that MNK can be used as a potential drug development target for metabolic diseases such as diabetes, obesity and NAFLD.

There is an urgent need for more types of small molecule compounds that can better inhibit MNK activity in this field.

### SUMMARY OF THE INVENTION

In order to solve the above technical problems, we have invented a series of imidazothiazole or imidazothiadiazole derivative compounds, which can regulate blood sugar in the body, reduce weight gain, reduce fat accumulation and other changes by regulating the activity of MNK protease, thereby improving the condition of patients with diabetes and other metabolic diseases.

The present invention provides an imidazothiazole derivative, a stereoisomer, a tautomer, a geometric isomer, or a pharmaceutically acceptable salt thereof, wherein the imidazothiazole derivative has a structure of formula (I) : 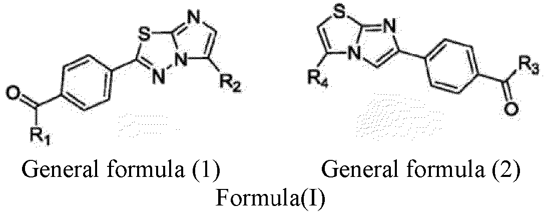 formula (I) comprises general formula (1) and general formula (2), R₁ and R₃are each independently selected from a 5 to 6 membered heterocyclyl containing 1 to 2 nitrogen atoms optionally substituted with one or more groups of C1-C6 alkyl, C1-C6 haloalkyl, C1-C6 alkylamino, C1-C6 alkoxy, 5 to 6 membered heterocyclic ring, halogen, hydroxyl, cyano, nitro, amino, and carbonyl, etc, or R₁ is selected from C1-C6 alkoxy optionally substituted with one or more of hydroxyl, halogen, amino, dimethylamino, and 5 to 6 membered heterocyclic ring, R₁ is selected form C1-C6 alkylthio optionally substituted with one or more of hydroxyl, halogen, amino, dimethylamino, and 5 to 6 membered heterocyclic ring, R₁ is selected from C1-C6 alkylamino optionally substituted with one or more of hydroxyl, halogen, amino, dimethylamino, 5 to 6 membered heterocyclic rings, R₂ and R₄ are each independently selected from aryl or heteroaryl that are optionally substituted with one or more groups of C1-C6 alkyl, C1-C6 haloalkyl, C1-C6 alkylamino, C1-C6 alkoxy, C1-C6 alkoxycarbonyl, C1-C6 hydroxyalkyl, halogen, hydroxyl, cyano, nitro, and amino and carbonyl and the like.

As described in the present invention, the C1-C6 alkyl refers to a straight or branched chain saturated monovalent hydrocarbon group containing 1, 2, 3, 4, 5 or 6 carbon atoms, and representative examples include but are not limited to methyl, ethyl, propyl, isopropyl, butyl, isobutyl, sec-butyl, tert-butyl, etc. C1-C6 alkyl can further preferably be a C1-C3 alkyl.

As described in the present invention, the C1-C6 haloalkyl refers to one or more hydrogen atoms in the "C1-C6 alkyl" as defined above is substituted with the same or different halogen atoms. C1-C6 haloalkyl can further preferably be a C1-C3 haloalkyl. Representative examples include, but are not limited to, trifluoromethyl, difluoromethyl, monofluoromethyl, trichloromethyl, 1,1,1-trifluoroethyl, pentafluoroethyl, etc.

As described in the present invention, the C1-C6 alkylamino refers to one or two hydrogen atoms in amino (-NH₂) are substituted with the same or different above-defined "C1-C6 alkyl"; i.e., it can be referred as -NR¹R², wherein R¹ and R² are each independently selected from H and C1-C6 alkyl, and R¹ and R² cannot be H at the same time.

As described in the present invention, the C1-C6 alkoxy refers to -OR³, wherein R³ is selected from C1-C6 alkyl; C1-C6 alkoxy can further preferably be C1-C3 alkoxy, and more further preferably be methoxy and ethoxy.

As described in the present invention, the C1-C6 alkoxycarbonyl refers to -C(O)R⁴, wherein R⁴ is selected from C1-C6 alkoxy.

As described in the present invention, the 5 to 6-membered heterocyclic ring refers to a ring system containing ring carbon atoms and 1 to 4 ring heteroatoms (preferably 1, 2 or 3 ring heteroatoms), wherein each ring heteroatom is independently selected from nitrogen, oxygen and sulfur; in heterocyclic groups containing one or more nitrogen atoms, the attachment site can be either carbon or nitrogen atoms, as long as the valence allows. Further preferred is piperazine ring, morpholine ring, piperidine ring, hexahydropyran ring, tetrahydrofuran ring, tetrahydrothiophene ring, pyrrole ring, tetrahydropyrrole ring and so on.

As described in the present invention, the aryl is preferably a monocyclic or polycyclic aryl containing 6-12 carbon atoms, preferably is phenyl, naphthalenyl, etc.; heteraryl is preferably 5 to 6-membered heteraryl, 5 to 6-membered heteraryl refers to a heteraryl system containg ring carbon atoms and 1 to 4 ring heteroatoms (preferably 1, 2, or 3 ring heteroatoms), wherein each ring heteroatom is independently selected from nitrogen, oxygen, sulfur. Further preferred is furanyl, thiophenyl, pyridinyl, thiazolyl, imidazolyl and so on.

As described in the present invention, the halogen atom or halogen is preferably fluorine, chlorine, bromine or iodine.

The above-mentioned imidazothiazole derivative having a structure of formula (I) is preferably compounds 1 to 55 or a stereoisomer, tautomer, geometric isomer or a pharmaceutically acceptable salt thereof,

| Number | Structure | Number | Structure |
|---|---|---|---|
| 1 | 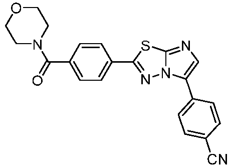 | 29 | 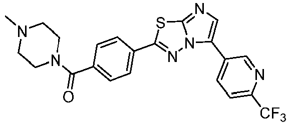 |
| 2 | 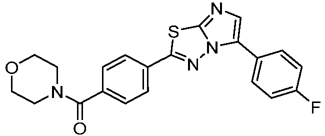 | 30 | 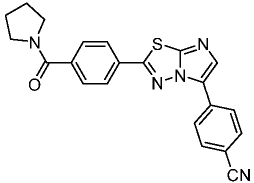 |
| 3 | 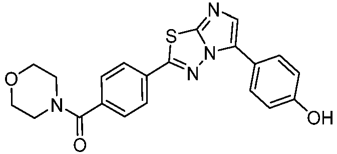 | 31 | 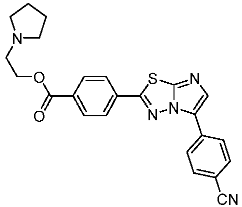 |
| 4 | 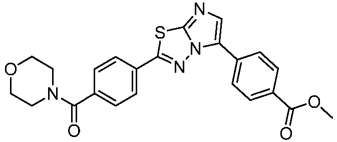 | 32 | 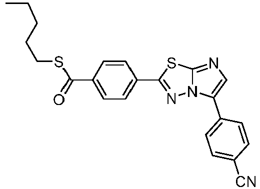 |
| 5 | 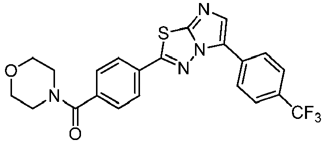 | 33 | 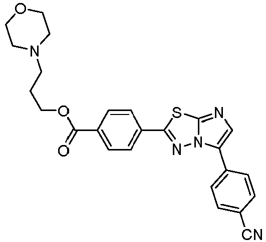 |
| 6 | 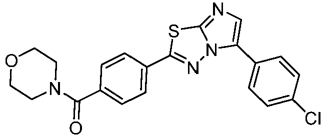 | 34 | 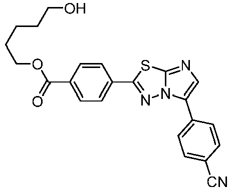 |
| 7 | 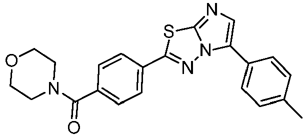 | 35 | 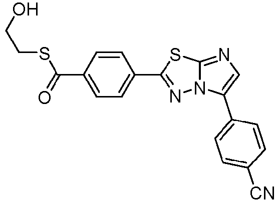 |
| 8 | 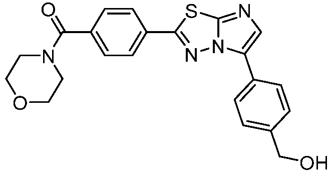 | 36 | 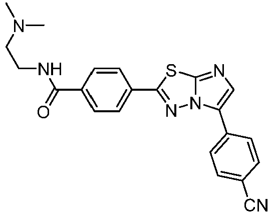 |
| 9 | 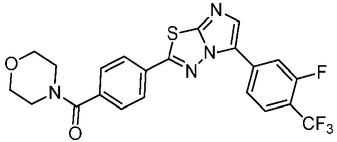 | 37 | 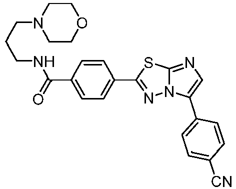 |
| 10 | 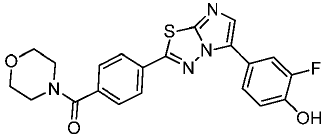 | 38 | 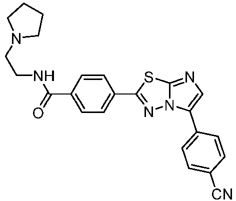 |
| 11 | 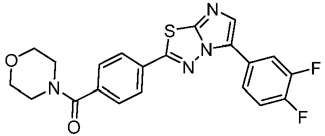 | 39 | 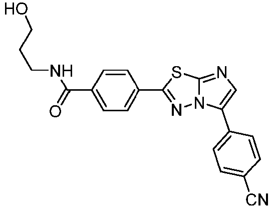 |
| 12 | 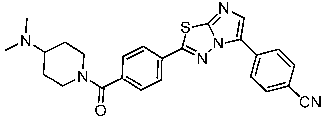 | 40 | 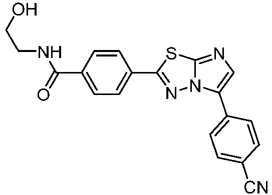 |
| 13 | 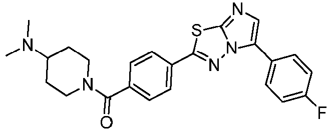 | 41 | 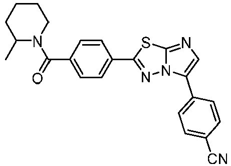 |
| 14 | 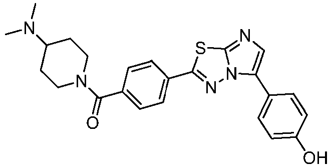 | 42 | 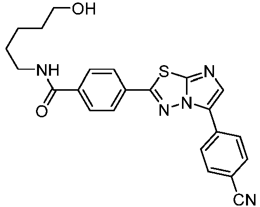 |
| 15 | 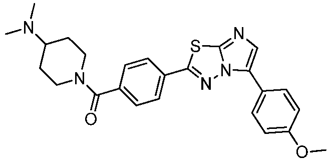 | 43 | 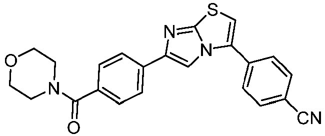 |
| 16 | 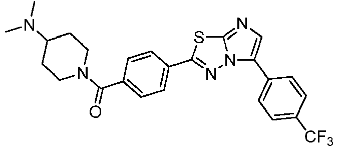 | 44 | 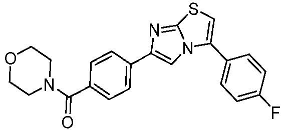 |
| 17 | 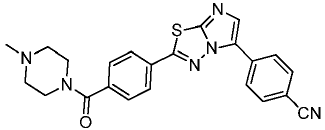 | 45 | 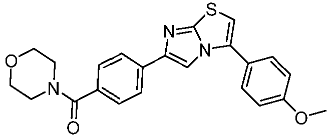 |
| 18 | 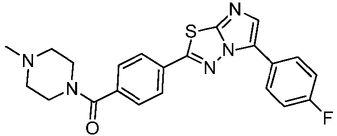 | 46 | 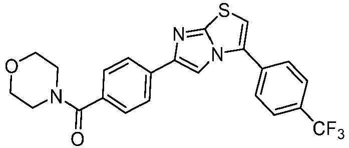 |
| 19 | 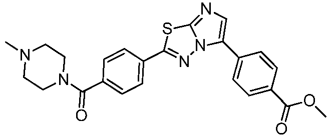 | 47 | 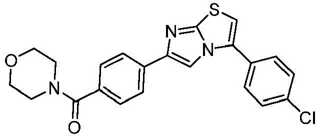 |
| 20 | 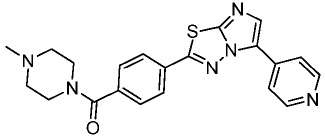 | 48 | 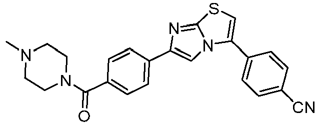 |
| 21 | 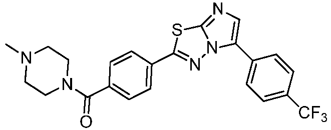 | 49 | 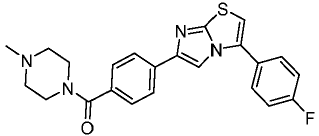 |
| 22 | 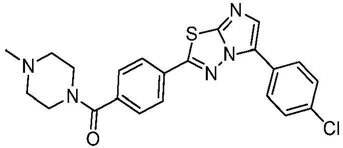 | 50 | 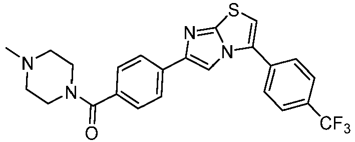 |
| 23 | 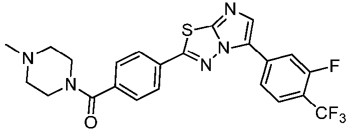 | 51 | 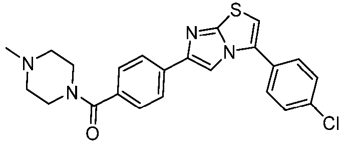 |
| 24 | 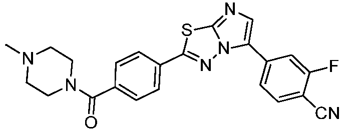 | 52 | 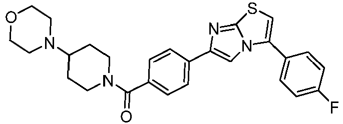 |
| 25 | 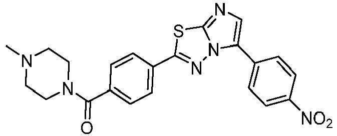 | 53 | 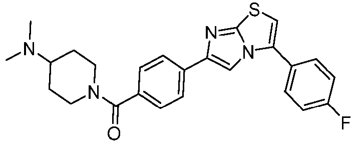 |
| 26 | 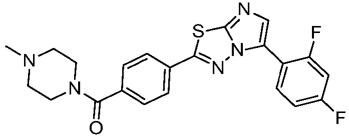 | 54 | 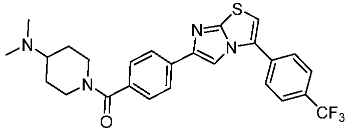 |
| 27 | 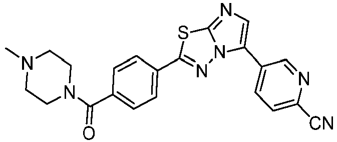 | 55 | 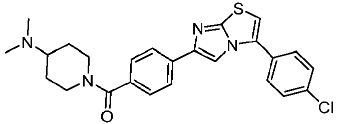 |
| 28 | 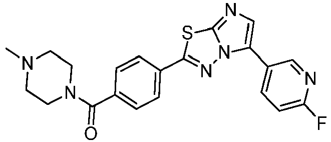 | | |

Another embodiment of the present invention provides a method for preparing the above imidazothiazole derivative having the structure of formula (I), wherein comprising the following steps:
synthesis method of general formula (1): 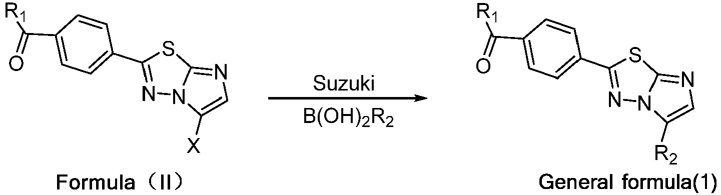
making a compound of formula (II) and a corresponding boronic acid derivative (B(OH)₂R₂) undergo Suzuki condensation reaction, thereby obtaining the compound of general form (1), wherein R₁ and R_{2 are} defined as above, X is a halogen, preferably is chlorine, bromine, or iodine;
synthesis method of general formula (2): 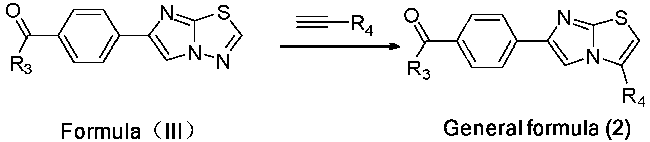
reacting a compound of formula (III) with a corresponding acetylene derivative ( 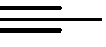 R₄) under alkaline conditions, thereby obtaining the compound of general formula (2), wherein R₃ and R_{4 are} defined as above.

Another embodiment of the present invention provides a method for preparing the above imidazothiazole derivative having the structure of formula (I), wherein comprising the following steps:
the synthesis method of general formula (1) comprises a step for preparing a compound of formula (II) from a compound of formula (IV) : 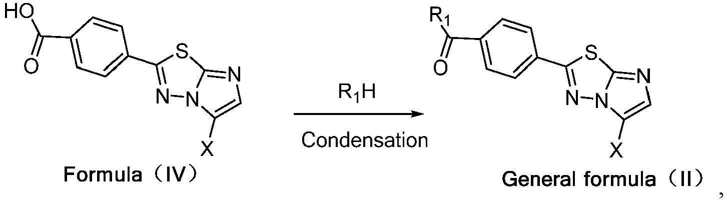 wherein R1 is defined as above, X is halogen, preferably chlorine, bromine, or iodine;
the synthesis method of general formula (2) comprises a step for preparing a compound of formula (III) from a compound of formula (V) : 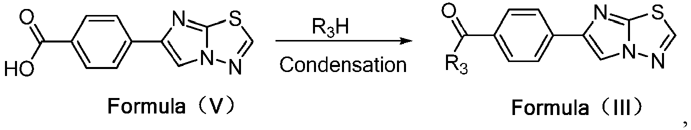 wherein R₃ is defined as above.

Another embodiment of the present invention provides a method for preparing the above imidazothiazole derivative having the structure of formula (I), wherein the imidazothiazole derivative having the structure of formula (I) is selected from general formula (1) wherein R₂ is 4-cyanophenyl, and is represented by compound N, comprising the following steps: 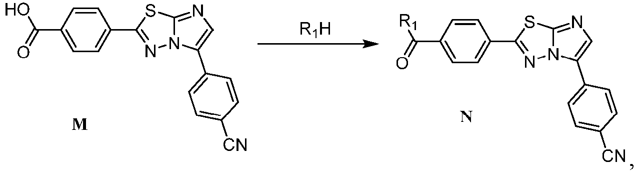 making compound M undergo a condensation reaction with R₁H, thereby obtaining compound N, and the definition of R₁ is the same as above.

Another embodiment of the present invention provides an intermediate for the preparation of the imidazothiazole derivative having the structure of formula (I), wherein the intermediate has a structure of formula (II) : 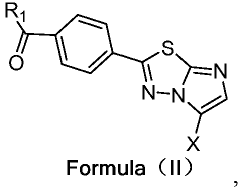 wherein the definition of R₁ is the same as above, and X is halogen, preferably is chlorine, bromine or iodine.

Another embodiment of the present invention provides an intermediate for the preparation of the imidazothiazole derivative having the structure of formula (I), wherein the intermediate has a structure of formula (III) : 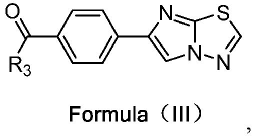 wherein the definition of R3 is the same as above.

Another embodiment of the present invention provides an intermediate for the preparation of formula (II) wherein the intermediate has a structure of formula (IV) : 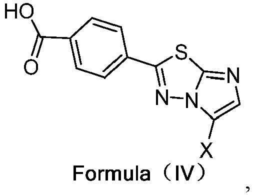 wherein X is a halogen, preferably is chlorine, bromine or iodine.

Another embodiment of the invention provides an intermediate for the preparation of formula (III) wherein the intermediate has a structure of formula (V):

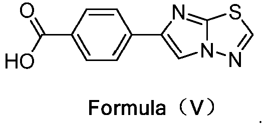

Another embodiment of the present invention provides an intermediate for the preparation of the imidazothiazole derivative having the structure of formula (I), wherein the intermediate has the following structure:

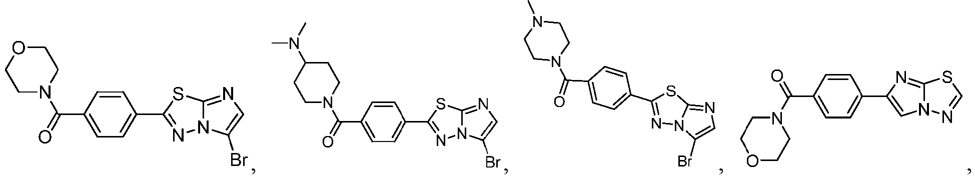

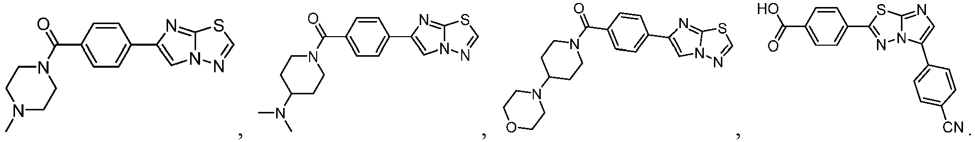

Another embodiment of the present invention provides a use of compounds of formula (II), formula (III), formula (IV), and formula (V) in preparing the imidazothiazole derivative having the structure of formula (I).

Another embodiment of the present invention provides a use of the imidazothiazole derivative having the structure of formula (I), the stereoisomer, tautomer, or geometric isomer thereof, or the pharmaceutically acceptable salt thereof in inhibiting kinase activity of MNK1 or MNK2 or variants thereof; or in the preparation of a drug for preventing and/or treating cancers caused by abnormal levels of MNK1 and/or MNK2.

Another embodiment of the present invention provides a use of the imidazothiazole derivative having the structure of formula (I), the stereoisomer, tautomer, or geometric isomer thereof, or the pharmaceutically acceptable salt thereof in the preparation of a drug for preventing and/or treating metabolic diseases associated with MNK activity. The metabolic diseases associated with MNK activity are selected from type 1 diabetes mellitus, type 2 diabetes mellitus, hyperlipidemia, obesity, fatty liver disease, and complications thereof and related disorders thereof.

Another embodiment of the present invention provides a use of the imidazothiazole derivative having the structure of formula (I), the stereoisomer, tautomer, or geometric isomer thereof, or the pharmaceutically acceptable salt thereof in the preparation of MNK1 and/or MNK2 inhibitors.

Another embodiment of the present invention provides a pharmaceutical composition wherein the pharmaceutical composition use the imidazothiazole derivative having the structure of formula (I), the stereoisomer, the tautomer, or the geometric isomer thereof, or the pharmaceutically acceptable salt thereof as an active ingredient. The pharmaceutical composition may also comprise pharmaceutically acceptable excipients. The pharmaceutical composition may also include other MNK1 and/or MNK2 inhibitors (marketed therapeutics). The dosage form thereof can be solid preparation, liquid preparation or semi-solid preparation, preferably tablet, capsule, injection and so on.

Methods and techniques of the present invention are generally implemented according to traditional methods known in the art, unless otherwise indicated. The terms described in the invention are named according to chemistry, biology and pharmacology, and the experimental methods and techniques are known and commonly used in the field. Standard techniques are used in chemosynthesis, chemical analysis, preparations and formulations for medicine, and the treatment for patients. Unless otherwise stated, the scientific and technical terms used in the present invention shall have those meanings commonly understood by those skilled in the art. While the following terms have the following definitions:
Unless otherwise specified, all compounds present in the present invention are intended to include all possible optical isomers, such as single chiral compounds or mixtures of various chiral compounds (i.e., racemes). Among all compounds of the present invention, each chiral carbon atom may optionally be in R configuration or S configuration, or a mixture of the two configurations.

Diabetes mellitus and its complications are specifically described as impaired glucose tolerance, diabetic gangrene, diabetic arthropathy, diabetic osteopenia, diabetic glomerulosclerosis, diabetic nephropathy, diabetic dermatopathy, diabetic neuropathy, diabetic cataract, diabetic retinopathy, diabetic macular degeneration, diabetic foot syndrome, diabetic coma, diabetic hyperosmolar coma, hypoglycemic coma, hyperglycemic coma, diabetic acidosis, diabetic ketoacidosis, intracapillary glomerular nephropathy, diabetic muscular atrophy, diabetic autonomic neuropathy, diabetic mononeuropathy, diabetic polyneuropathy, diabetic vascular disease, diabetic peripheral vascular disease, diabetic ulcer, diabetic arthropathy, diabetic obesity.

Hyperlipidemia and its complications are: hypercholesterolemia, familial hypercholesterolemia, Verde's hyperlipoproteinemia, hyperβ-lipoproteinemia, hyperlipidemia, low density lipoproteinemia, pure hypertriglyceridemia, endogenous hypertriglyceridemia, simple hypercholesterolemia, simple hypertriglyceridemia, cardiovascular disease. Wherein cardiovascular diseases include: hypertension, ischemia, varicose veins, retinal vein occlusion, atherosclerosis, angina pectoris, myocardial infarction, stenocardia, pulmonary hypertension, congestive heart failure, glomerular disease, tubular interstitial disorders of the kidney, renal failure, vascular stenosis or cerebrovascular disease (stroke).

Fatty liver disease includes, but is not limited to, non-alcoholic fatty liver disease (NAFLD), non-alcoholic steatohepatitis (NASH), and the resulting chronic inflammation leading to progressive fibrosis, cirrhosis, etc.

### DESCRIPTION OF THE DRAWINGS

Figure 1 shows the changes of blood sugar in each group of mice in the experiment.
Figure 2 shows the changes of glucose tolerance and insulin tolerance in each administration group.
Figure 3 is an example diagram of the changes of serum related indexes in each administration group.
Figure 4 is an example of the insulin tolerance test of each group in db/db mice.
Figure 5 is an example diagram of the changes of various related indexes of liver function in db/db mice.
Figure 6 is an example of the changes of various relevant indexes in the serum of db/db mice.

### EMBODIMENTS FOR CARRYING OUT THE INVENTION

### General method of synthesis of compounds

### Synthesis method of general formula (1):

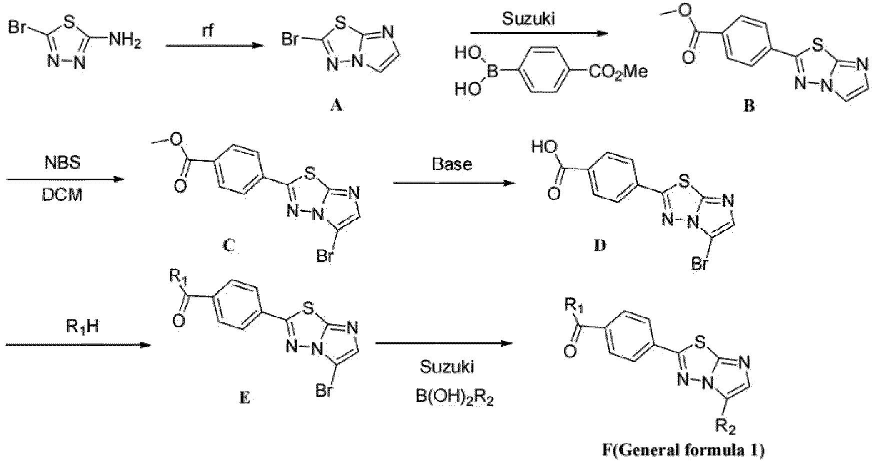

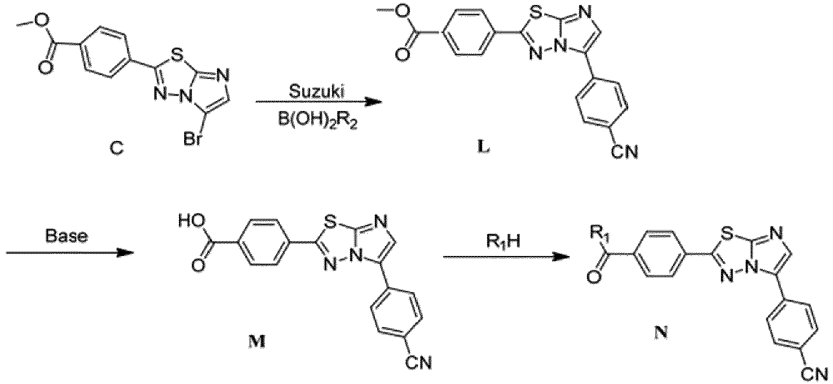

Using commercially available 5-bromo-2-amino-1,3, 4-thiadiazole as raw material, chloracetaldehyde or 2-bromo-1, 1-diethoxyethane was reflow in alcohol solution (such as ethanol, n-butanol, etc.) for 1-2 days to obtain cycloidal product A, and then underwent Suzuki condensation reaction with 4-methyl phenylboronic acid to obtain compound B. C is obtained by nucleophilic substitution reaction with NBS(or NIS) in dichloromethane solution, and is hydrolyzed under alkaline conditions (e.g., in lithium hydroxide, sodium hydroxide, or potassium hydroxide aqueous solution, which may be a mixture of water, tetrahydrofuran, and alcohols), and then adjusted to acid (acid can be hydrochloric acid, sulfuric acid, acetic acid, hydrochloric acid). The carboxylic acid structure of compound D is obtained, and then amidation with amino compound R₁H under the condition of amide condensation reagent (such as EDCI and NHS, EDCI and Hobt or EDCI and Hoat combination) is performed to obtain compound E. Finally, the Suzuki condensation reaction with the boronic acid compound B(OH)₂R₂ results in the final compound F (general formula 1), where R₁ and R₂ are defined as before.

The intermediate C obtained from the above reaction undergoes Suzuki condensation reaction with 4-cyanophenylboronic acid to obtain compound L, which is hydrolyzed under alkaline conditions (for example, in an aqueous solution of lithium hydroxide, sodium hydroxide, or potassium hydroxide, the solution can be a mixture of water, tetrahydrofuran, and alcohol), and then adjusted to acid (acid can be hydrochloric acid, sulfuric acid, acetic acid, hydrochloric acid). Aqueous solution such as formic acid) to obtain carboxylic acid structure compound M, and then under the condition of condensation reagent (such as EDCI and NHS, EDCI and Hobt or EDCI and Hoat combination) and R₁H (amine compound, alcohol compound, sulfhydryl compound) condensation reaction to obtain the final compound N (R₂ is the general formula 1 of 4-cyanophenyl). R₁ is defined as above.

### Synthesis method of general formula 2:

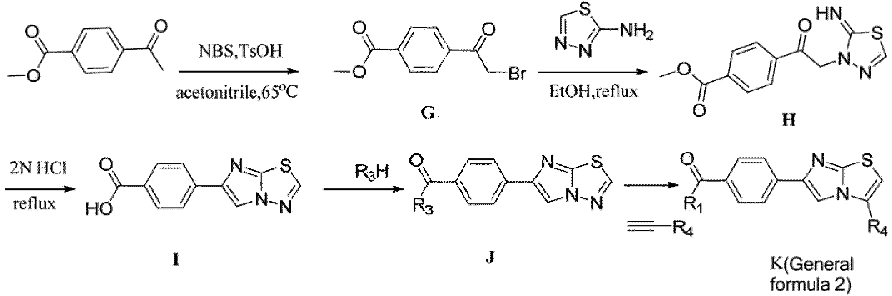

compound G is obtained by reflux reaction with NBS in p-toluenesulfonic acid and acetonitrile solution, followed by reflux reaction with 2-amino-1,3, 4-thiadiazole in alcohol solution for 6h, and compound H is obtained under alkaline conditions (for example, in aqueous solution of lithium hydroxide, sodium hydroxide, or potassium hydroxide, the solution may be water). Tetrahydrofuran, in a mixed solution of alcohols, is hydrolyzed, and after acid conditioning (acid can be hydrochloric acid, sulfuric acid, acetic acid, formic acid and other aqueous solutions), carboxylic acid structure compound I is obtained, and then under the conditions of amide condensation reagents (such as EDCI and NHS), EDCI and Hobt or a combination of EDCI and Hoat) amides with amino compound R₃H to obtain compound J. Finally, potassium tert-butanol reacts with phenylacetylene compound R₄CCH at room temperature for 6h in DMF solution to obtain compound K (general formula 2), wherein R₃ and R₄ refer to the above claims.

### EMBODIMENTS FOR CARRYING OUT THE INVENTION

The following are detailed descriptions of the invention through specific preparation examples and embodiments, but the use and purpose of these exemplary embodiments are only used to illustrate the invention, and do not constitute any limitation of the actual scope of protection of the invention in any form, let alone limit the scope of protection of the invention to this.

### Example 1: 2-bromoimidazo[2,1-b][1,3,4]thiadiazole (compound A)

compound A was obtained by reflux reaction of 5-bromo-2-amino-1,3, 4-thiadiazole with chloracetaldehyde in ethanol with a yield of 17.6%, ¹H NMR (500 MHz, CDCl₃) *δ* 7.76 (s, 1H), 7.36 (s, 1H).

### Example 2: methyl 4-(imidazo[2,1-b][1,3,4]thiadiazol-2-yl)benzoate (compound B)

2-bromoimidazo[2,1-b][1,3,4] thiadiazole (compound A) 300 mg (1.47 mmol) and methyl 4-formate phenylboronic acid 317 mg (1.76 mmol) under the action of catalyst, Silica gel column chromatography (PE:EA=10:1) was used to obtain a white solid of 93.2 mg (compound B) with a yield of 24.3% after Suzuki condensation reaction. ¹H NMR (500 MHz, CDCl3)*δ* 8.19-8.15 (m, 2H), 7.97-7.93 (m, 2H), 7.81 (d, *J =* 1.4Hz, 1H), 7.37 (d, *J =* 1.4Hz, 1H), 3.97 (s, 3H).

### Example 3: methyl 4-(5-bromoimidazo[2,1-b][1,3,4]thiadiazol-2-yl)benzoate (compound C)

After the reaction of 4-(imidazole [2,1-b][1,3,4] thiadiazole) methyl benzoate 350 mg (1.35 mmol) with 286 mg (1.62 mmol) n-bromosuccinimide, 340 mg (compound C) of light green solid was obtained by silica gel column chromatography, with a yield of 74.7%. ¹H NMR (500 MHz, CDCl₃)*δ*8.18 (d, *J =* 8.4Hz, 2H), 7.99 (d, *J =* 8.4Hz, 2H), 7.29 (s, 1H), 3.98 (s, 3H). ¹³C NMR (125 MHz, CDCl₃) *δ* 165.9, 161.5, 144.2, 133.8, 133.7, 133.1, 130.5, 126.9, 96.3, 52.5.

### Example 4: 4-(5-bromoimidazo[2,1-b][1,3,4] thiadiazole-2-yl) benzoic acid (compound D)

Take 4-(5-bromoimidazo[2,1-b][1,3,4] thiadiazole-2-methyl benzoate 340 mg (1 mmol), 424 mg (10 mmol) lithium hydroxide monohydrate mixed in 30 mL of THF/H₂O (V:V=1:1) solution, At the end of the reaction, 320 mg of white solid was purified, that is, compound D, and the yield was 98%.

### Example 5: methyl 4-(2-bromoacetyl)benzoate (compound G)

Methyl 4-acetylbenzoate (5 g, 28 mmol) was dissolved in acetonitrile (100 mL), then added to NBS (6.0 g, 33.6 mmol) and TsOH (482 mg, 2.8 mmol), and purified by column chromatography (PE:EA=10:1). The white solid was 4.6 g (i.e., compound G), and the yield was 63.9 %. ¹H NMR (400 MHz, CDCl₃)*δ*8.13 (d, *J =* 8.1 Hz, 2H), 8.02 (d, *J =* 8.1Hz, 2H), 4.46 (s, 2H), 3.94 (s, 3H); ¹³C NMR (100 MHz, CDCl₃)*δ*190.84, 165.95, 137.16, 134.62, 130.01, 128.87, 52.58, 30.71.

### Example 6: methyl4-(2-(2-imino-1,3,4-thiadiazol-3(2H)-yl)acetyl)benzoate (compound H)

4-(2-bromoacetyl) methyl benzoate (4 g, 15.6 mmol), 2-amino-1,3, 4-thiadiazole (1.6 g, 16 mmol) and ethanol (100 mL) were added into 250 mL bottle of eggplant, and the reaction was carried out at 80°C for 6 h under magnetic stirring. TLC detection of raw material reaction is complete. The reaction bottle was cooled to room temperature, filtered, and washed with ethanol for 3 times to obtain 3.2 g of white solid (i.e., compound H). The yield was 74%. ¹H NMR (400 MHz, CDCl₃) *δ* 8.15 (d, *J =* 8.1Hz, 2H), 8.05 (d, *J =* 8.1Hz, 2H), 7.67 (s, 1H), 5.41 (s, 2H), 3.96 (s, 3H); ¹³C NMR (100 MHz, CDCl₃)*δ*192.06, 166.05, 161.55, 137.93, 134.56, 133.53, 130.03, 128.07, 53.71, 52.55.

### Example 7: 4-(imidazo[2,1-b][1,3,4]thiadiazol-6-yl)benzoic acid (compound I)

4-(2-(2-amino-1,3, 4-thiadiazole) acetyl) methyl benzoate (3 g, 10.8 mmol) and 2N hydrochloric acid (60 mL) were added into 100 mL round-bottom flask for reflux reaction for 6 h at 100°C. TLC detection of raw material reaction is complete. The reaction bottle was cooled to room temperature, 1N sodium hydroxide was added under the ice bath to adjust the pH to 9-10, ethyl acetate was added, transferred to the separating funnel, Shake well and let it settle, the water layer was separated, the pH of the water layer was adjusted to 2-3 with 1N hydrochloric acid, and the solid (that is, compound I, 2.2g) was extracted and filtered, the yield was 83%. ¹H NMR (400 MHz, DMSO-*d6*)*δ*9.24 (s, 1H), 8.87 (s, 1H), 7.98-7.92 (m, 4H); ¹³C NMR (100 MHz, DMSO-*d6*)*δ*167.62, 151.69, 145.50, 145.26, 138.49, 130.38, 129.83, 125.12, 112.39.

### Example 8: methyl 4-(5-(4-cyanophenyl)imidazo[2,1-b][1,3,4]thiadiazol-2-yl) benzoate (compound L)

compound C (50 mg, 0.12 mmol), 4-cyanophenylboronic acid (35.2 mg, 0.24 mmol), Pd(PPh₃)₄ (28 mg, 0.025 mmol), CsF (51 mg, 0.24 mmol) were added into 100 mL round-bottom flask. Dioxane (40 mmol) and water (10 mL), N₂ protection, 100°C reaction for 6 h. TLC test material reaction is complete, cooled to room temperature after filtration yellow solid 16mg (i.e., compound L), yield 30%. ¹H NMR (400 MHz, DMSO-*d6*)*δ* 8.14 (m, 9H), 3.91 (s, 3H).

### Example 9: (4-(5-bromoimidazo[2,1-b][1,3,4]thiadiazol-2-yl)phenyl)

### (morpholino)methanone (Intermediate 1)

The compound D (2 g, 6.2 mmol), EDCI (2.4 g,12.4 mmol), NHS (1.4 g,12.4 mmol), morpholine (2.7 g, 31 mmol) were dissolved in DMF (50 mL). The reaction was carried out at room temperature until the reaction of the raw material was completely detected by TLC. After the DMF was removed by spin evaporation, the white solid was purified by column chromatography (CH₂Cl₂:CH₃OH=50:1), and 1.8 g (i.e., intermediate 1) was obtained. The yield was 75%. 1H NMR (400 MHz, CDCl₃) *δ* 7.97 (d, *J =* 8.3Hz, 2H), 7.58 (d, *J =* 8.3Hz, 2H), 7.39 (s, 1H), 3.61 (s, 4H), 3.51 (s, 2H), 3.30 (s, 2H).¹³C NMR (101 MHz, CDCl₃) *δ*168.95, 161.56, 144.10, 138.68, 133.74, 131.25, 128.14, 127.24, 96.26, 66.82, 48.17, 42.65.

### Example 10: (4-(5-bromoimidazo[2,1-b][1,3,4]thiadiazol-2-yl)phenyl) (4-(dimethylamino)piperidin-1-yl)methanone (Intermediate 2)

Compound D (2 g, 6.2 mmol), EDCI (2.4 g,12.4 mmol), NHS (1.4 g,12.4 mmol), 4-dimethylaminopiperidine (1.6 g,12.4 mmol) were dissolved in DMF (50 mL), The reaction was carried out at room temperature until the reaction of the raw material detected by TLC was complete. After removal of DMF by rotary evaporation, it was purified by column chromatography (CH₂Cₗ₂:CH₃OH=50:1), and 1.6 g of white solid (intermediate 2) was obtained with a yield of 59.3%. ¹H NMR (400 MHz, DMSO-d6) *δ* 8.01 (d, *J =* 8.4 Hz, 2H), 7.60 (d, *J =* 8.0 Hz, 2H), 7.46 (s, 1H), 4.45 (d, *J=* 13.1 Hz, 1H), 3.56 (d, *J =* 13.6 Hz, 1H), 3.06 (t, *J =* 12.9 Hz, 1H), 2.83 (t, *J =* 12.6 Hz, 1 H), 2.47 2.37 (m, 1H), 2.21 (s, 6H), 1.86 (d, *J=* 12.9 Hz, 1H), 1.70 (d, *J=* 12.6 Hz, 1 H), 1.39 (q, *J =* 13.5 Hz, 2H ); ¹³C NMR (101 MHz, DMSO-*d6*)*δ*168.13, 162.63, 144.34, 140.26, 133.98, 130.48, 128.44, 127.58, 96.51, 61.73, 46.71, 41.78, 28.74, 28.10.

### Example 11: (4-(5-bromoimidazo[2,1-b][1,3,4]thiadiazol-2-yl)phenyl)(4-m-ethylpiperazin-1-yl) methanone (Intermediate 3)

Compound D 8 mg (0.18 mmol), (1-ethyl-3 (3-dimethylpropylamine) carbodiimide 69 mg (0.36 mmol), N-hydroxysuccinimide 42 mg(0.36 mmol) were mixed in 10 mL DMF. After the reaction was stirred for 10 h at room temperature, 99 µL N-methylpiperazine was added, and after stirring for 1 h, the mixture was washed with water, extracted with chloroform, and column chromatography was performed to obtain 50 mg of yellow solid (intermediate 3), with a yield of 68%. ¹H NMR (400 MHz, CDCl₃)*δ* 7.97-7.93 (m, 1H), 7.57-7.52 (m, 2H), 7.25 (s, 1H), 3.84-3.77 (m, 2H), 3.52-3.34 (m, 2H) 2H), 2.57-2.45 (m, 2H), 2.35 (s, 2H), 2.31 (s, 3H). ¹³C NMR (100 MHz, CDCl₃) *δ*168.8, 161.7, 144.1, 139.2, 133.7, 131.0, 128.1, 127.2, 96.2, 55.2, 54.6, 47.6, 46.0, 42.1.

### Example 12: (4-(imidazo[2,1-b][1,3,4]thiadiazol-6-yl)phenyl) (morpholino)methanone (Intermediate 4)

Compound I (2 g, 8.2 mmol), EDCI (3.2 g, 16.4 mmol), NHS (1.9 g, 16.4 mmol), morpholine (3.6 g, 41 mmol) were dissolved in DMF (50 mL), The reaction was carried out at room temperature until the reaction of the raw material detected by TLC was complete. The DMF was removed by rotary evaporation and purified by column chromatography (CH₂Cl₂:CH₃OH=50:1) to obtain 1.8 g of white solid (intermediate 4) with a yield of 69.8%. ¹H NMR (400 MHz, CDCl₃)*δ*8.57 (s, 1H), 8.14 (s, 1H), 7.88 (d, *J* = 8.1 Hz, 2H), 7.47 (d, *J =* 8.0 Hz, 2H), 3.72 (s, 6H), 3.56 (s, 2H); ¹³C NMR (100 MHz, CDCl₃)*δ*170.22, 147.03, 146.49, 144.67, 135.43, 134.24, 127.80, 125.18, 110.13, 66.89, 48.27, 42.68.

### Example 13: (4-(imidazo[2,1-b][1,3,4]thiadiazol-6-yl)phenyl)(4-methylpi- perazin-1-yl) methanone (Intermediate 5)

Compound I (2 g, 8.2 mmol) and N-methylpiperazine (1.6 g, 16.4 mmol) were used as raw materials to obtain a white solid (1.6 g) according to the synthesis method of intermediate 4, and the yield was 59.6%. ¹H NMR (400 MHz, CDCl₃)*δ*8.56 (s, 1H), 8.14 (s, 1H), 7.87 (d, *J =* 8.3 Hz, 2H), 7.47 (d, *J =* 8.3 Hz, 2H), 3.82 (s, 2H), 3.51 (s, 2H), 2.50 (s, 2H), 2.39 (s, 2H), 2.34 (s, 3H); ¹³C NMR (100 MHz, CDCl₃)δ170.13, 146.80, 146.70, 144.61, 135.22, 134.82, 127.76, 125.15, 110.08, 55.18, 47.73, 46.04, 42.15.

### Example 14: (4-(dimethylamino)piperidin-1-yl)(4-(imidazo[2,1-b][1,3,4]thia-diazol-6-yl) phenyl)methanone (Intermediate 6)

Compound I (2 g, 8.2 mmol) and 4-dimethylaminopiperidine (2.1 g, 16.4 mmol) were used as raw materials to obtain a white solid (1.5 g) according to the synthesis method of intermediate 4 in a yield of 51.5%. ¹H NMR (400 MHz, CDCl₃) *δ* 8.66 (s, 1H), 8.17 (s, 1H), 7.87 (d, *J =* 8.2 Hz, 2H), 7.46 (d, *J* = 8.2 Hz, 2H), 4.76 (s, 1H), 3.92 (s, 1 H), 3.06 (s, 1H), 2.82 (s, 1H), 2.53 (ddt, *J=* 11.4, 7.5, 3.6 Hz, 1H), 2.35 (s, 6H), 1.87 (s, 2H), 1.52 (s, 2H); ¹³C NMR (100 MHz, CDCl₃)*δ*170.52, 147.55, 146.49, 144.95, 135.26, 134.73, 127.62, 125.29, 110.34, 62.19, 47.17, 41.66, 41.27, 28.78, 27.63.

### Example 15: (4-(imidazo[2,1-b][1,3,4]thiadiazol-6-yl)phenyl) (4-morpholi- nopiperidin-1-yl) methanone (Intermediate 7)

Compound I (2 g, 8.2 mmol) and 4-morpholine piperidine hydrochloride (3.4 g, 16.4 mmol) were used as raw materials to obtain a white solid (900 mg) according to the synthesis method of intermediate 4 in a yield of 27.6%. ¹H NMR (400 MHz, CDCl₃-CD₃OD)*δ*8.59 (s, 1H), 8.10 (s, 1H), 7.80 (d, *J =* 8.2 Hz, 2H), 7.40 (d, *J =* 8.2 Hz, 2H), 4.67 (s, 1H), 3.85 (s, 1H), 3.71 (t, *J =* 4.7 Hz, 4H), 3.01 (s, 1H), 2.75 (s, 1H), 2.59 (t, *J=* 4.8 Hz, 4H), 2.52 (s, 1H),1.97(s,1H), 1.83 (s,1H), 1.46 (s, 2H); ¹³C NMR (100 MHz, CDCl₃-CD₃OD)*δ*172.74, 170.12, 147.22, 146.08, 144.60, 134.86, 134.34, 127.27, 124.95, 109.98, 66.36, 61.72, 49.24, 46.70, 41.26, 25.06.

### Example 16: 4-(5-4-cyanophenyl)imidazo[2,1-b][1,3,4]thiadiazole-2-yl) benzoic acid (Intermediate 8)

Compound L (340 mg, 1.01 mmol) and LiOH•H₂O (420 mg, 10 mmol) were mixed in 30 mL THF/H₂O (V:V=1:1) and stirred for 12 h at room temperature. After adjusting the pH to 2-3 with HCl, the white solid compound M, intermediate 8, was filtered and used in the next step without further purification.

### Example 17: 4-(2-(4-(morpholine-4-carbonyl)phenyl)imidazo[2,1-b][1,3,4] thiadiazol-5-yl)benzonitrile (compound 1)

(4-(5-bromoimidazo[2,1-*b*][1,3,4]thiadiazol-2-yl)phenyl)(morpholino)methanone(intermediate 1) (200 mg, 0.5 mmol), 4-cyanophenylboronic acid (150 mg, 1 mmol), Reactions were performed with Pd(PPh₃)₄ (120 mg, 0.1 mmol), CsF (380 mg, 2.5 mmol), dioxane (40 mmol) and water (10 mL) under N₂ protection at 100 °C for 6 h. Dichloromethane and water were added and transferred to a separating funnel. The organic layer was separated. The aqueous layer was extracted twice with dichloromethane, the organic layer was combined, the organic layer was washed twice with saturated NaCl solution, anhydrous Mg(SO₄)₂ was dried, filtered, and the filtrate was dried. After purification by column chromatography (CH₂Cl₂:CH₃OH=50:1), 40 mg of white solid was obtained in 19.2% yield. ¹H NMR (500 MHz, CDCl₃)*δ*8.14 (d, *J =* 6.9 Hz, 2H), 8.02 (d, *J =* 7.2 Hz, 2H), 7.77 (d, *J =* 7.8 Hz, 3H), 7.62 (d, *J* = 7.3 Hz, 2H), 3.82 (s, 4H), 3.68 (s, 2H), 3.48 (s, 2H); ¹³C NMR(125MHz,CDCl₃)*δ*168.85, 162.00, 146.97, 138.84, 132.76, 132.52, 132.06, 131.13,128.58,128.25,127.21, 124.83, 118.78, 110.66, 66.81, 48.18, 42.62; HRMS calcd for (C₂₂H₁₈O₂N₃S + H)⁺ 416.1176, found 416.1168.

### Example 18: (4-(5-(4-fluorophenyl)imidazo[2,1-b][1,3,4]thiadiazol-2-yl) phenyl)(morpholino)methanone (compound 2)

(4-(5-bromoimidazo[2,1-b][1,3,4]thiadiazol-2-yl)phenyl)(morpholino)methanone (intermediate 1) (200 mg, 0.5 mmol), 4-fluorobenzoboronic acid (140 mg, 1 mmol) were added to a 100-ml solanaceous flask, Reactions with Pd(PPh₃)₄ (120 mg, 0.1 mmol), CsF (380 mmol, 2.5 mmol), dioxane (40 mmol) and water (10 mL) were performed under N₂ protection at 100 °C for 6 h. Dichloromethane and water were added and transferred to a separating funnel. The organic layer was separated. The aqueous layer was extracted twice with dichloromethane, the organic layer was merged, the organic layer was washed twice with saturated NaCl solution, anhydrous Mg(SO₄)₂ was dried, filtered, and the filtrate was spun dry. After purification by column chromatography (CH₂Cl₂:CH₃OH=50:1), 56 mg of white solid was obtained in a yield of 27.5%. ¹H NMR (500 MHz, CDCl₃)*δ*8.00 (d, *J =* 6.4 Hz, 2H), 7.98-7.94 (m, 2H), 7.59 (d, *J =* 6.4 Hz, 3H), 7.20 (t, *J =* 7.8 Hz, 2H), 3.82 (s, 4H), 3.68 (s, 2H), 3.48 (s, 2H); ¹³C NMR (125 MHz, CDCl₃)*δ*168.92, 163.29, 161.50, 144.92, 138.62, 135.70, 131.33, 129.69, 128.17, 127.16, 126.99, 124.36, 116.08, 114.45, 66.82, 48.12, 42.64; HRMS calcd for (C₂₁H₁₈O₂N₄FS + H)⁺ 409.1129, found 409.1119.

### Example 19: (4-(5-(4-hydroxyphenyl)imidazo[2,1-b][1,3,4]thiadiazol-2-yl) phenyl)(morpholino)methanone (compound 3)

(4-(5-(4-hydroxyphenyl)imidazo[2,1-b][1,3,4]thiadiazol-2-yl)phenyl)(morpholino)methanone (intermediate 1) (200 mg, 0.5 mmol), 4-hydroxyphenylboronic acid (140 mg, 1 mmol), Reactions with Pd(PPh₃)₄ (120 mg, 0.1 mmol), CsF (380 mmol, 2.5 mmol), dioxane (40 mmol) and water (10 mL) were performed under N₂ protection at 100 °C for 6 h. Dichloromethane and water were added and transferred to a separating funnel. The organic layer was separated. The aqueous layer was extracted twice with dichloromethane, the organic layer was merged, the organic layer was washed twice with saturated NaCl solution, anhydrous Mg(SO₄)₂ was dried, filtered, and the filtrate was spun dry. After purification by column chromatography (CH₂Cl₂:CH₃OH=50:1), 60 mg of white solid was obtained in 29.5% yield. ¹H NMR (500 MHz, DMSO-d6) *δ* 9.69 (s, 1H), 8.08 (d, *J =* 7.9 Hz, 2H), 7.87 (d, *J =* 8.2 Hz, 2H), 7.64 (d, *J =* 8.2 Hz, 3H), 6.91 (d, *J =* 8.8 Hz, 2H), 3.65 (s, 4H), 3.57 (s, 2H), 3.36 (s, 2H); ¹³C NMR (125 MHz, DMSO-*d6*)*δ*168.41,161.20, 157.55, 139.21, 131.02, 129.93, 128.65, 128.05, 127.47, 126.71, 119.54, 116.22, 66.48, 48.08, 42.48; HRMS calcd for (C₂₁H₁₉O₃N₄S + H)⁺ 407.1172, found 407.1172.

### Example 20: (4-(5-(4-hydroxyphenyl)imidazo[2,1-b][1,3,4]thiadiazol-2-yl) phenyl)(morpholino)methanone (compound 4)

Using intermediate 1 (200 mg, 0.5 mmol) and 4-methoxycarbonyl phenylboronic acid (180 mg, 1 mmol) as raw materials, 78 mg white solid was obtained according to the synthesis method of compound 1, and the yield was 34.8%. ¹H NMR (400 MHz, CDCl₃)*δ*8.15 (d, *J=* 8.5 Hz, 3H), 8.08 (d, *J= 8.6* Hz, 3H), 8.02 (d, *J= 8.4* Hz, 2H), 7.77 (s, 1H), 7.60 (d, *J =* 8.4 Hz, 2H), 3.95 (s, 3H), 3.82 (s, 4H), 3.66 (s, 2H), 3.48 (s, 2H); ¹³C NMR (100 MHz, CDCl₃)*δ*169.02, 166.77, 161.97, 146.13, 138.83, 133.72, 132.34, 131.25, 130.40, 129.11, 128.79, 128.35, 127.36, 124.63, 66.93, 52.37, 48.28, 42.75; HRMS calcd for (C₂₃H₂₁O₄N₄S + H)⁺ 499.1278, found 499.1269.

### Example 21: morpholino(4-(5-(4-(trifluoromethyl)phenyl)imidazo[2,1-b][1, 3,4]thiadiazol-2-yl)phenyl)methanone (compound 5)

Using intermediate 1 (200 mg, 0.5 mmol) and 4-trifluoromethylphenylboronic acid (190 mg, 1 mmol) as raw materials, 80 mg white solid was obtained according to the synthesis method of compound 1, and the yield was 34.9%. ¹H NMR (400 MHz, CDCl₃) *δ* 8.12 (d, *J=* 8.1 Hz, 2H), 8.01 (d, *J=* 8.1 Hz, 2H), 7.73 (d, *J= 8.1* Hz, 3H), 7.59 (d, *J =* 8.2 Hz, 2H), 3.82 (s, 4H), 3.66 (s, 2H), 3.48 (s, 2H); ¹³C NMR (100 MHz, CDCl₃) *δ* 168.98, 161.50, 146.46, 138.67, 132.29, 131.80, 131.41, 129.44, 128.24, 127.20, 125.92, 124.85, 122.76, 66.85, 48.22, 42.77; HRMS calcd for (C₂₂H₁₈O₂N₄F₃S + H)⁺ 459.1097, found 459.1086.

### Example 22: (4-(5-(4-chlorophenyl)imidazo[2,1-b][1,3,4] thiadiazol-2-yl) phenyl)(morpholino)methanone (compound 6)

Using intermediate 1 (200 mg, 0.5 mmol) and 4-chlorobenylboronic acid (160 mg, 1 mmol) as raw materials, 76 mg white solid was obtained according to the synthesis method of compound 1, and the yield was 35.8%. ¹H NMR (400 MHz, CDCl₃)*δ*8.00 (d, *J =* 8.6 Hz, 2H), 7.93 (d, *J =* 8.7 Hz, 2H), 7.61 (s, 1H), 7.58 (d, *J =* 8.6 Hz, 2H), 7.45 (d, *J =* 8.7 Hz, 2 H), 3.80 (s, 4 H), 3.67 (s, 2 H), 3.51 (s, 2 H), 3.47 (s, 2 H); ¹³C NMR (100 MHz, CDCl₃)*δ*169.02, 161.15, 145.70, 138.53, 133.34, 131.54, 131.23, 129.14, 128.20, 127.17, 126.94, 126.18, 66.85, 48.23, 42.58; HRMS calcd for (C₂₁H₁₈O₂N₄ClS + H)⁺ 425.0834, found 425.0823.

### Example 23: morpholino(4-(5-(p-tolyl)imidazo[2,1-b][1,3,4]thiadiazol-2-yl) phenyl)methanone(compound 7)

With intermediate 1 (200 mg, 0.5 mmol) and 4-methylphenylboronic acid (140 mg, 1 mmol) as raw materials, 64 mg of white solid was obtained according to the synthesis method of compound 1, and the yield was 31.7%.¹H NMR (400 MHz, CDCl₃) *δ* 8.00 (d, *J =* 8.6 Hz, 2H), 7.87 (d, *J =* 8.2 Hz, 2H), 7.58 (m, 3H), 7.30 (d, *J =* 7.9 Hz, 3H), 3.80 (s, 4H), 3.67 (s, 2H), 3.50 (s, 2H), 2.42 (s, 3H); ¹³C NMR (100 MHz, CDCl₃) *δ* 169.11, 160.63, 145.06, 138.33, 137.64, 131.79, 130.55, 129.59, 128.33, 128.13, 127.14, 125.63, 125.09, 66.86, 48.14, 42.67, 21.36; HRMS calcd for (C₂₂H₂₁O₂N₄S + H)⁺ 405.1380, found 405.1374.

### Example 24: morpholino(4-(5-(p-tolyl)imidazo[2,1-b][1,3,4]thiadiazol-2-yl) phenyl)methanone(compound 8)

54 mg of white solid was obtained from intermediate 1 (200 mg, 0.5 mmol) and 4-hydroxymethylphenylboronic acid (152 mg, 1 mmol) according to the synthesis method of compound 1, and the yield was 25.7%.¹H NMR (400 MHz, CDCl₃) *δ* 7.95 (dd, *J =* 8.4, 5.1 Hz, 4H), 7.58 (s, 1H), 7.54 (d, *J =* 8.4 Hz, 2H), 7.46 (d, *J =* 8.2 Hz, 2H), 4.74 (s, 2H), 3.81 (s, 4H), 3.65 (s, 2H), 3.46 (s, 2H), 2.40 (s, 1H); ¹³C NMR (100 MHz, CDCl₃) *δ* 169.19, 161.00, 145.43, 140.62, 138.36, 131.65, 130.86, 128.21, 128.01, 127.58, 127.19, 125.19, 66.92, 65.02, 48.31, 42.72; HRMS calcd for (C₂₂H₂₁O₃N₄S + H)⁺ 421.1329, found 421.1322.

### Example 25: (4-(5-(3-fluoro-4-(trifluoromethyl)phenyl)imidazo[2,1-b][1,3,4] thiadiazol-2-yl)phenyl)(morpholino)methanone (compound 9)

Using intermediate 1 (200 mg,0.5mmol) and 3-fluoro-4-trifluoromethylphenylboronic acid (208 mg, 1 mmol) as raw materials, 78 mg of white solid was obtained according to the synthesis method of compound 1, and the yield was 32.8%.¹H NMR (400 MHz, CDCl₃) *δ* 8.01 (d, *J =* 8.2 Hz, 2H), 7.92 (dd, *J=* 11.8, 1.6 Hz, 1H), 7.83 (d, *J=* 8.3 Hz, 1H), 7.74 (s, 1H), 7.69 (t, *J=* 7.8 Hz, 1H), 7.60 (d, *J =* 8.2 Hz, 2H), 3.81 (s, 4H), 3.67 (s, 2H), 3.47 (s, 2H); ¹³C NMR (100 MHz, CDCl₃) *δ* 168.91, 161.95, 147.00, 138.85, 134.21, 133.03, 131.18, 128.29, 127.80, 127.24, 125.74, 119.84, 112.71, 66.84, 48.24, 42.67; HRMS calcd for (C₂₂H⁺₇O₂N₄F₄S + H)⁺ 477.1003, found 477.0989.

### Example 26: (4-(5-(3-fluoro-4-hydroxyphenyl)imidazo[2,1-b][1,3,4]thiadia-zol-2-yl)phenyl)(morpholino)methanone (compound 10)

Using intermediate 1(200 mg, 0.5 mmol) and 3-fluoro-4-hydroxyphenylboronic acid (156 mg, 1 mmol) as raw materials, 98 mg white solid was obtained according to the synthesis method of compound 1, and the yield was 46.2%.¹H NMR (400 MHz, CDCl₃-CD₃OD) *δ* 7.94 (d, *J =* 8.3 Hz, 2H), 7.66 (dd, *J=* 12.2, 2.1 Hz, 1H), 7.51 (dd, *J =* 8.6, 2.5 Hz, 3H), 7.41 (s, 1H), 6.98 (t, *J =* 8.7 Hz, 1H), 3.75 (s, 4H), 3.60 (s, 2H), 3.42 (s, 2H); ¹³C NMR (100 MHz, CDCl₃-CD₃OD) *δ* 169.60, 161.23, 152.93, 150.54, 144.95, 138.31, 131.76, 129.74, 128.24, 127.41, 121.70, 120.44, 118.34, 113.07, 66.91, 42.84; HRMS calcd for (C₂₁H₁₈O₃N₄FS + H)⁺ 425.1078, found 425.1066.

### Example 27: (4-(5-(3,4-difluorophenyl)imidazo[2,1-b][1,3,4]thiadiazol-2-yl) phenyl)(morpholino)methanone (compound 11)

Using intermediate 1 (200 mg, 0.5 mmol) and 3, 4-difluorobenboronic acid (160 mg, 1 mmol) as raw materials, 68 mg white solid was obtained according to the synthesis method of compound 1, and the yield was 31.9%.¹H NMR (400 MHz, CDCl₃) *δ* 8.00 (d, *J =* 8.3 Hz, 2H), 7.87 (ddd, *J =* 11.6, 7.5, 2.2 Hz, 1H), 7.68 (dddd, *J=* 8.7, 3.9, 2.2, 1.4 Hz, 1H), 7.59 (m, 3H), 7.26 (dt, *J =* 10.1, 8.5 Hz, 1H), 3.80 (s, 4H), 3.67 (s, 2H), 3.48 (s, 2H); ¹³C NMR (100 MHz , CDCl₃) *δ* 169.00, 161.50, 151.90, 150.99, 149.43, 148.50, 145.76, 138.63, 131.38, 128.23, 127.20, 126.29, 125.49, 121.11, 118.03, 114.14, 66.84, 48.21, 42.69; HRMS calcd for (C₂₁H₁₇O₂N₄F₂S + H)⁺ 427.1035, found 427.1024.

### Example 28: 4-(2-(4-(4-(dimethylamino)piperidine-1-carbonyl) phenyl)imid-azo[2,1-b][1,3,4]thiadiazol-5-yl)benzonitrile (compound 12)

From intermediate 2 (300 mg, 0.7 mmol) and 4-cyanophenylboronic acid (200 mg, 1.4 mmol), 50 mg white solid was obtained according to the synthesis method of compound 1, and the yield was 15.6%.¹H NMR (400 MHz, CDCl₃) *δ* 8.14 (d, *J =* 8.1 Hz, 2H), 8.01 (d, *J =* 7.9 Hz, 2H), 7.80 - 7.73 (m, 3H), 7.60 (d, *J =* 7.9 Hz, 2H), 4.82 (s, 1H), 3.86 (s, 1H), 3.12 (s, 1H), 2.80 (m, 1H), 2.71 (s, 1H), 2.50 (s, 6H), 2.06 (s, 2H) 1.62 (s, 2H); ¹³C NMR (100 MHz, CDCl₃) *δ* 168.82, 161.88, 147.05, 139.18, 133.01, 132.78, 132.64, 131.13, 130.45, 128.06, 127.20, 126.58, 126.37, 124.79, 118.86, 110.53, 62.29, 46.59, 41.28, 40.89, 28.62, 26.98; HRMS calcd for (C₂₅H₂₅ON₆S + H)⁺ 457.1805, found 457.1792.

### Example 29: (4-(dimethylamino)piperidin-1-yl)(4-(5-(4-fluorophenyl)imida-zo[2,1-b][1,3,4]thiadiazol-2-yl)phenyl)methanone (compound 13)

From intermediate 2 (300 mg, 0.7 mmol) and 4-fluorobenboronic acid (200 mg, 1.4 mmol), 40 mg of white solid was obtained according to the synthesis method of compound 1, and the yield was 12.7%.¹H NMR (400 MHz, DMSO-*d*₆) *δ* 8.09 (m, 4H), 7.82 (s, 1H), 7.61 (d, *J =* 8.0 Hz, 2H), 7.36 (d, *J= 7.9* Hz, 2H), 4.46 (s, 1H), 3.56 (s, 1H), 3.06 (s, 1H), 2.83 (s, 1H), 2.26 (s, 6H), 1.89 (s, 1H), 1.74 (s, 1H), 1.41 (s, 2H); ¹³C NMR (100 MHz, DMSO-*d*₆) *δ* 168.29, 161.80, 160.64, 145.44, 139.58, 131.69, 130.92, 128.45, 127.57, 127.18, 126.78, 125.23, 116.59, 116.37, 62.21, 55.06, 29.51, 26.30; HRMS calcd for (C₂₄H₂₅ON₅FS + H)⁺ 450.1758, found 450.1750.

### Example 30: (4-(dimethylamino)piperidin-1-yl)(4-(5-(4-hydroxyphenyl)imidazo[2,1-b][1,3,4]thiadiazol-2-yl)phenyl)methanone (compound 14)

From intermediate 2 (300 mg, 0.7 mmol) and 4-hydroxyphenylboronic acid (190 mg, 1.4 mmol), 50 mg white solid was obtained according to the synthesis method of compound 1, and the yield was 15.9%.¹H NMR (400 MHz, DMSO-*d*₆) *δ* 9.71 (s, 1H), 8.04 (d, *J =* 8.2 Hz, 2H), 7.84 (d, *J =* 8.7 Hz, 2H), 7.63 (s, 1H), 7.59 (d, *J =* 8.2 Hz, 2H), 6.88 (d, *J =* 8.7 Hz, 2H), 4.46 (s, 1H), 3.53 (s, 1H), 3.03 (s, 1H), 2.82 (s, 1H), 2.51 (m, 1H), 2.24 (s, 6H), 1.84 (s, 1H), 1.69 (s, 1H), 1.38 (s, 2H); ¹³C NMR (100 MHz, DMSO-*d*₆) *δ* 168.17, 161.26, 157.60, 144.25, 139.90, 130.85, 129.92, 128.34, 128.06, 127.45, 126.71, 119.54, 116.25, 61.73, 46.60, 41.54, 41.05, 28.54, 27.89; HRMS calcd for (C₂₄H₂₆O₂N₅S + H)⁺ 448.1802, found 448.1798.

### Example 31: (4-(dimethylamino)piperidin-1-yl)(4-(5-(4-methoxyphenyl)imidazo[2,1-b][1,3,4]thiadiazol-2-yl)phenyl)methanone (compound 15)

From intermediate 2 (300 mg, 0.7 mmol) and 4-methoxyphenylboronic acid (210 mg, 1.4 mmol), 40 mg light yellow solid was obtained according to the synthesis method of compound 1, and the yield was 12.4%.¹H NMR (400 MHz, CDCl₃) *δ* 7.98 (d, *J =* 8.4 Hz, 2H), 7.90 (d, *J =* 8.9 Hz, 2H), 7.55 (d, *J =* 8.5 Hz, 2H), 7.51 (s, 1H), 7.02 (d, *J =* 8.9 Hz, 2H), 4.78 (s, 1H), 3.87 (s, 4H), 2.85 (s, 1H), 2.70 (m, 1H), 2.44 (s, 6H), 2.00 (s, 2H), 1.54 (s, 2H); ¹³C NMR (101 MHz, CDCl₃) *δ* 169.05, 160.73, 159.24, 144.76, 138.81, 132.58, 131.70, 129.93, 127.98, 127.14, 126.65, 121.23, 114.40, 62.37, 55.47, 41.11, 29.80; HRMS calcd for (C₂₅H₂₈O₂N₅S + H)⁺ 462.1958, found 462.1947.

### Example 32: 4-(dimethylamino)piperidin-1-yl)(4-(5-(4-(trifluoromethyl)phenyl)imidazo[2,1-b][1,3,4]thiadiazol-2-yl)phenyl)methanone (compound 16)

From intermediate 2 (300 mg, 0.7 mmol) and 4-trifluoromethylphenylboronic acid (260 mg, 1.4 mmol), 32 mg white solid was obtained according to the synthesis method of compound 1, and the yield was 9.2%.¹H NMR (400 MHz, CDCl₃) *δ* 8.11 (d, *J =* 8.1 Hz, 2H), 7.99 (d, *J =* 8.0 Hz, 2H), 7.72 (d, *J =* 9.7 Hz, 3H), 7.57 (d, *J =* 8.6 Hz, 2H), 4.76 (s, 1H), 3.80 (s, 1H), 3.07 (s, 2H), 2.58 (m, 1H), 2.39 (s, 6H), 2.00 (s, 2H), 1.56 (m, 2H); ¹³C NMR (100 MHz, CDCl₃) *δ* 168.87, 161.55, 146.44, 139.10, 132.24, 131.79, 131.28, 128.01, 127.15, 126.83, 125.94, 124.84, 62.31, 46.67, 41.04, 31.93, 29.71, 29.37; HRMS calcd for (C₂₅H₂₅ON₅F₃S + H)⁺ 500.1726, found 500.1718.

### Example 33: 4-(2-(4-(4-methylpiperazine-1-carbonyl)phenyl)imidazo[2,1-b] [1,3,4]thiadiazol-5-yl)benzonitrile (compound 17)

45 mg of white solid was obtained from intermediate 3 (200 mg, 0.5 mmol) and 4-cyanophenylboronic acid (150 mg, 1 mmol) according to the synthesis method of compound 1, and the yield was 21%.¹H NMR (400 MHz, CDCl₃) *δ* 8.13 (d, *J =* 8.3 Hz, 2H), 7.99 (d, *J =* 7.9 Hz, 2H), 7.75 (d, *J =* 8.3 Hz, 3H), 7.59 (d, *J =* 8.0 Hz, 2H), 3.83 (s, 2H), 3.45 (s, 2H), 2.51 (s, 2H), 2.36 (s, 2H), 2.33 (s, 3H); ¹³C NMR (100 MHz, CDCl₃) *δ* 168.78, 161.94, 147.06, 139.34, 133.01, 132.77, 132.65, 131.01, 128.20, 127.15, 126.37, 124.79, 118.85, 110.55, 55.23, 54.64, 47.65, 46.02, 42.18; HRMS calcd for (C₂₃H₂₁ON₆S + H)⁺ 429.1492, found 429.1485.

### Example 34: (4-(5-(4-fluorophenyl)imidazo[2,1-b][1,3,4]thiadiazol-2-yl) phenyl)(4-methylpiperazin-1-yl)methanone (compound 18)

With intermediate 3 (200 mg, 0.5 mmol) and 4-fluorobenboronic acid (140 mg, 1 mmol) as raw materials, 60 mg white solid was obtained according to the synthesis method of compound 1, and the yield was 28.6%.¹H NMR (400 MHz, CDCl₃) *δ* 7.97 (m, 4H), 7.56 (m, 3H), 7.17 (m, 2H), 3.82 (s, 2H), 3.45 (s, 2H), 2.52 (s, 2H), 2.37 (s, 2H), 2.37 (s, 3H); ¹³C NMR (100 MHz, CDCl₃) *δ* 168.99, 163.51, 161.27, 161.05, 145.36, 139.06, 131.43, 130.65, 128.20, 127.42, 127.17, 126.94, 124.77, 116.17, 115.95, 55.32, 54.71, 47.70, 46.12, 42.18; HRMS calcd for (C₂₂H₂₁ON₅FS + H)⁺ 422.1445, found 422.1437.

### Example 35: methyl4-(2-(4-(4-methylpiperazine-1-carbonyl)phenyl)imidazo[2,1-b][1,3,4]thiadiazol-5-yl)benzoate (compound 19)

46 mg of white solid was obtained from intermediate 3 (200 mg, 0.5 mmol) and 4-methoxycarbonyl phenylboronic acid (180 mg, 1 mmol) according to the synthesis method of compound 1 in a yield of 20%.¹H NMR (400 MHz, CDCl₃) *δ* 8.14 (d, *J =* 8.6 Hz, 2H), 8.08 (d, *J =* 8.7 Hz, 2H), 8.00 (d, *J =* 8.3 Hz, 2H), 7.73 (s, 1H), 7.58 (d, *J =* 8.3 Hz, 2H), 3.94 (s, 3H), 3.83 (s, 2H), 3.46 (s, 2H), 2.52 (s, 2H), 2.38 (s, 2H), 2.34 (s, 3H); ¹³C NMR (100 MHz, CDCl₃) *δ* 168.95, 166.83, 161.54, 146.61, 139.20, 132.73, 132.56, 131.30, 130.37, 128.81, 128.24, 127.27, 127.22, 124.42, 55.33, 52.33, 47.73, 46.15, 42.25, 29.80; HRMS calcd for (C₂₄H₂₄O₃N₅S + H)⁺ 462.1594, found 462.1582.

### Example 36: (4-methylpiperazin-1-yl)(4-(5-(pyridin-4-yl)imidazo[2,1-b] [1, 3,4]thiadiazol-2-yl)phenyl)methanone (compound 20)

From intermediate 3 (200 mg, 0.5 mmol) and pyridine-4-boronic acid (120 mg, 1 mmol), 50 mg white solid was obtained according to the synthesis method of compound 1, and the yield was 24.7%.¹H NMR (400 MHz, CDCl₃) *δ* 8.67 (d, *J =* 6.3 Hz, 2H), 7.99 (d, *J =* 8.4 Hz, 2H), 7.89 (d, *J =* 6.3 Hz, 2H), 7.81 (s, 1H), 7.57 (d, *J =* 8.5 Hz, 2H), 3.82 (s, 2H), 3.45 (s, 2H), 2.51 (s, 2H), 2.36 (s, 2H), 2.32 (s, 3H); ¹³C NMR (100 MHz, CDCl₃) *δ* 168.86, 162.03, 150.51, 147.41, 139.33, 135.55, 133.49, 131.07, 128.27, 127.23, 125.53, 118.44, 55.30, 54.68, 47.69, 46.11, 42.20; HRMS calcd for (C₂₁H₂₀ON₆S + H)⁺ 405.1458, found 405.1465.

### Example 37: (4-methylpiperazin-1-yl)(4-(5-(4-(trifluoromethyl)phenyl)imidazo[2,1-b][1,3,4]thiadiazol-2-yl)pheny l)methanone (compound 21)

54 mg of white solid was obtained from intermediate 3 (200 mg, 0.5 mmol) and 4-trifluoromethylphenylboronic acid (190 mg, 1 mmol) according to the synthesis method of compound 1, and the yield was 23%.¹H NMR (400 MHz, CDCl₃) *δ* 8.10 (d, *J =* 8.1 Hz, 2H), 7.98 (d, *J =* 8.4 Hz, 2H), 7.71 (d, *J =* 8.7 Hz, 3H), 7.57 (d, *J =* 8.4 Hz, 2H), 3.82 (s, 2H), 3.45 (s, 2H), 2.51 (s, 2H), 2.36 (s, 2H), 2.33 (s, 3H); ¹³C NMR (100 MHz, CDCl₃) *δ* 168.93, 161.71, 146.51, 139.25, 132.25, 131.83, 131.23, 128.25, 127.21, 126.88, 125.99, 124.91, 55.34, 54.72, 47.74, 46.15, 42.24; HRMS calcd for (C₂₃H₂₁ON₅F₃S + H)⁺ 472.1413, found 472.1407.

### Example 38: (4-(5-(4-chlorophenyl)imidazo[2,1-b][1,3,4]thiadiazol-2-yl)phenyl)(4-methylpiperazin-1-yl)methano ne (compound 22)

Using intermediate 3 (200 mg, 0.5 mmol) and 4-chlorobenylboronic acid (160 mg, 1 mmol) as raw materials, 56 mg white solid was obtained according to the synthesis method of compound 1, and the yield was 25.6%.¹H NMR (400 MHz, CDCl₃) *δ* 7.98 (d, *J =* 8.3 Hz, 2H), 7.93 (d, *J =* 8.6 Hz, 2H), 7.61 (s, 1H), 7.57 (d, *J =* 8.3 Hz, 2H), 7.45 (d, *J =* 8.6 Hz, 2H), 3.83 (s, 2H), 3.46 (s, 2H), 2.52 (s, 2H), 2.38 (s, 2H), 2.34 (s, 3H); ¹³C NMR (100 MHz, CDCl₃) *δ* 168.99, 161.37, 145.83, 139.18, 133.42, 131.43, 131.30, 129.24, 128.23, 127.26, 127.20, 127.07, 126.28, 55.38, 54.78, 47.78, 46.17, 42.30; HRMS calcd for (C₂₂H₂₁ON₅ClS + H)⁺ 438.1150, found 438.1145.

### Example 39: (4-(5-(3-fluoro-4-(trifluoromethyl)phenyl)imidazo[2,1-b][1,3,4] thiadiazol-2-yl)phenyl)(4-methylpiperazin-1-yl)methanone (compound 23)

From intermediate 3(200 mg, 0.5 mmol) and 3-fluoro-4-trifluoromethylphenylboronic acid (210 mg, 1 mmol), 78 mg of white solid was obtained according to the synthesis method of compound 1, and the yield was 31.8%.¹H NMR (600 MHz, CDCl₃) *δ* 8.00 - 7.89 (m, 3H), 7.83 (d, *J =* 8.2 Hz, 1H), 7.73 (d, *J* = 2.2 Hz, 1H), 7.68 (t, *J =* 7.9 Hz, 1H), 7.57 (m, 2H), 3.82 (s, 2H), 3.45 (s, 2H), 2.51 (s, 2H), 2.37 (m, 2H), 2.33 (m, 3H); ¹³C NMR (150 MHz, CDCl₃) *δ* 168.86, 162.14, 147.09, 139.44, 134.28, 133.77, 133.07, 131.06, 128.29, 128.16, 127.25, 119.95, 112.59, 55.31, 54.72, 47.70, 46.09, 42.22; HRMS calcd for (C₂₃H₂₀ON₅F₄S + H)⁺ 490.1319, found 490.1307.

### Example 40: 2-fluoro-4-(2-(4-(4-methylpiperazine-1-carbonyl)phenyl) imidazo[2,1-b][1,3,4]thiadiazol-5-yl)benzonitrile (compound 24)

Using intermediate 3 (200 mg, 0.5 mmol) and 3-fluoro-4-cyanophenylboronic acid (160 mg, 1 mmol) as raw materials, 66 mg white solid was obtained according to the synthesis method of compound 1, and the yield was 29.6%.¹H NMR (600 MHz, CDCl₃) *δ* 7.99 (d, *J =* 8.3 Hz, 2H), 7.96 (dd, *J =* 10.4, 1.6 Hz, 1H), 7.85 (dd, *J =* 8.1, 1.6 Hz, 1H), 7.78 (s, 1H), 7.69 (dd, *J =* 8.2, 6.7 Hz, 1H), 7.59 (d, *J =* 8.3 Hz, 2H), 3.82 (s, 2H), 3.45 (s, 2H), 2.51 (s, 2H), 2.37 (s, 2H), 2.33 (s, 3H); ¹³C NMR (150 MHz, CDCl₃) *δ* 168.79, 162.50, 147.71, 139.60, 135.22, 134.00, 130.87, 128.34, 127.28, 120.44, 114.11, 111.73, 55.33, 54.71, 47.70, 46.10, 42.22; HRMS calcd for (C₂₃H₂₀ON₆FS + H)⁺ 477.1398, found 477.1400.

### Example 41: (4-methylpiperazin-1-yl)(4-(5-(4-nitrophenyl)imidazo[2,1-b][1, 3,4]thiadiazol-2-yl)phenyl)methanone (compound 25)

From intermediate 3 (200 mg, 0.5 mmol) and 4-nitrophenylboronic acid (170 mg, 1 mmol), 88 mg white solid was obtained according to the synthesis method of compound 1, and the yield was 39.3%.¹H NMR (400 MHz, CDCl₃) *δ* 8.35 (d, *J =* 8.9 Hz, 2H), 8.20 (d, *J =* 8.6 Hz, 2H), 8.02 (d, *J =* 8.0 Hz, 2H), 7.83 (s, 1H), 7.61 (d, *J =* 8.0 Hz, 2H), 3.84 (s, 2H), 3.48 (s, 2H), 2.53 (s, 2H), 2.39 (s, 2H), 2.35 (s, 3H); ¹³C NMR (100 MHz, CDCl₃) *δ* 167.43, 160.80, 146.13, 145.11, 138.12, 133.23, 132.28, 129.64, 126.91, 125.86, 124.84, 123.44, 123.17, 53.92, 53.37, 46.36, 44.72, 40.83; HRMS calcd for (C₂₂H₂₁O₃N₆S + H)⁺ 449.1390, found 449.1386.

### Example 42: (4-(5-(2,4-difluorophenyl)imidazo[2,1-b][1,3,4]thiadiazol-2-yl) phenyl)(4-methylpiperazin-1-yl)methanone (compound 26)

Using intermediate 3 (200 mg, 0.5 mmol) and 3, 4-difluoro-phenylboronic acid (160 mg, 1 mmol) as raw materials, 78 mg white solid was obtained according to the synthesis method of compound 1, and the yield was 35.6%.¹H NMR (400 MHz, CDCl₃) *δ* 8.39 - 7.90 (m, 3H), 7.75 - 7.50 (m, 3H), 7.25 (s, 1H) 7.09 - 6.92 (m, 1H), 3.81 (s, 2H), 3.44 (s, 2H), 2.50 (s, 2H), 2.36 (s, 3H), 2.32 (d, *J* = 1.0 Hz, 5H); ¹³C NMR (100 MHz, CDCl₃) *δ* 168.86, 161.68, 161.11, 144.13, 139.23, 139.09, 134.16, 133.71, 131.31, 131.05, 128.09, 127.17, 111.78, 104.67, 55.23, 54.67, 47.64, 46.03, 42.18; HRMS calcd for (C₂₂H₂₀ON₅F₂S + H)⁺ 440.1351, found 440.1348.

### Example 43: 5-(2-(4-(4-methylpiperazine-1-carbonyl)phenyl)imidazo[2,1-b] [1,3,4]thiadiazol-5-yl)picolinonitrile (compound 27)

A white solid 76 mg was obtained from intermediate 3 (200 mg, 0.5 mmol) and 2-cyano-5-pyridine boronic acid (150 mg, 1 mmol) according to the synthesis method of compound 1 in a yield of 35%.¹H NMR (400 MHz, CDCl₃) ð 9.40 (d, *J =* 2.3 Hz, 1H), 8.46 (ddd, *J =* 8.2*,* 2.3, 0.8 Hz, 1H), 7.99 (d, *J =* 8.5 Hz, 2H), 7.82 - 7.76 (m, 2H), 7.59 (d, *J =* 8.6 Hz, 2H), 3.83 (s, 2H), 3.46 (s, 2H), 2.52 (s, 2H), 2.38 (s, 2H), 2.34 (s, 3H); ¹³C NMR (100 MHz, CDCl₃) *δ* 168.70, 162.68, 147.88, 146.78, 139.59, 133.72, 131.37, 130.72, 128.62, 128.27, 127.80, 127.20, 123.53, 117.34, 55.20, 54.60, 47.63, 46.01, 42.15; HRMS calcd for (C₂₂H₂₀ON₇S + H)⁺ 430.1445, found 430.1435.

### Example 44: (4-(5-(6-fluoropyridin-3-yl)imidazo[2,1-b][1,3,4]thiadiazol-2-yl) phenyl)(4-methylpiperazin-1-yl)methanone (compound 28)

From intermediate 3(200 mg, 0.5 mmol) and 2-fluoro-5-pyridine boronic acid (140 mg, 1 mmol), 65 mg white solid was obtained according to the synthesis method of compound 1, and the yield was 30.8%.¹H NMR (400 MHz, CDCl₃) *δ* 8.88 (d, *J =* 2.5 Hz, 1H), 8.36 (ddd, *J =* 8.6, 7.5, 2.5 Hz, 1H), 7.98 (d, *J =* 8.5 Hz, 2H), 7.65 (s, 1H), 7.58 (d, *J =* 8.5 Hz, 2H), 7.07 (ddd, *J=* 8.6, 3.1, 0.7 Hz, 1H), 3.83 (s, 2H), 3.46 (s, 2H), 2.50 (d, *J =* 5.9 Hz, 2H), 2.38 (s, 2H), 2.34 (s, 3H); ¹³C NMR (100 MHz, CDCl₃) *δ* 168.83, 163.91, 161.87, 161.52, 146.18, 144.13, 139.25, 137.46, 131.39, 131.09, 128.18, 127.12, 124.19, 122.98, 110.08, 109.71, 55.21, 54.65, 47.60, 46.00, 42.13; HRMS calcd for (C₂₁H₂₀ON₆FS + H)⁺ 423.1398, found 423.1393.

### Example 45: (4-methylpiperazin-1-yl)(4-(5-(6-(trifluoromethyl)pyridin-3-yl) imidazo[2,1-b][1,3,4]thiadiazol-2-yl)phenyl)methanone (compound 29)

75 mg white solid was obtained from intermediate 3 (200 mg, 0.5 mmol) and 2-(trifluoromethyl) pyridyl-5-boronic acid (190 mg, 1 mmol) according to the synthesis method of compound 1, and the yield was 31.8%.¹H NMR (400 MHz, CDCl₃) *δ* 9.35 (dd, *J =* 2.3, 0.9 Hz, 1H), 8.45 (dd, *J=* 8.2, 2.3 Hz, 1H), 7.98 (d, *J =* 8.3 Hz, 2H), 7.83 (s, 1H), 7.78 (dd, *J =* 8.2, 0.8 Hz, 1H), 7.58 (d, *J =* 8.4 Hz, 2H), 3.82 (s, 2H), 3.45 (s, 2H), 2.51 (s, 2H), 2.38 (s, 2H), 2.33 (s, 3H); ¹³C NMR (100 MHz, CDCl₃) *δ* 168.75, 162.34, 147.31, 145.88, 139.48, 132.99, 132.37, 130.88, 128.22, 127.46, 127.14, 123.86, 120.66, 55.26, 54.61, 47.64, 46.03, 42.16; HRMS calcd for (C₂₂H₂₀ON₆F₃S + H)⁺ 473.1366, found 473.1355.

### Example 46: 4-(2-(4-(pyrrolidine-1-carbonyl)phenyl)imidazo[2,1-b][1,3,4]th iadiazol-5-yl)benzonitril (compound 30)

A mixture of intermediate 8 (65 mg, 0.18 mmol), EDCI (69 mg, 0.36mmol), and NHS (42 mg, 0.36mmol) with 10 mL DMF was stirred for 10 h at room temperature. Then pyrrolidine (65µL, 0.9 mmol) was added and the reaction was continued for another 1 h. The reaction of the raw material was completely detected by TLC, quenched with water, extracted three times with DCM, the organic layer was washed three times with saturated NaCl solution, dried with anhydrous Mg(SO₄)₂, filtered, and the filtrate spun dry. Column chromatography purification (DCM:MeOH=15:1) was performed to give a white solid (50mg) in 68% yield.¹H NMR (400 MHz, DMSO-d6) *δ* 8.24 (d, *J =* 8.3 Hz, 2H), 8.08 - 8.02 (m, 3H), 7.91 (d, *J =* 8.3 Hz, 2H), 7.71 (d, *J =* 8.1 Hz, 2H), 3.46 (t, *J =* 6.6 Hz, 2H), 3.37 (t, *J =* 6.2 Hz, 2H), 1.89-1.76 (m, 4H). ¹³C NMR (100 MHz, DMSO-d6) *δ* 167.5, 162.5, 147.3, 141.0, 134.3, 133.5, 132.8, 130.8, 128.7, 127.4, 126.1, 124.9, 119.4, 109.7, 49.3, 46.6, 26.5, 24.5.HRMS calculated for (M+H)+ 400.1227, found 400.1217.

### Example 47:2-(pyrrolidin-1-yl)ethyl 4-(5-(4-cyanophenyl)imidazo[2,1-b] [1, 3,4]thiadiazol-2-yl)benzoate (compound 31)

From 1-(2-hydroxyethyl) pyrrolidine (104 mg, 0.9 mmol) and intermediate 8(65 mg, 0.18 mmol), a white solid was obtained according to the synthesis method of compound 30 in a yield of 60%.¹H NMR (400 MHz, DMSO-d6) *δ* 8.29 (d, *J =* 8.2 Hz, 2H), 8.16 - 8.09 (m, 3H), 8.05 (d, *J =* 8.2 Hz, 2H), 7.96 (d, *J =* 8.2 Hz, 2H), 4.42 (dd, *J =* 12.5, 6.3 Hz, 2H), 2.80 (t, *J =* 6.8 Hz, 2H), 2.51 - 2.47 (m, 4H), 1.64 - 1.74 (m, 4H). HRMS calculated for (M+H)+ 443.1449, found 443.1452

### Example 48: 2-(pyrrolidin-1-yl)ethyl 4-(5-(4-cyanophenyl)imidazo[2,1-b][1, 3,4]thiadiazol-2-yl)benzoate (compound 32)

From n-pentanthiol (91.8 mg, 0.9 mmol) and intermediate 8 (65 mg, 0.18 mmol), a yellow solid was obtained according to the synthesis method of compound 30 in a yield of 53%.¹H NMR (400 MHz, DMSO-d6) *δ* 8.08 (d, *J =* 8.4 Hz, 2H), 8.04 (d, *J =* 9.7 Hz, 3H), 8.01 (d, *J =* 8.4 Hz, 2H), 7.96 (d, *J* = 8.4 Hz, 2H), 3.53 (t, *J =* 5.1 Hz, 1H), 1.97 (dd, *J =* 11.5, 6.1 Hz, 2H), 1.82 -1.77 (m, 4H), 1.32 (p, *J* = 6.5 Hz, 2H),0.8 (p, *J =* 6.5 Hz, 2H). HRMS calculated for (M+H)+ 432.1176, found 432.1163.

### Example 49: 2-morpholinoethyl 4-(5-(4-cyanophenyl)imidazo[2,1-b][1, 3,4]thiadiazol-2-yl)benzoate (compound 33)

A yellow solid was obtained from N-(3-hydroxypropyl) morpholine (131 mg, 0.9 mmol) and intermediate 8 (65 mg, 0.18 mmol) according to the synthesis method of compound 30 in a yield of 45%.¹H NMR (400 MHz, DMSO-d6) *δ* 8.17 (d, *J =* 8.4 Hz, 2H), 8.03 - 8.09 (m, 3H), 7.82 (d, *J =* 8.4 Hz, 2H), 7.71 (d, *J =* 8.4 Hz, 2H), 3.50 (t, *J =* 4.3 Hz, 4H), 3.35-3.30 (m, 2H), 2.41-2.31 (m, 2H), 1.72 (p, *J =* 7.0 Hz, 2H).HRMS calculated for (M+H)+ 473.1528, found 473.1542.

### Example 50: 5-hydroxypentyl 4-(5-(4-cyanophenyl)imidazo[2,1-b] [1, 3, 4]thiadiazol-2-yl)benzoate (compound 34)

From 1, 5-pentanediol (94 mg, 0.9 mmol) and intermediate 8 (65 mg, 0.18 mmol), a yellow solid was obtained as described for compound 30 in a yield of 45%.¹H NMR (400 MHz, DMSO-d6) *δ* 8.19 (d, *J =* 8.5 Hz, 2H), 8.10 (d, *J =* 9.5 Hz, 3H), 7.96 (d, *J =* 8.4 Hz, 2H), 7.87 (d, *J =* 8.5 Hz, 2H), 3.21 (t, *J =* 5.1 Hz, 2H), 3.41 (dd, *J =* 11.6, 6.3 Hz, 2H), 1.69 (dd, *J =* 12.9, 6.7 Hz, 2H), 1.31-1.22 (m, 2H), 1.06 (m, 2H).HRMS calculated for (M+H)+ 432.1347, found 432.1356.

### Example 51: S-(2-hydroxyethyl)4-(5-(4-cyanophenyl)imidazo[2,1-b][1, 3,4]thiadiazol-2-yl)benzothioate (compound 35)

From 2-mercaptoethanol (70.2 mg, 0.9 mmol) and intermediate 8(65 mg, 0.18 mmol), a yellow solid was obtained according to the synthesis method of compound 30 in a yield of 35%.¹H NMR (400 MHz, DMSO-*d6*)*δ*8.18 (d, *J =* 8.4 Hz, 2H), 8.10-8.01 (m, 3H), 7.90 (d, *J =* 8.5 Hz, 2H), 7.81 (d, *J* = 8.4 Hz, 2H), 4.80 (t, *J =* 5.6 Hz, 1H), 3.66 (q, *J* = 6.0 Hz, 2H), 3.28 (dd, *J* = 11.7, 5.8 Hz, 2H).HRMS calculated for (M+H)+ 406.0622, found 406.0635.

### Example 52: 4-(5-(4-cyanophenyl)imidazo[2,1-b][1,3,4]thiadiazol-2- yl)-N- (2 -(dimethylamino)ethyl)benzamide (compound 36)

From N, N-dimethyl-1, 2-ethylenediamine (80 mg, 0.9 mmol) and intermediate 8 (65 mg, 0.18 mmol), a yellow solid was obtained according to the synthesis method of compound 30 in a yield of 35%.¹H NMR (400 MHz, DMSO-d6) *δ* 8.63 (s, 1H), 8.26 (d, *J =* 7.8 Hz, 2H), 8.10 (d, *J =* 10.0 Hz, 3H), 8.03 (d, *J =* 7.5 Hz, 2H), 7.94 (d, *J =* 7.8 Hz, 2H), 3.37 (t, *J =* 4.0 Hz, 2H), 2.43 (t, *J =* 8.0 Hz, 2H), 2.20 (s, 6H). ¹³C NMR (100 MHz, DMSO-d6) *δ* 162.5, 147.4, 138.1, 134.4, 133.5, 132.9, 132.1, 128.9, 127.5, 126.1, 125.1, 119.5, 109.8, 100.00, 58.6, 45.8, 38.1.HRMS calculated for (M+H)+ 417.192, found, 417.1488.

### Example 53: 4-(5-(4-cyanophenyl)imidazo[2,1-b][1,3,4]thiadiazol-2-yl)-N -(3-morpholinopropyl)benzamide (compound 37)

A yellow solid was obtained from N-(3-aminopropyl) morpholine (130 mg, 0.9 mmol) and intermediate 8 (65 mg, 0.18 mmol) according to the synthesis method of compound 30 in 47% yield.¹H NMR (400 MHz, DMSO-d6) *δ* 8.72 (t, *J* = 5.4 Hz, 1H), 8.27 (d, *J =* 8.4 Hz, 2H), 8.08 - 8.14 (m, 3H), 8.04 (d, *J = 8.4* Hz, 2H), 7.95 (d, *J =* 8.4 Hz, 2H), 3.58 (t, *J =* 4.3 Hz, 4H), 3.35 - 3.30 (m, 2H), 2.41 - 2.31 (m, 6H), 1.72 (p, *J =* 7.0 Hz, 2H). ¹³C NMR (100 MHz, DMSO-d6) *δ 165.5,* 162.4, 147.3, 138.1, 134.3, 133.4, 132.8, 131.9, 128.7, 127.4, 126.0, 124.9, 119.4, 109.7, 66.7, 56.5, 53.8, 38.4, 26.4.HRMS calculated for (M+H)+ 473.1730, found 473.1742.

### Example 54: 4-(5-(4-cyanophenyl)imidazo[2,1-b][1,3,4]thiadiazol-2-yl)-N-(2-(pyrrolidin-1-yl)ethyl)benzamide (compound 38)

From 1-(2-aminoethyl) pyrrolidine (102 mg, 0.9 mmol) and intermediate 8(65 mg, 0.18 mmol), a white solid was obtained as described for compound 30 in a yield of 62%.¹H NMR (400 MHz, *DMSO-d6) δ* 8.69 (t, *J =* 5.2 Hz, 1H), 8.29 (d, *J =* 8.2 Hz, 2H), 8.16-8.09 (m, 3H), 8.05 (d, *J =* 8.2 Hz, 2H), 7.96 (d, *J =* 8.2 Hz, 2H), 3.42 (dd, *J =* 12.5, 6.3 Hz, 2H), 2.60 (t, *J =* 6.8 Hz, 2H), 2.51-2.47 (m, 4H), 1.64-1.74 (m, 4H). ¹³C NMR (100 MHz, DMSO-d6) *δ* 165.5, 162.4, 147.2, 138.0, 134.4, 133.5, 132.9, 132.0, 128.8, 127.4, 126.1, 125.0, 119.4, 109.7, 55.3, 54.2, 23.6.HRMS calculated for (M+H)+ 443.1649, found 473.1642.

### Example 55: 4-(5-(4-cyanophenyl)imidazo[2,1-b][1,3,4]thiadiazol-2-yl)-N-(3-hydroxypropyl)benzamide (compound 39)

From 3-aminopropanol (68 mg, 0.9 mmol) and intermediate 8 (65 mg, 0.18 mmol), a yellow solid was obtained as described for compound 30 in a yield of: 28%.¹H NMR (400 MHz, DMSO-d6) δ 8.70 (t, *J =* 5.4 Hz, 1H), 8.28 (d, *J =* 8.4 Hz, 2H), 8.12 (d, *J =* 9.7 Hz, 3H), 8.05 (d, *J =* 8.4 Hz, 2H), 7.96 (d, *J =* 8.4 Hz, 2H), 4.53 (t, *J =* 5.1 Hz, 1H), 3.50 (dd, *J =* 11.5, 6.1 Hz, 2H), 3.40 - 3.35 (m, 2H), 1.72 (p, *J =* 6.5 Hz, 2H)..¹³C NMR (100 MHz, DMSO-d6) *δ* 165.5, 162.3, 147.2, 138.1, 134.3, 133.4, 132.8, 131.9, 128.7, 127.3, 126.0, 124.9, 119.4, 109.7, 59.1, 37.3, 32.8.HRMS calculated for (M+H)+ 404.1176, found 404.1173.

### Example 56: 4-(5-(4-cyanophenyl)imidazo[2,1-b][1,3,4]thiadiazol-2-yl)-N-(2-hydroxyethyl)benzamide (compound 40)

From ethanolamine (55 mg, 0.9 mmol) and intermediate 8(65 mg, 0.18 mmol), a yellow solid was obtained as described for compound 30 in a yield of 36%.¹H NMR (400 MHz, DMSO-d6) *δ* 8.71 (t, *J =* 5.5 Hz, 1H), 8.28 (d, *J =* 8.4 Hz, 2H), 8.15 - 8.10 (m, 3H), 8.07 (d, *J =* 8.5 Hz, 2H), 7.96 (d, *J =* 8.4 Hz, 2H), 4.80 (t, *J =* 5.6 Hz, 1H), 3.56 (q, *J =* 6.0 Hz, 2H), 3.38 (dd, *J =* 11.7, 5.8 Hz, 2H). ¹³C NMR (100 MHz, DMSO-d6) *δ* 165.7, 162.4, 147.3, 138.0, 134.3, 133.5, 132.9, 131.9, 128.8, 127.3, 126.0, 124.9, 119.4, 109.7, 60.1, 42.8.HRMS calculated for (M+H)+ 390.1019, found 390.1018.

### Example 57: 4-(2-(4-(2-methylpiperidine-1-carbonyl)phenyl)imidazo[2,1-b][1, 3,4]thiadiazol-5-yl)benzonitrile (compound 41)

From 2-methylpiperidine (89 mg, 0.9 mmol) and intermediate 8(65 mg, 0.18 mmol), a yellow solid was obtained as described for compound 30 in a yield of: 46%.¹H NMR (400 MHz, DMSO-d6) *δ* 8.28 (d, *J =* 8.5 Hz, 2H), 8.09 (d, *J =* 8.6 Hz, 3H), 7.96 (d, *J =* 8.5 Hz, 2H), 7.60 (d, *J =* 8.1 Hz, 2H), 5.03 - 3.59 (m, 2H), 2.99 (d, *J =* 37.9 Hz, 1H), 1.75-1.33 (m, 6H), 1.22 (d, *J =* 6.8 Hz, 3H).¹³C NMR (100 MHz, DMSO-d6) *δ* 168.4, 162.5, 147.2, 140.9, 134.3, 133.4, 132.9, 130.3, 127.8, 127.7, 126.0, 124.9, 119.4, 109.7, 30.2, 25.9, 18.9. HRMS calculated for (M+H)+ 428.1540, found 428.1533.

### Example 58: 4-(5-(4-cyanophenyl)imidazo[2,1-b][1,3,4]thiadiazol-2-yl)-N-(5-hydroxypentyl)benzamide (compound 42)

From 5-amino-1-amyl alcohol (93 mg, 0.9 mmol) and intermediate 8 (65 mg, 0.18 mmol), a yellow solid was obtained according to the synthesis method of compound 30 in yield: 48%.¹H NMR (400 MHz, DMSO-d6) *δ* 8.70 (t, *J =* 5.4 Hz, 1H), 8.29 (d, *J =* 8.5 Hz, 2H), 8.13 (d, *J =* 9.5 Hz, 3H), 8.06 (d, *J =* 8.4 Hz, 2H), 7.97 (d, *J =* 8.5 Hz, 2H), 4.39 (t, *J =* 5.1 Hz, 1H), 3.41 (dd, *J =* 11.6, 6.3 Hz, 2H), 3.29 (dd, *J =* 12.9, 6.7 Hz, 2H), 1.61 - 1.52 (m, 2H), 1.46 (dd, *J =* 13.9, 6.8 Hz, 2H), 1.40 - 1.31 (m, 2H).¹³C NMR (100 MHz, DMSO-d6) *δ* 165.4, 162.4, 147.3, 138.2, 134.3, 133.5, 132.8, 131.9, 128.8, 127.4, 126.0, 125.0, 119.4, 109.7, 61.1, 32.7, 29.5, 23.6. HRMS calculated for (M+H)⁺ 432.1489, found 432.1479.

### Example 59: 4-(6-(4-(morpholine-4-carbonyl)phenyl)imidazo[2,1-b]thiazol- -3-yl)benzonitrile (compound 43)

Intermediate 4 (500 mg, 1.6 mmol), 4-cyanophenylacetylene (240 mg, 1.9 mmol), potassium tert-butyl alcohol (540 mg, 4.8 mmol), and DMF (40 mL) were added to a 50 mL tomato bottle. The reaction was carried out for 6 h at room temperature under magnetic stirring. The reaction of the raw material was complete as determined by TLC. Dichloromethane and water were added to the reaction flask, transferred to a separating funnel, shaken to rest, and the organic layer was separated. The aqueous layer was extracted twice with dichloromethane, the organic layer was merged, the organic layer was washed twice with saturated NaCl solution, anhydrous Mg(SO₄)₂ was dried, filtered, the filtrate was dried, and purified by column chromatography (CH₂Cl₂:CH₃OH=50:1). White solid (200 mg) was obtained in 30.2% yield.¹H NMR (400 MHz, CDCl₃) *δ* 7.94 (s, 1H), 7.89 (m, 1H), 7.87 (m, 3H), 7.81 (m, 2H), 7.46 (d, *J =* 8.3 Hz, 2H), 6.98 (s, 1H), 3.76 (s, 6H), 3.52 (s, 2H); ¹³C NMR (100 MHz, CDCl₃) *δ* 170.29, 150.45, 147.16, 135.21, 134.26, 133.86, 133.28, 130.78, 127.84, 127.29, 125.39, 117.98, 113.43, 111.64, 107.75, 66.91, 48.08, 42.57; HRMS calcd for (C₂₃H₁₉O₂N₆S + H)⁺ 415.1223, found 415.1226.

### Example 60: (4-(3-(4-fluorophenyl)imidazo[2,1-b]thiazol-6-yl)phenyl)(morp-holino)methanone (compound 44)

A white solid (170 mg) was obtained from intermediate 4 (500 mg, 1.6 mmol) and 4-fluorophenylacetylene (230 mg, 1.9 mmol) according to the synthesis method of compound 30 in 26% yield.¹H NMR (400 MHz, CDCl₃) *δ* 7.88 (m, 3H), 7.65 (dd, *J =* 8.8, 5.1 Hz, 2H), 7.45 (d, *J =* 8.3 Hz, 2H), 7.24 (m, 2H), 6.77 (s, 1H), 3.75 (s, 6H), 3.51 (s, 2H); ¹³C NMR (100 MHz, CDCl₃) *δ* 170.36, 164.64, 162.14, 150.41, 146.96, 135.73, 133.99, 131.57, 129.04, 128.96, 127.78, 126.03, 126.00, 125.25, 116.76, 116.54, 108.72, 107.71, 66.93, 48.10, 42.46; HRMS calcd for (C₂₂H₁₉O₂N₃FS + H)⁺ 408.1177, found 408.1178.

### Example 61: (4-(3-(4-methoxyphenyl)imidazo[2,1-b]thiazol-6-yl)phenyl)(morpholino)methanone (compound 45)

A white solid (280 mg) was obtained from intermediate 4 (500 mg, 1.6 mmol) and 4-methoxyphenylacetylene (250 mg, 1.9 mmol) according to the synthesis method of compound 30, and the yield was 41.7%.¹H NMR (400 MHz, DMSO-*d*₆) *δ* 8.56 (s, 1H), 8.00 (d, *J =* 8.2 Hz, 2H), 7.77 (d, *J =* 8.4 Hz, 2H), 7.44 (d, *J =* 8.2 Hz, 2H), 7.35 (d, *J =* 1.2 Hz, 1H), 7.13 (d, *J =* 8.5 Hz, 2H), 3.85 (s, 3H), 3.73 - 3.47 (m, 8H); ¹³C NMR (100 MHz, DMSO-*d*₆) *δ* 169.53, 162.85, 161.52, 160.58, 149.93, 146.21, 135.86, 134.35, 132.17, 128.84, 128.07, 125.20, 122.03, 115.17, 110.04, 108.62, 67.19, 66.21, 55.87, 45.58; HRMS calcd for (C₂₃H₂₂O₃N₃S + H)⁺ 420.1376, found 420.1370.

### Example 62: morpholino(4-(3-(4-(trifluoromethyl)phenyl)imidazo[2,1-b]thiazol-6-yl)phenyl)methanone (compound 46)

A white solid (220 mg) was obtained from intermediate 4 (500 mg, 1.6 mmol) and 4-trifluoromethylphenylacetylene (320 mg, 1.9 mmol) according to the synthesis method of compound 30, and the yield was 30.1%.¹H NMR (400 MHz, CDCl₃) *δ* 7.93 (s, 1H), 7.89 (d, *J =* 8.3 Hz, 2H), 7.82 (m, 4H), 7.46 (d, *J =* 8.3 Hz, 2H), 6.93 (s, 1H), 3.76 (s, 6H), 3.52 (s, 2H); ¹³C NMR (100 MHz, CDCl₃) *δ* 170.31, 150.45, 147.19, 135.53, 134.15, 133.22, 131.19, 127.81, 127.22, 126.53, 125.30, 110.52, 107.73, 66.93, 48.32, 42.60; HRMS calcd for (C₂₃H₁₉O₂N₃S + H)⁺ 458.1145, found 458.1144.

### Example 63: (4-(3-(4-chlorophenyl)imidazo[2,1-b]thiazol-6-yl)phenyl)(morpholino)methanone (compound 47)

A white solid (265 mg) was obtained from intermediate 4 (500 mg, 1.6 mmol) and 4-chlorophenylacetylene (260 mg, 1.9 mmol) according to the synthesis method of compound 30 in 39.1% yield.¹H NMR (400 MHz, CDCl₃) *δ* 7.88 (d, *J =* 4.4 Hz, 2H), 7.85 (s, 1H), 7.59 (d, *J =* 8.6 Hz, 2H), 7.51 (d, *J =* 8.6 Hz, 2H), 7.44 (d, *J =* 8.4 Hz, 2H), 6.80 (s, 1H), 3.73 (s, 6H), 3.51 (s, 2H); ¹³C NMR (100 MHz, CDCl₃) *δ* 170.32, 150.40, 146.95, 135.77, 135.66, 134.01, 131.44, 129.71, 128.17, 127.78, 125.24, 109.22, 107.80, 66.92, 48.30, 42.71; HRMS calcd for (C₂₂H₁₉O₂N₃ClS + H)⁺ 424.0881, found 424.0870.

### Example 64: 4-(6-(4-(4-methylpiperazine-1-carbonyl)phenyl)imidazo[2,1-b] thiazol-3-yl)benzonitrile ( compound 48)

A white solid (280 mg) was obtained from intermediate 5 (500 mg, 1.5 mmol) and 4-cyanophenyl acetylene (230 mg, 1.8 mmol) according to the synthesis method of compound 30, and the yield was 43.7%.¹H NMR (400 MHz, CDCl₃) *δ* 7.92 (s, 1H), 7.88 - 7.84 (m, 4H), 7.80 (d, *J=* 8.7 Hz, 2H), 7.45 (d, *J =* 8.6 Hz, 2H), 6.97 (s, 1H), 3.80 (s, 2H), 3.49 (s, 2H), 2.48 (s, 2H), 2.36 (s, 2H), 2.32 (s, 3H); ¹³C NMR (100 MHz, CDCl₃) *δ* 170.15, 150.41, 147.48, 135.24, 134.71, 133.99, 133.26, 130.77, 127.77, 127.26, 125.26, 118.00, 113.37, 111.39, 107.63, 55.05, 47.56, 45.96, 41.99; HRMS calcd for (C₂₄H₂₂ON₅S + H)⁺ 428.1540, found 428.1535.

### Example 65: (4-(3-(4-fluorophenyl)imidazo[2,1-b]thiazol-6-yl)phenyl)(4-methylpiperazin-1-yl)methanone (compound 49)

The white solid (220 mg) was obtained from intermediate 5 (500 mg, 1.5 mmol) and 4-fluorophenylacetylene (220 mg, 1.8 mmol) according to the synthesis method of compound 30, and the yield was 34.9%.¹H NMR (400 MHz, CDCl₃) *δ* 7.85 (d, *J =* 8.0 Hz, 3H), 7.64 (dd, *J =* 8.8, 5.1 Hz, 2H), 7.43 (d, *J =* 8.5 Hz, 2H), 7.22 (d, *J* = 8.8 Hz, 2H), 6.75 (s, 1H), 3.79 (s, 2H), 3.49 (s, 2H), 2.48 (s, 2H), 2.35 (s, 2H), 2.31 (s, 3H); ¹³CNMR (100 MHz, CDCl₃) *δ* 170.24, 164.61, 162.11, 150.35, 147.03, 135.51, 134.48, 131.55, 129.02, 127.71, 126.04, 125.17, 116.51, 108.65, 107.65, 55.21, 54.82, 47.70, 46.02, 42.15; HRMS calcd for (C₂₃H₂₂ON₄FS + H)⁺ 421.1493, found 421.1490.

### Example 66: (4-methylpiperazin-1-yl)(4-(3-(4-(trifluoromethyl)phenyl)imidazo[2,1-b]thiazol-6-yl)phenyl)methanone (compound 50)

A white solid (300 mg) was obtained from intermediate 5 (500 mg, 1.5 mmol) and 4-trifluoromethylphenylacetylene (310 mg, 1.8 mmol) according to the synthesis method of compound 30 in 42.5% yield.¹H NMR (400 MHz, CDCl₃) *δ* 7.92 (s, 1H), 7.86 (d, *J =* 8.6 Hz, 2H), 7.80 (m, 4H), 7.44 (d, *J =* 8.6 Hz, 2H), 6.91 (s, 1H), 3.79 (s, 2H), 3.49 (s, 2H), 2.47 (s, 2H), 2.36 (s, 2H), 2.32 (s, 3H); ¹³C NMR (100 MHz, CDCl₃) *δ* 168.66, 148.87, 145.72, 133.82, 133.04, 131.68, 129.63, 126.19, 125.66, 124.97, 123.68, 108.92, 106.15, 53.55, 46.07, 44.43, 40.52; HRMS calcd for (C₂₄H₂₂ON₄F_{3S} + H)⁺ 471.1461, found 471.1452.

### Example 67: (4-(3-(4-chlorophenyl)imidazo[2,1-b]thiazol-6-yl)phenyl)(4-m-ethylpiperazin-1-yl)methanone (compound 51)

The white solid (264 mg) was obtained from intermediate 5 (500 mg, 1.5 mmol) and 4-chlorophenylacetylene (245 mg, 1.8 mmol) according to the synthesis method of compound 30, and the yield was 40.3%.¹H NMR (400 MHz, CDCl₃) *δ* 7.87 (s, 1H), 7.84 (d, *J =* 8.5 Hz, 2H), 7.58 (d, *J=* 8.7 Hz, 2H), 7.50 (d, *J =* 8.7 Hz, 2H), 7.42 (d, *J =* 8.5 Hz, 2H), 6.78 (s, 1H), 3.78 (s, 2H), 3.48 (s, 2H), 2.46 (s, 2H), 2.35 (s, 2H),2.30 (s, 3H); ¹³C NMR (101 MHz, CDCl₃) *δ* 170.22, 150.37, 147.04, 135.77, 135.45, 134.47, 131.44, 129.70, 128.23, 128.17, 127.70, 125.17, 109.15, 107.72, 55.18, 54.86, 47.68, 46.00, 42.09; HRMS calcd for (C₂₃H₂₂ON₄ClS + H)⁺ 437.1197, found 437.1197.

### Example 68: 6-(4-(4-morpholine piperidine base carbonyl)phenyl)-3-(4-flu orinated phenyl) imidazole [2, 1-b] thiazole compounds (compound 52)

The white solid (280 mg) was obtained from intermediate 6 (500 mg, 1.3 mmol) and 4-fluorophenylacetylene (190 mg, 1.6 mmol) according to the synthesis method of compound 30, and the yield was 43.9%.¹H NMR (400 MHz, CDCl₃) *δ* 7.85 (m, 3H), 7.64 (dd, *J =* 8.8, 5.1 Hz, 2H), 7.42 (d, *J =* 8.6 Hz, 2H), 7.23 (d, *J =* 8.7 Hz,2H), 6.75 (s, 1H), 4.71 (s, 1H), 3.88 (s, 1H), 3.71 (t, *J =* 4.6 Hz, 4H), 3.02 (s, 1H), 2.81 (s, 1H), 2.55 (t, *J =* 4.6 Hz, 4H), 2.43 (m, 1H), 1.96 (s, 1H), 1.82(s, 1H), 1.58 (s, 2H); ¹³C NMR (100 MHz, CDCl₃) *δ* 170.19, 164.62, 162.13, 150.36, 147.07, 135.44, 134.75, 131.56, 129.03, 128.95, 127.53, 126.02, 125.18, 116.75, 116.52, 108.64, 107.62, 67.14, 61.98, 49.81, 46.93, 41.55, 28.96, 28.15; HRMS calcd for (C₂₇H₂₈O₂N₄FS + H)⁺ 491.1912, found 491.1908.

### Example 69: (4-(dimethylamino)piperidin-1-yl)(4-(3-(4-fluorophenyl) imidazo[2,1-b]thiazol-6-yl)phenyl)methanone (compound 53)

A white solid (215 mg) was obtained from intermediate 7 (500 mg, 1.4 mmol) and 4-fluorophenylacetylene (200 mg, 1.7 mmol) according to the synthesis method of compound 30, and the yield was 34.2%.¹H NMR (400 MHz, CDCl₃) *δ* 7.85 (d, *J =* 9.1 Hz, 3H), 7.64 (dd, *J =* 8.8, 5.1 Hz, 2H), 7.42 (d, *J =* 8.4 Hz, 2H), 7.22 (d, *J* = 8.6 Hz, 2H), 6.75 (s, 1H), 4.73 (s, 1H), 3.87 (s, 1H), 3.01 (s, 1H), 2.80 (s, 1H), 2.48 (m, 1H), 2.34 (s, 6H), 2.03 - 1.78 (m, 2H), 1.48 (s, 2H); ¹³C NMR (100 MHz, CDCl₃) *δ* 170.22, 164.61, 162.12, 150.35, 147.05, 135.47, 134.69, 131.56, 129.02, 128.94, 127.54, 126.04, 126.01, 125.18, 116.73, 116.52, 108.64, 107.65, 62.26, 46.93, 41.38, 28.88, 27.86; HRMS calcd for (C₂₅H₂₆ON₄FS + H)⁺ 499.1806, found 499.1795.

### Example 70: (4-(dimethylamino)piperidin-1-yl)(4-(3-(4-(trifluoromethyl)ph- enyl)imidazo [2,1-b]thiazol-6-yl)phenyl)methanone (compound 54)

The white solid (284 mg) was obtained from intermediate 7 (500 mg, 1.4 mmol) and 4-trifluoromethylphenylacetylene (290 mg, 1.7 mmol) according to the synthesis method of compound 30, and the yield was 40.7%.¹H NMR (400 MHz, CDCl₃) *δ* 7.91 (s, 1H), 7.85 (d, *J =* 8.3 Hz, 2H), 7.79 (m, 4H), 7.42 (d, *J =* 8.3 Hz, 2H), 6.91 (s, 1H), 4.73 (s, 1H), 3.87 (s, 1H), 3.01 (s, 1H), 2.80 (s, 1H), 2.49 (m, 1H), 2.34 (s, 6H), 1.97 (s, 2H), 1.49 (m, 2H); ¹³C NMR (100 MHz, CDCl₃) *δ* 170.17, 150.40, 147.25, 135.31, 134.81, 133.22, 131.17, 127.56, 127.20, 126.50, 125.22, 122.33, 110.49, 107.72, 62.26, 46.96, 41.36, 28.88, 27.77; HRMS calcd for (C₂₆H₂₆ON₄F₃S + H)⁺ 499.1774, found 499.1783.

### Example 71: (4-(3-(4-chlorophenyl)imidazo[2,1-b]thiazol-6-yl) phenyl)(4-(dimethylamino)piperidin-1-yl)methanone (compound 55)

A white solid (254 mg) was obtained from intermediate 7 (500 mg, 1.4 mmol) and 4-chlorophenylacetylene (230 mg, 1.7 mmol) according to the synthesis method of compound 30 in a yield of 39%.¹H NMR (400 MHz, CDCl₃) *δ* 7.87 (s, 1H), 7.84 (d, *J =* 8.3 Hz, 2H), 7.59 (d, *J =* 8.6 Hz, 2H), 7.50 (d, *J =* 8.6 Hz, 2H), 7.41 (d, *J =* 8.3 Hz, 2H), 6.79 (s, 1H), 4.73 (s, 1H), 3.88 (s, 1H), 3.01 (s, 1H), 2.79 (s, 1H), 2.52 (m, 1H), 2.35 (s, 6H), 1.88 (s, 2H), 1.49 (s, 2H); ¹³C NMR (100 MHz, CDCl₃) *δ* 170.21, 150.38, 147.08, 135.79, 135.44, 134.66, 131.46, 129.72, 128.25, 128.18, 127.55, 125.19, 109.13, 107.71, 62.29, 46.89, 41.27, 28.70, 27.68; HRMS calcd for (C₂₅H₂₆ON₄ClS + H)⁺ 465.1510, found 465.1501.

### Example 72: Preparation of compounds 56-61

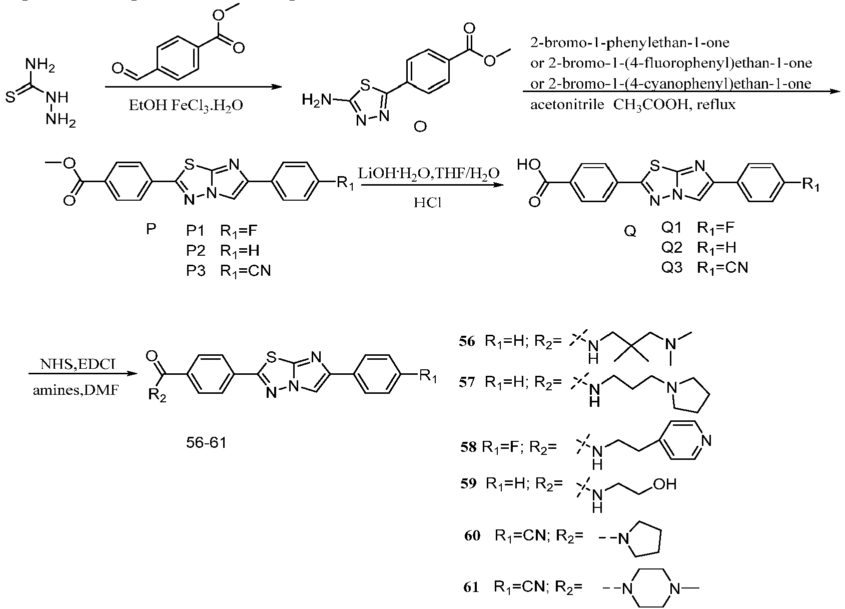

Thiocarbamide was mixed with methyl p-formyl benzoate and stirred in methanol for 2 hours at room temperature. Then FeCl₃·6H₂O dissolved in methanol was added, heated and stirred at 75°C for 30 min, cooled to room temperature, and the solid was filtered and rinsed with methanol until the filtrate was colorless to obtain compound O. Then the mixture with 2-bromo-1 -(4-fluorophenyl) one, 2-bromo-1 -(4-fluorophenyl) one or 2-bromo-1 -(4-cyanophenyl) one was refluxed and stirred in acetonitrile for 12 h. The yellow intermediate was obtained by column chromatography, and the intermediate was refluxed and stirred in acetic acid for 5 h. The mixture was then poured into ice water and filtered to obtain the corresponding compounds P1 or P2 or P3. The mixtures of P1 or P2 or P3, respectively, and LiOH·H₂O were stirred in THF/H₂O(1:1) at room temperature for 12 hours. After adjusting the pH to 2-3 with HCl, the yellow solid Q1 or Q2 or Q3 was filtered and used in the next step without further purification. compounds 51-56 were obtained by adding Q1, Q2, or Q3 with EDCI, NHS, and the corresponding amines to DMF solvent, mixing and stirring, and stirring for 12 h at room temperature.

### N-(3-(dimethyl amine)-2, 2-dimethyl propyl)-4 -(6-phenyl imidazole [2, 1-b][1,3,4] thiadiazole-2-yl) benzamide (compound 56)

Q2 (0.5 mmol), EDCI (1 mmol), NHS (1 mmol), and N,N,2, 2-tetramethyl-1, 3-propylene diamine (2.5 mmol) were added to a round-bottom flask, followed by solvent DMF, and the mixture was stirred for 12 h at room temperature. After the completion of the reaction as judged by TLC, the reaction mixture was diluted with water and extracted three times with ethyl acetate (20 mL). The bound organic layer was washed with water and brine, dried over anhydrous MgSO₄, filtered, and concentrated in vacuo. The crude product was purified by chromatography (DCM:MeOH=10:1) to give a white solid compound 51 in 45% yield.¹H NMR (500 MHz, DMSO-*d*₆) *δ* 8.78 (s, 1H), 8.08 (s, 4H), 7.90 (d, *J =* 7.2 Hz, 2H), 7.43 (t, *J =* 7.7 Hz, 2H), 7.30 (t, *J =* 7.4 Hz, 1H), 3.30 (s, 2H), 3.01 (s, 2H), 2.88 (s, 6H), 1.07 (s, 6H). ¹³C NMR (125 MHz, DMSO-*d*₆) *δ* 166.9, 161.1, 159.1, 158.8, 158.5, 158.1, 145.9, 145.0, 137.3, 133.7, 132.4, 129.1 (d, *J* = 3.4 Hz), 128.0, 127.0, 125.2, 118.8, 116.5, 114.2, 111.9, 111.2, 65.6, 47.2, 46.7, 36.3, 24.2.HRMS calculated for (M+H)+ 434.2009, found 434.2010.

### 4-(6-phenyl imidazole [2, 1-b][1,3,4] thiadiazole-2-yl)-N-(3-(tetrahydropyrrolidyl) propyl) benzamide (compound 57)

From Q2 and 1-(3-aminopropyl) tetrahydropyrrole, a white solid compound 52 was obtained in 49% yield according to the synthesis method of compound 51.¹H NMR (500 MHz, DMSO-*d*₆) *δ* 8.82 - 8.77 (m, 2H), 8.05 (q, *J =* 8.4 Hz, 4H), 7.91 (d, *J =* 7.6 Hz, 2H), 7.43 (t, *J =* 7.7 Hz, 2H), 7.30 (t, *J* = 7.3 Hz, 1H), 3.37 - 3.34 (m, 2H), 2.81 (d, *J =* 86.5 Hz, 6H), 1.83 (s, 6H). ¹³C NMR (125 MHz, DMSO-*d*₆) *δ* 165.8, 160.9, 146.3, 145.02, 137.5, 134.1, 132.3, 129.2, 128.8, 127.9, 127.1, 125.2, 111.1, 53.9, 52.9, 37.4, 26.9, 23.2. HRMS calculated for (M+H)+ 432.1853, found 432.1855.

### 4-(6-(4-fluorinated phenyl) imidazole [2, 1-b][1,3,4] thiadiazole-2-yl)-N-(2-(pyr- idine-4-yl) ethyl) benzamide (compound 5)

From Q1 and 4-(2-aminoethyl) pyridine, a white solid compound 53 was obtained in 31% yield according to the synthesis method of compound 51.¹H NMR (500 MHz, DMSO-*d*₆) *δ* 8.79 (t, *J =* 5.6 Hz, 1H), 8.77 (s, 1H), 8.47 (d, *J =* 5.9 Hz, 2H), 8.05 (d, *J =* 8.4 Hz, 2H), 7.99 (d, *J =* 8.4 Hz, 2H), 7.96 - 7.91 (m, 2H), 7.27 (dd, *J =* 15.5, 6.7 Hz, 4H), 3.57 (dd, *J =* 12.9, 6.9 Hz, 2H), 2.90 (dd, *J =* 11.9, 4.7 Hz, 2H). ¹³C NMR (125 MHz, DMSO-d6) *δ* 165.6, 162.1 (d, *J =* 244.2 Hz), 160.9, 149.8, 148.9, 145.2 (d, *J =* 29.8 Hz), 137.6, 132.2, 130.7 (d, *J=* 2.8 Hz), 128.7, 127.2 (d, *J =* 6.9 Hz), 124.7, 116.1 (d, *J =* 21.6 Hz), 111.0, 109.9, 40.56, 34.57. HRMS calculated for (M+H)+ 444.1289, found 444.1291.

### N-(2-hydroxyethyl)-4-(6-phenyl imidazole group [2, 1-b][1,3,4] thiadiazole-2-yl) benzamide (compound 59)

From Q2 and ethanolamine, a white solid compound 54 was obtained in a yield of 51% according to the synthesis method of compound 51.¹H NMR (500 MHz, DMSO-*d*₆) *δ* 8.78 (s, 1H), 8.66 (t, *J =* 5.5 Hz, 1H), 8.05 (s, 4H), 7.91 (d, *J =* 7.3 Hz, 2H), 7.43 (t, *J =* 7.7 Hz, 2H), 7.30 (t, *J =* 7.3 Hz, 1H), 4.76 (s, 1H), 3.53 (t, *J =* 6.2 Hz, 2H), 3.36 (d, *J =* 5.0 Hz, 2H). ¹³C NMR (125 MHz, DMSO-*d*₆) *δ* 165.7, 160.9, 146.2, 145.0, 137.7, 134.1, 132.2, 129.2, 128.8, 127.9, 127.0, 125.2, 111.2, 60.1, 42.8.HRMS calculated for (M+H)+ 365.1067, found 365.1065.

### 4-(2-(4 -(4-carbonyl pyrrole base-1 hydrogen) phenyl) imidazole [2, 1-b][1,3,4] thiadiazole-6-yl) Cyanobenzene (compound 60)

The white solid compound 55 was obtained from Q3 and tetrahydropyrrole according to the synthesis method of compound 51, and the yield was 51%.¹H NMR (400 MHz, TFA-*d1*) *δ* 8.47 (s, 1H), 8.19 (d, *J =* 7.9 Hz, 2H), 7.92 (d, *J =* 8.8 Hz, 4H), 7.86 (d, *J =* 7.9 Hz, 2H), 3.93 (s, 2H), 3.70 (s, 2H), 2.18 (d, *J =* 6.3 Hz, 2H), 2.10 (d, *J =* 5.9 Hz, 2H).¹³C NMR (400 MHz, TFA-d1) 166.4, 145.3, 137.6, 133.5, 131.1, 130.2, 128.6, 128.1, 126.6, 51.8, 49.3, 24.8, 23.5.

### 4-(2-(4-(4-methyl-carbonyl) piperazine-1 phenyl) imidazole [2, 1-b][1,3,4] thiadiazole-6-yl) Cyanobenzene (compound 61)

The green solid compound 56 was obtained from Q3 and N-methylpiperazine in 63% yield according to the synthesis method of compound 51.¹H NMR (400 MHz, TFA-*d1*) *δ* 8.47 (s, 1H), 8.15 (d, *J =* 8.1 Hz, 2H), 7.96 - 7.89 (m, 4H), 7.73 (d, *J =* 8.1 Hz, 2H), 5.04 (d, *J =* 14.5 Hz, 1H), 4.08 (d, *J =* 14.0 Hz, 1H), 3.85 (d, *J =* 6.8 Hz, 2H), 3.73 (d, *J =* 12.0 Hz, 1H), 3.57 (t, *J =* 13.0 Hz, 1H), 3.32 (dt, *J* = 37.8, 11.3 Hz, 2H), 3.11 (s, 3H).¹³C NMR (100 MHz, DMSO-d6) *δ* 168.2, 161.9, 145.8, 144.3, 139.7, 138.7, 133.3, 130.7, 128.6, 127.5, 125.7, 119.5, 113.4, 109.9, 55.0, 46.0.

### Example 73: Test of the protease inhibitory activity of the compounds of the invention against MNKs

MNK1 and MNK2 kinase inhibition assays were performed using LANCE Ultra kinase activity assay developed by PerkinElmer. Staurosporine (STSP) was used as a positive control in this assay. The test steps are as follows: At 25 °C, 1.00 ng MNK1 or 0.05 ng MNK2 was prepared with different concentrations (10000, 1000, 100, 10, 1 nM) of the tested compound to form a total volume of 10 µL reaction mixture (MNK1: 12.5 nM CREB, 450 µM ATP, 2 mM DTT, 1× buffer; MNK2: 12.5 nM CREB, 100 µM ATP, 2 mM DTT, 1×buffer), and incubation for 60 min. The reaction was terminated by adding 5 µL EDTA/Detection buffer and 5 µL Eu-CREB/Detection buffer. The ratio of HTRF signals at 615 nm and 665 nm was detected after 60 min of incubation. The inhibition rate was calculated by measuring the compounds at 1000 nM. When the inhibition rate > At 75%, 50%, and 25%, they were recorded as +++, ++, and +, respectively, as shown in Table 1.

Then five different concentrations (10000, 1000, 100, 10, 1 nM) of the tested compounds were measured, and the IC₅₀ value was calculated according to the inhibition rate fitting curve. In vitro test of compound of IC₃₀ MNK1/2 results are shown in table 2, including IC₅₀ < 0.05 uM (* * * *), 0.05 to 0.2 uM (* * *), 0.2 to 0.8 uM (* *), and 0.8 1.5 uM (*).

**Table 1. Protease inhibitory activity of 1µM compounds against MNKs**

| Number | Inhibitory activity(1µM) | | Number | Inhibitory activity(1µM) | |
|---|---|---|---|---|---|
| | MNK1 | MNK2 | | MNK1 | MNK2 |
| 1 | +++ | +++ | 29 | + | + |
| 2 | +++ | +++ | 33 | + | + |
| 3 | +++ | +++ | 34 | + | + |
| 4 | +++ | +++ | 35 | + | + |
| 5 | +++ | +++ | 36 | +++ | +++ |
| 6 | +++ | +++ | 37 | +++ | +++ |
| 7 | +++ | +++ | 38 | +++ | +++ |
| 8 | +++ | +++ | 39 | +++ | +++ |
| 9 | +++ | +++ | 40 | +++ | +++ |
| 10 | +++ | +++ | 41 | +++ | +++ |
| 11 | +++ | +++ | 42 | +++ | +++ |
| 12 | +++ | +++ | 43 | + | + |
| 13 | +++ | +++ | 44 | + | + |
| 14 | +++ | +++ | 45 | + | + |
| 15 | +++ | +++ | 46 | + | + |
| 16 | +++ | +++ | 47 | + | + |
| 17 | +++ | +++ | 48 | + | + |
| 18 | +++ | +++ | 49 | + | + |
| 19 | +++ | +++ | 50 | + | + |
| 20 | +++ | +++ | 51 | + | + |
| 21 | +++ | +++ | 52 | + | + |
| 22 | +++ | +++ | 53 | + | + |
| 23 | +++ | +++ | 54 | + | + |
| 24 | +++ | +++ | 55 | + | + |
| 25 | +++ | +++ | 56 | - | - |
| 26 | + | + | 57 | - | - |
| 27 | + | + | 58 | - | - |
| 28 | ++ | +++ | 59 | - | - |
| 30 | +++ | +++ | 60 | - | - |
| 31 | + | + | 61 | - | - |
| 32 | + | + | | | |

**Table 2. IC₅₀ values of compounds against MNKs proteases**

| | MNK1 | MNK2 |
|---|---|---|
| Number | IC₅₀ (nM) | IC₅₀ (nM) |
| STSP | **** | **** |
| 1 | **** | **** |
| 2 | ** | **** |
| 3 | **** | **** |
| 5 | *** | *** |
| 6 | *** | *** |
| 8 | ** | ** |
| 10 | *** | **** |
| 12 | **** | **** |
| 13 | **** | **** |
| 14 | **** | **** |
| 15 | **** | **** |
| 16 | **** | **** |
| 17 | **** | **** |
| 18 | *** | **** |
| 19 | ** | *** |
| 20 | ** | *** |
| 21 | **** | **** |
| 22 | **** | **** |
| 23 | ** | ** |
| 24 | *** | *** |
| 25 | **** | **** |
| 28 | * | ** |
| 30 | **** | **** |
| 36 | **** | **** |
| 37 | **** | **** |
| 38 | **** | **** |
| 39 | **** | **** |
| 40 | **** | **** |
| 41 | **** | **** |
| 42 | **** | **** |
| | | |

We tested the effects of the above compounds on different mouse disease models, illustrated below by compound 12 as a specific Example.

### Example 74: Weight loss trial in a high-fat feeding obesity model

C57BL/6J male mice were fed with high-fat diet (60 kcal%) and low-fat diet (10 kcal%), respectively. After 16 weeks, the obesity model was successfully established (the body weight of high-fat diet was higher than 20% of the body weight of low-fat diet). They were divided into blank group, 12 high dose group (100 mg/kg), 12 low dose group (50 mg/kg) and orlistat group (50 mg/kg). The mice in the control group were given normal saline as the control, and the daily afternoon gavage of the drug for 4 weeks. During the period of regular measurement of food intake and body weight changes, the results showed that after 1 month of treatment, the body weight of the treated group decreased significantly, but the food intake did not decrease.

### Example 75: Hypoglycemic test in STZ+ high-fat fed diabetic mouse model

A total of 50 male Kunming mice were fed in a constant temperature of 25 °C with free access to food and water. After 5 days of adaptive feeding, the mice were divided into two groups for the first time. The first group (n = 10) was the blank group (C) fed with normal diet, and the rest were the second group (n = 40) fed with 60% high-fat diet to induce high-fat model and free access to water for 4 weeks. At the fifth week, the mice in the second group were injected with streptozotocin (STZ) to induce type 2 diabetes model. The total injection dose was 150 mg/kg, and the feeding conditions were unchanged. Type 2 diabetic mice with blood glucose levels higher than 11mmol/L were divided into model group (M, n = 10) and control group (M, n = 10). Distilled water), metformin group (Meft, 225 mg/kg/d), compound 12 low-dose group (2A-L, 50 mg/kg/d), compound 12 high-dose group (2A-H, 100 mg/kg/d). The rats in group M were given distilled water by gavage every morning, and the dosage was Meft: 225 mg/kg. 2A-L: 50 mg/kg; 2A-H: 100 mg/kg. The mice were administrated by gavage at a dose of 0.2mL/g for 8 weeks, and the body weight and fasting blood glucose were measured regularly during the period.

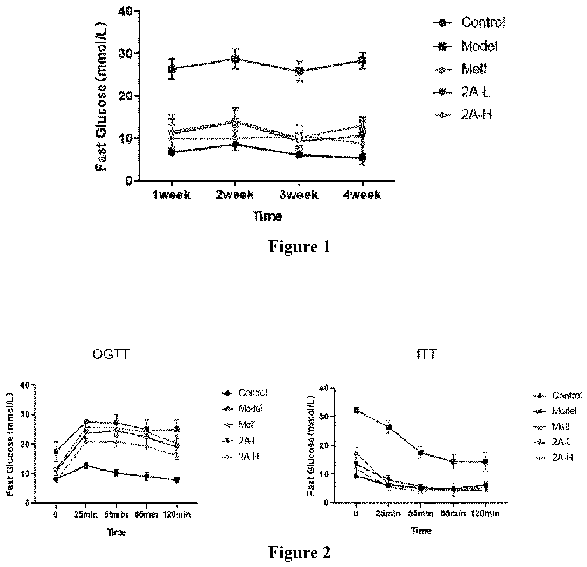

The results showed (Figure 1) that compound 12 significantly reduced blood glucose in mice in both the low dose group (50 mg/kg) and the high dose group (100 mg/kg). The hypoglycemic effect of the low dose group was equivalent to that of the positive control group (metformin, 225 mg/kg), and the high dose group showed better hypoglycemic effect than the positive control.

compound 12 improved glucose tolerance (Figure 2). The results of insulin tolerance test showed that after intraperitoneal injection of insulin, the blood glucose decreased gradually and reached the lowest value in 55 minutes. Compared with the model group, both the low and high dose groups of compound 12 showed lower insulin sensitivity than the model group, indicating that compound 12 restored insulin sensitivity in mice. These results suggested that compound 12 could reduce blood glucose and restore insulin sensitivity.

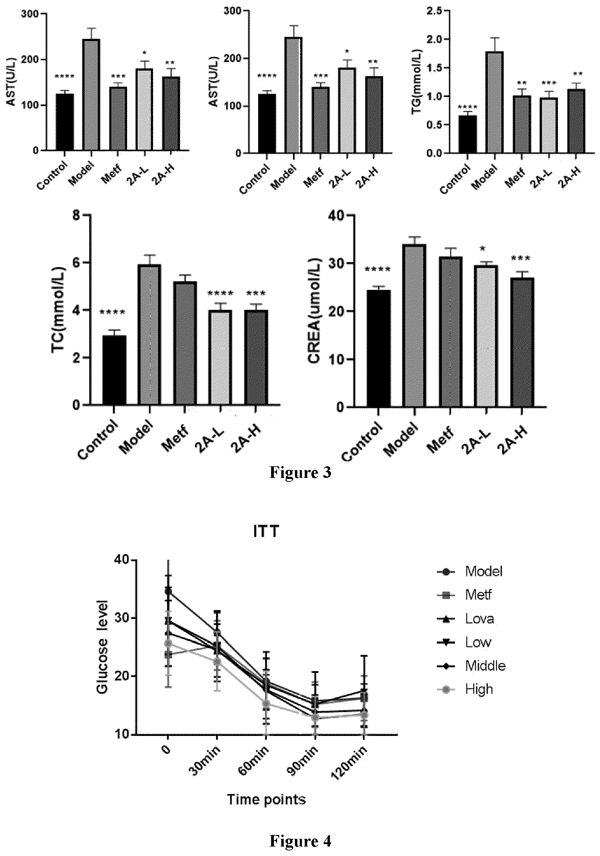

compound 12 significantly reduced serum AST levels (Figure 3). Compared with the model group, the serum TG and TC of the mice treated with compound 12 were significantly decreased, indicating that compound 12 had a certain lipid-lowering effect. compound 12 is similar to metformin in reducing TG, and even better than metformin in reducing TC. Elevated serum creatinine values usually indicate renal damage. CREA was significantly increased in the model group compared with the control group, indicating that the model caused damage to the kidney. After administration, CREA decreased significantly, indicating that compound 12 could repair diabetic kidney damage.

### Example 76: Insulin tolerance and liver function improvement test in male db/db mouse model

Six-week-old male db/db mice were randomly divided into 6 groups of 12 mice in each group: Model group (M, distilled water), metformin group (Meft, 200 mg/kg/d), lovastatin group (6mg/kg), compound 12 low dose group (20 mg/kg/d), compound 12 middle dose group (40 mg/kg/d), compound 12 high dose group (80 mg/kg/d). They were raised in a constant temperature of 25 °C and fed and drinking freely. The mice were administrated by gavage at a dose of 0.2 mL/g for 6 weeks, and the body weight and fasting blood glucose were measured regularly during the period.

Insulin tolerance test (ITT) was performed with normal saline (1.0U/ ml, 1.0U/kg) and fasting for 4 hours in the morning. In the afternoon experiment, weight was weighed, blood glucose was measured before insulin injection, insulin injection volume was calculated according to body weight, and blood glucose was measured at 15min, 30min, 45min, and 60min. At the end of the experiment, each cage was supplemented with feed. As observed by insulin tolerance test (Figure 4), the insulin sensitivity of db/db mice was significantly improved by drug treatment in each group, especially in the 12 high-dose group.

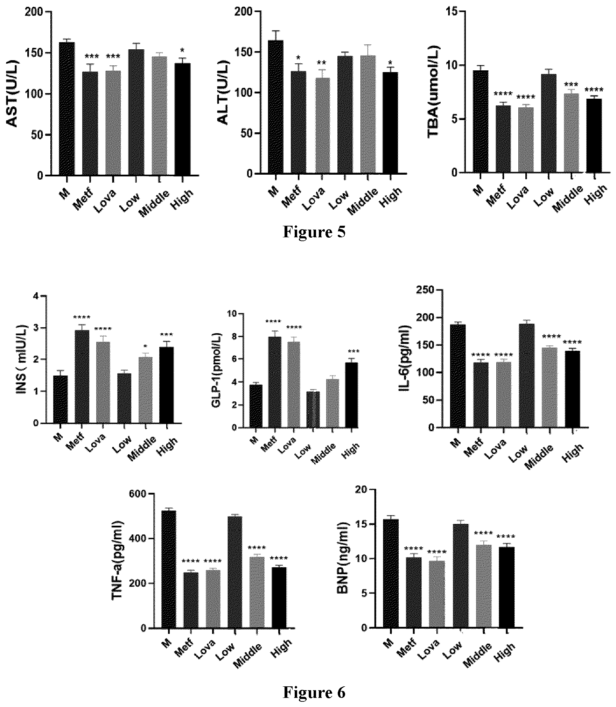

In the serum biochemical indicators of liver function (Figure 5), compared with the model group, the high-dose administration group had significant decreases in ALT, AST and total bile acid content, indicating that the drug could attenuate liver injury and alleviate liver inflammation in db/db mice.

In the serum active protein index (Figure 6), the content of insulin INS was significantly increased after high dose administration, indicating that the compound had a promoting effect on insulin secretion by islet cells. Increased blood levels of GLP-1, a brain-gut peptide, indicating the beneficial therapeutic effect of the drug; At the same time, plasma BNP, a marker of heart failure, and inflammatory cytokines TNF and IL-6 were significantly decreased at low doses, which further confirmed that the drug had a good effect on the elimination of inflammation in the mice.

The above test results indicate that the compounds of the invention have good MNK1/2 protein kinase inhibitory effect and exhibit good safety in cells. Taking example compound 12 as a representative, this class of compounds can significantly control blood glucose, reduce body weight, improve various blood biochemical indicators, and protect important organs such as liver in diabetic mice and obese mice models, which has a good prospect for drug development and application.

The present invention is described in detail above, and specific examples are used in this article to describe the principles and embodiments of the present invention. The above examples are only used to help understand the methods of the present invention and its core ideas, including the best way, and also to enable any skilled person in the art to practice the present invention, including the manufacture and use of any device or system. And the implementation of any combined approach. It should be pointed out that for ordinary technicians in the art, the invention can also be improved and modified on the premise of not separated from the principle of the invention, and these improvements and modifications also fall within the scope of protection of the claims of the invention. The scope of protection of the invention patent is defined by the claims.

## Claims

1. An imidazothiazole derivative, or a stereoisomer, a tautomer, or a geometric isomer thereof, or a pharmaceutically acceptable salt thereof, wherein the imidazothiazole derivative has a structure of Formula (I) : 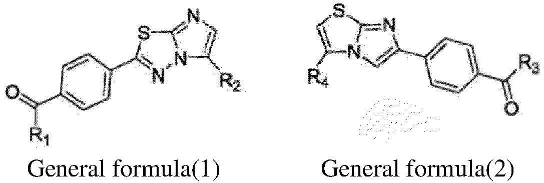 wherein
Formula (I) is general formula (1) or general formula (2);
R₁ and R₃ are independently selected from a 5 to 6 membered heterocyclyl containing 1 to 2 nitrogen atoms optionally substituted with one or more groups selected from the group consisting of C1-C6 alkyl, C1-C6 haloalkyl, C1-C6 alkylamino, C1-C6 alkoxy, 5 to 6 membered heterocyclic ring, halogen, hydroxyl, cyano, nitro, amino, and carbonyl; or
R₁ is C1-C6 alkoxy optionally substituted with one or more groups selected from the group consisting of hydroxyl, halogen, amino, dimethylamino, and 5 to 6 membered heterocyclic ring, or R₁ is C1-C6 alkylthio optionally substituted with one or more groups selected from the group consisting of hydroxyl, halogen, amino, dimethylamino, and 5 to 6 membered heterocyclic ring, or R₁ is C1-C6 alkylamino optionally substituted with one or more groups selected from the group consisting of hydroxyl, halogen, amino, dimethylamino, and 5 to 6 heterocyclic rings, provided at your option; and
R₂ and R₄ are each independently aryl or heteroaryl, wherein the aryl and heteroaryl are optionally substituted with one or more groups selected from the group consisting of C1-C6 alkyl, C1-C6 haloalkyl, C1-C6 alkylamino, C1-C6 alkoxy, C1-C6 alkoxy-carbonyl, C1-C6 hydroxyalkyl, halogen, hydroxyl, cyano, nitro, amino, and carbonyl.

2. The imidazothiazole derivative of claim 1, or the stereoisomer, the tautomer, or geometric isomer thereof, or the pharmaceutically acceptable salt thereof, wherein the 5 to 6 membered heterocyclyl is selected from the group consisting of piperazinyl, morpholinyl, piperidinyl, hexahydropyranyl, tetrahydrofuranyl, tetrahydrothiophenyl, pyrrolyl, and pyrrolidinyl.

3. The imidazothiazole derivative of any one of claims 1-2, or the stereoisomer, tautomer, or geometric isomer thereof, or the pharmaceutically acceptable salt thereof, wherein the aryl is phenyl, or naphthyl, and the heteroaryl is selected from the group consisting of furyl, thiophenyl, pyridyl, thiazolyl, and imidazolyl.

4. The imidazothiazole derivative of any one of claims 1-3, or the stereoisomer, tautomer, or geometric isomer thereof, or the pharmaceutically acceptable salt thereof, wherein
the C1-C6 alkylamino is -NR¹R², wherein R¹ and R² are each independently H or C1-C6 alkyl, with the proviso that R¹ and R² are not H at the same time.

5. The imidazothiazole derivative of any one of claims 1-4, wherein the compound is selected from compounds 1-55 as shown in the below Table
| Num ber | Structure | Num ber | Structure |
|---|---|---|---|
| 1 | 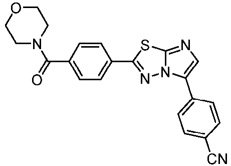 | 29 | 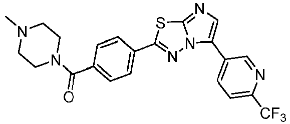 |
| 2 | 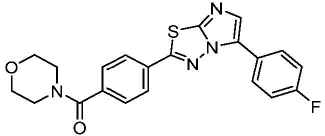 | 30 | 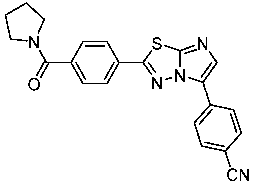 |
| 3 | 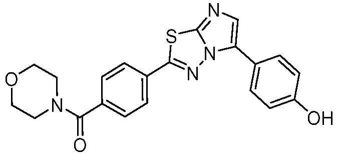 | 31 | 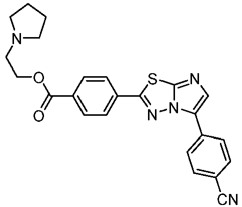 |
| 4 | 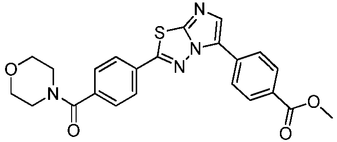 | 32 | 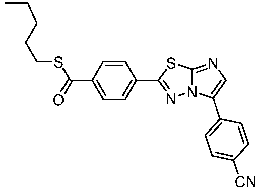 |
| 5 | 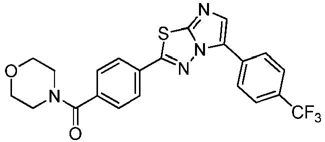 | 33 | 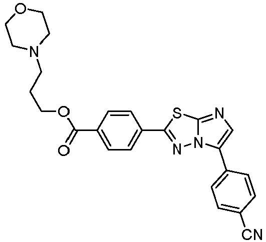 |
| 6 | 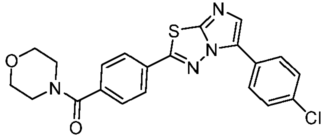 | 34 | 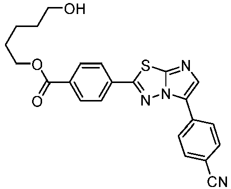 |
| 7 | 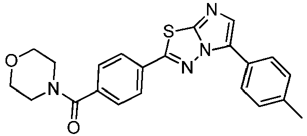 | 35 | 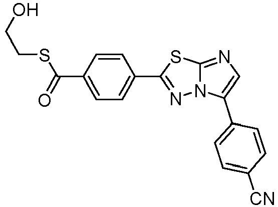 |
| 8 | 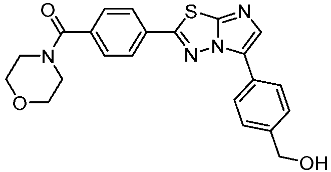 | 36 | 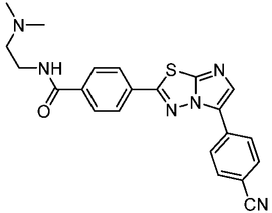 |
| 9 | 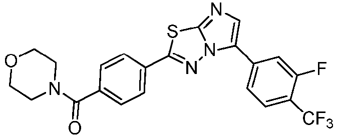 | 37 | 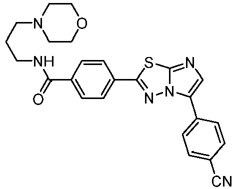 |
| 10 | 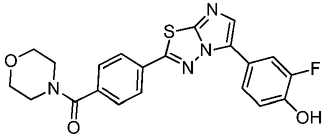 | 38 | 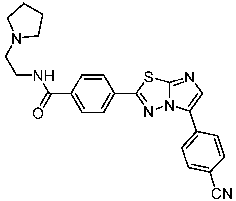 |
| 11 | 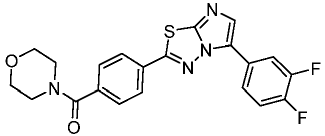 | 39 | 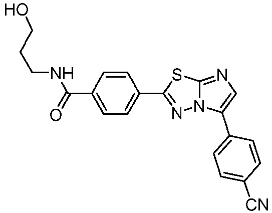 |
| 12 | 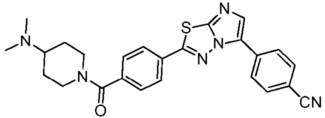 | 40 | 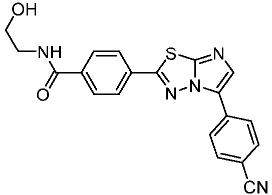 |
| 13 | 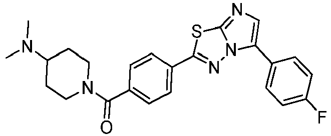 | 41 | 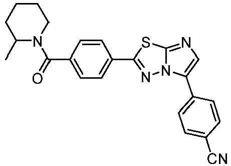 |
| 14 | 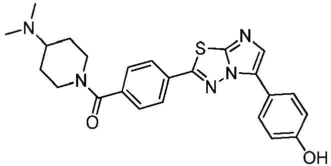 | 42 | 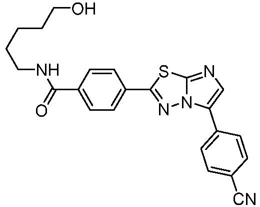 |
| 15 | 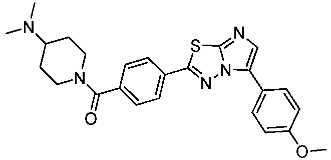 | 43 | 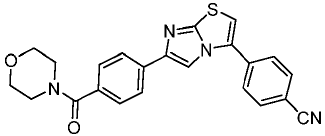 |
| 16 | 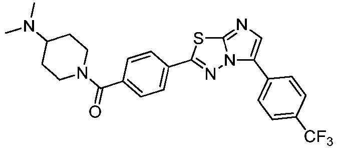 | 44 | 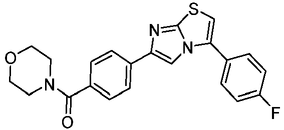 |
| 17 | 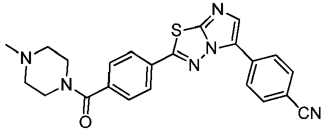 | 45 | 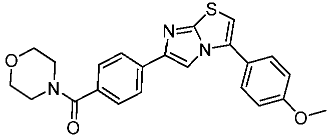 |
| 18 | 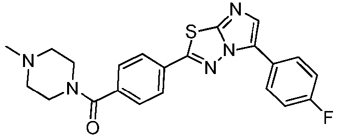 | 46 | 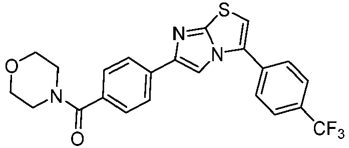 |
| 19 | 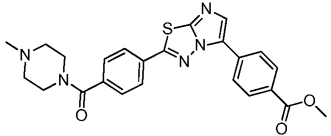 | 47 | 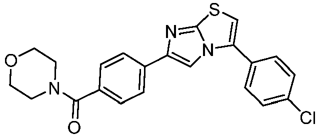 |
| 20 | 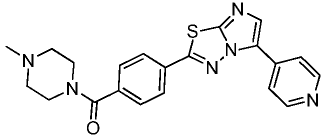 | 48 | 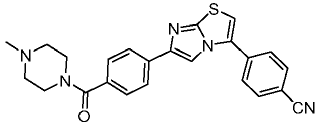 |
| 21 | 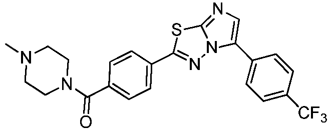 | 49 | 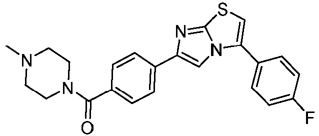 |
| 22 | 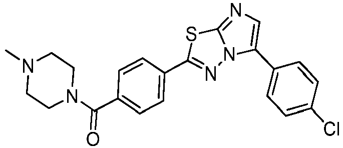 | 50 | 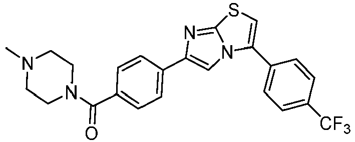 |
| 23 | 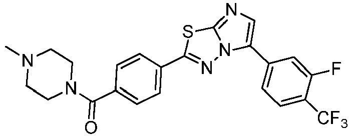 | 51 | 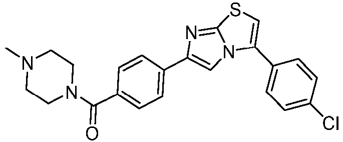 |
| 24 | 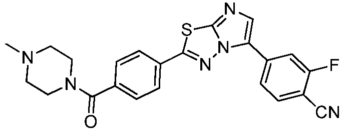 | 52 | 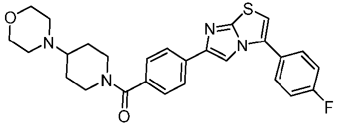 |
| 25 | 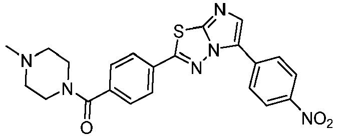 | 53 | 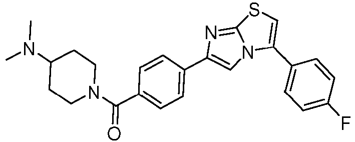 |
| 26 | 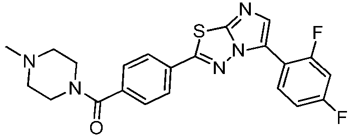 | 54 | 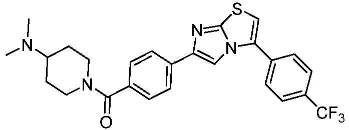 |
| 27 | 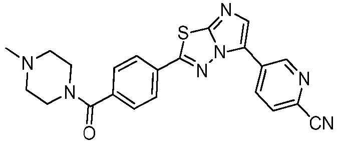 | 55 | 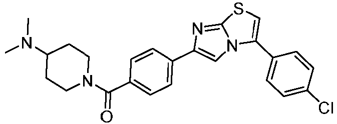 |
| 28 | 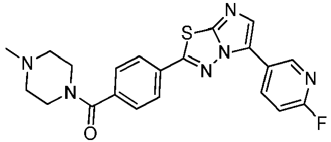 | | |
or the stereoisomer, tautomer, or geometric isomer thereof, or the pharmaceutically acceptable salt thereof.

6. A preparation method of the imidazothiazole derivative of any one of claims 1-5, wherein the preparation method is a synthesis method of general formula 1 or a synthesis method of general formula 2; wherein
the synthesis method of general formula 1 comprises a step of: 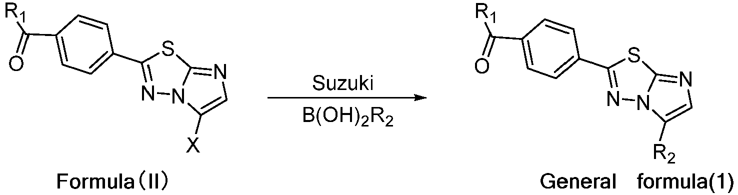
making a compound of formula (II) undergo Suzuki condensation reaction with and the corresponding boronic acid derivative (B(OH)₂R₂), thereby obtaining a compound of general formula 1, wherein the definitions of R₁ and R₂ are the same as in any one of claims 1-5, X is a halogen, preferably X is chlorine, bromine or iodine;
and, the synthesis method of general formula 2 comprises a step of: 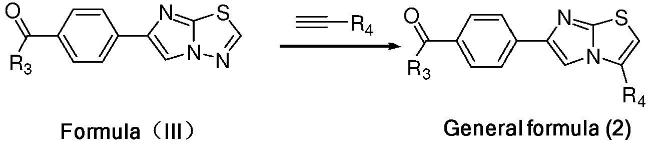
reacting a compound of formula (III) with a corresponding acetylene derivative ( 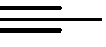 R₄) under alkaline condition, thereby obtaining the compound of general formula 2, wherein the definitions of R₃ and R₄ are the same as in any one of claims 1-5.

7. The preparation method of claim 6, wherein:
the synthetic method of general formula 1 further comprises a step of preparing a compound of formula (II) from a compound of formula (IV) : 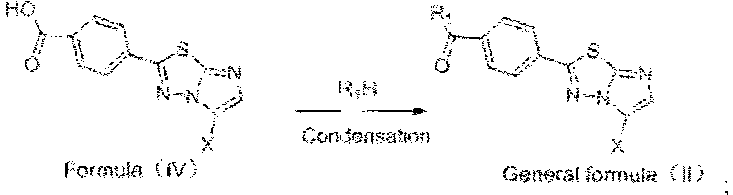
the synthesis method of general formula 2 further comprises a step of preparing a compound of formula (III) from a compound of formula (V) :

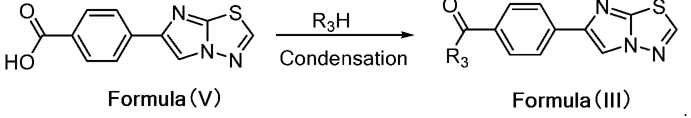

8. An intermediate for preparing the imidazothiazole derivative of any one of claims 1-5, wherein the intermediate has a structure of Formula (II), Formula (III), Formula (IV), or Formula (V): 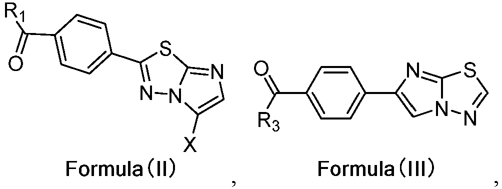 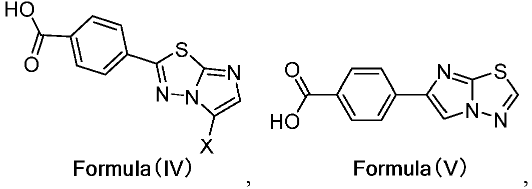
wherein the definitions of R₁ and R₃ are the same as in claim 1, and X is a halogen;
preferably,
the intermediate is selected from the group consisting of:

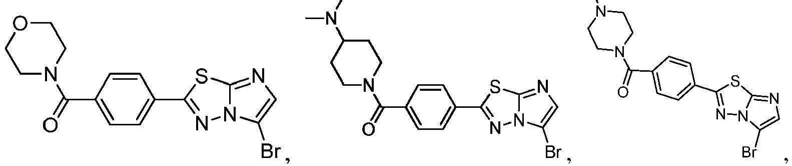

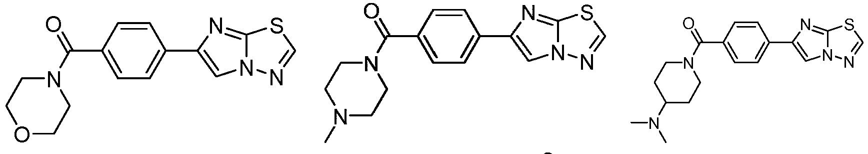

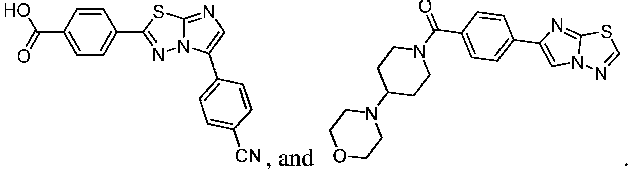

9. The intermediate of claim 8 for use in the preparation of the imidazothiazole derivative of any one of claims 1-5.

10. The imidazothiazole derivativeof any one of claims 1-5, or the stereoisomer, tautomer, geometric isomer thereof, or the pharmaceutically acceptable salt thereof for use for:
(a) inhibiting the kinase activity of MNK1 or MNK2 or a variant thereof; and/or
(b) preventing and/or treating metabolic diseases associated with MNK activity; and/or
(c) preventing and/or treating cancers caused by abnormal levels of MNK1 and/or MNK2, and/or
(d) inhibiting the kinase activity of MNK1 and/or MNK2.

11. The compound for use of claims 10, wherein the metabolic disease associated with MNK activity is selected from the group consisting of type 1 diabetes mellitus, type 2 diabetes mellitus, hyperlipemia, obesity, and fatty liver disease, or complications thereof or related disorders thereof.

12. The compound for use of claim 11, wherein
diabetes mellitus and the complication thereof are selected from the group consisting of impaired glucose tolerance, diabetic gangrene, diabetic arthropathy, diabetic osteopenia, diabetic glomerulosclerosis, diabetic nephropathy, diabetic dermatopathy, diabetic neuropathy, diabetic cataract, diabetic retinopathy, diabetic macular degeneration, diabetic foot syndrome, diabetic coma, diabetic hyperosmolar coma, hypoglycemic coma, hyperglycemic coma, diabetic acidosis, diabetic ketoacidosis, intracapillary glomerular nephropathy, diabetic muscular atrophy, diabetic autonomic neuropathy, diabetic mononeuropathy, diabetic polyneuropathy, diabetic vascular disease, diabetic peripheral vascular disease, diabetic ulcer, diabetic arthropathy, and diabetic obesity;
hyperlipidemia and the complications thereof are selected from the group consisting of hypercholesterolemia, familial hypercholesterolemia, Verde's hyperlipoproteinemia, hyperβ-lipoproteinemia, hyperlipidemia, low density lipoproteinemia, pure hypertriglyceridemia, endogenous hypertriglyceridemia, simple hypercholesterolemia, simple hypertriglyceridemia, cardiovascular disease. Wherein cardiovascular diseases include: hypertension, ischemia, varicose veins, retinal vein occlusion, atherosclerosis, angina pectoris, myocardial infarction, stenocardia, pulmonary hypertension, congestive heart failure, glomerular disease, tubular interstitial disorders of the kidney, renal failure, vascular stenosis, and cerebrovascular disease (stroke); and
fatty liver disease is selected from non-alcoholic fatty liver disease (NAFLD), or non-alcoholic steatohepatitis (NASH), and the resulting chronic inflammation, progressive fibrosis, or cirrhosis.

13. A pharmaceutical composition, comprising the imidazothiazole derivative of any one of claims 1-5, or the stereoisomer, tautomer, or geometric isomer thereof, or the pharmaceutically acceptable salt thereof as active ingredient; preferably, the pharmaceutical composition further comprises pharmaceutically acceptable excipients.

14. The pharmaceutical composition of claim 13, wherein the pharmaceutical composition further comprises other MNK1 and/or MNK2 inhibitor.

15. The pharmaceutical composition of any one of claims 13-14, wherein the dosage form of the pharmaceutical composition is a solid preparation, a liquid preparation or a semi-solid preparation, preferably, the dosage form of the pharmaceutical composition is a tablet, capsule, or injection.

## Patentansprüche

1. Imidazothiazol-Derivat oder Stereoisomer, Tautomer oder geometrisches Isomer davon oder pharmazeutisch annehmbares Salz davon, wobei das Imidazothiazol-Derivat eine Struktur von Formel (I) aufweist: 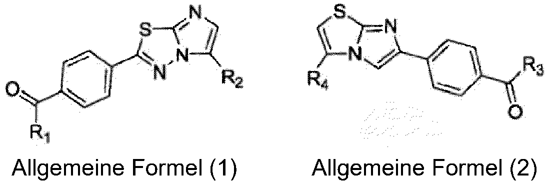 wobei
Formel (I) allgemeine Formel (1) oder allgemeine Formel (2) ist;
R₁ und R₃ unabhängig ausgewählt sind aus einem 5- bis 6-gliedrigen Heterocyclyl, das 1 bis 2 Stickstoffatome enthält, die optional substituiert sind mit einer oder mehreren Gruppen, die ausgewählt sind aus der Gruppe, bestehend aus C1-C6-Alkyl, C1-C6-Haloalkyl, C1-C6-Alkylamino, C1-C6-Alkoxy, 5- bis 6-gliedrigem heterocyclischem Ring, Halogen, Hydroxyl, Cyano, Nitro, Amino und Carbonyl; oder
R₁ C1-C6-Alkoxy ist, optional substituiert mit einer oder mehreren Gruppen, ausgewählt aus der Gruppe, bestehend aus Hydroxyl, Halogen, Amino, Dimethylamino und 5- bis 6-gliedrigem heterocyclischem Ring; oder R₁ C1-C6-Alkylthio ist, optional substituiert mit einer oder mehreren Gruppen, ausgewählt aus der Gruppe, bestehend aus Hydroxyl, Halogen, Amino, Dimethylamino und einem 5- bis 6-gliedrigen heterocyclischen Ring, oder R₁ C1-C6-Alkylamino ist, optional substituiert mit einer oder mehreren Gruppen, ausgewählt aus der Gruppe, bestehend aus Hydroxyl, Halogen, Amino, Dimethylamino und 5 bis 6 heterocyclischen Ringen besteht, die nach Ihrer Wahl bereitgestellt sind; und
R₂ und R₄ jeweils unabhängig Aryl oder Heteroaryl sind, wobei das Aryl und Heteroaryl optional mit einer oder mehreren Gruppen substituiert sind, die ausgewählt sind aus der Gruppe, bestehend aus C1-C6-Alkyl, C1-C6-Halogenalkyl, C1-C6-Alkylamino, C1-C6-Alkoxy, C1-C6-Alkoxycarbonyl, C1-C6-Hydroxyalkyl, Halogen, Hydroxyl, Cyano, Nitro, Amino und Carbonyl.

2. Imidazothiazol-Derivat nach Anspruch 1 oder Stereoisomer, Tautomer oder geometrische Isomer davon oder pharmazeutisch annehmbares Salz davon, wobei das 5- bis 6-gliedrige Heterocyclyl ausgewählt ist aus der Gruppe, bestehend aus Piperazinyl, Morpholinyl, Piperidinyl, Hexahydropyranyl, Tetrahydrofuranyl, Tetrahydrothiophenyl, Pyrrolyl und Pyrrolidinyl.

3. Imidazothiazol-Derivat nach einem der Ansprüche 1-2 oder Stereoisomer, Tautomer oder geometrisches Isomer davon oder pharmazeutisch annehmbares Salz davon, wobei das Aryl Phenyl oder Naphthyl ist und das Heteroaryl ausgewählt ist aus der Gruppe, bestehend aus Furyl, Thiophenyl, Pyridyl, Thiazolyl und Imidazolyl.

4. Imidazothiazol-Derivat nach einem der Ansprüche 1-3 oder Stereoisomer, Tautomer oder geometrisches Isomer davon oder pharmazeutisch annehmbares Salz davon, wobei
das C1-C6-Alkylamino -NR¹R² ist, wobei R¹ und R² jeweils unabhängig H oder C1-C6-Alkyl sind, mit der Maßgabe, dass R¹ und R² nicht gleichzeitig H sind.

5. Imidazothiazolderivat nach einem der Ansprüche 1 bis 4, wobei die Verbindung ausgewählt ist aus Verbindungen 1-55, wie es in der Tabelle unten gezeigt ist,
| Ziffer | Struktur | Ziffer | Struktur |
|---|---|---|---|
| 1 | 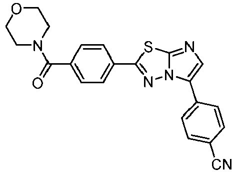 | 29 | 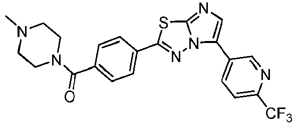 |
| 2 | 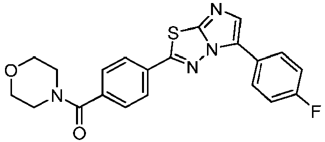 | 30 | 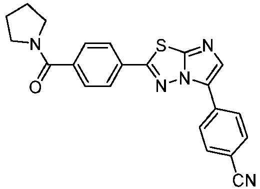 |
| 3 | 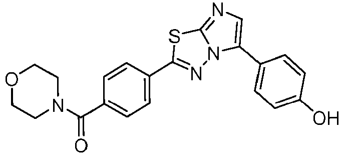 | 31 | 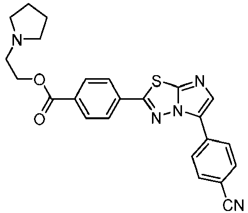 |
| 4 | 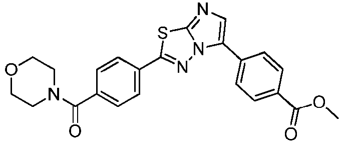 | 32 | 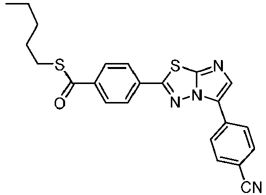 |
| 5 | 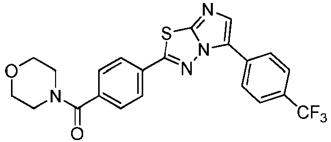 | 33 | 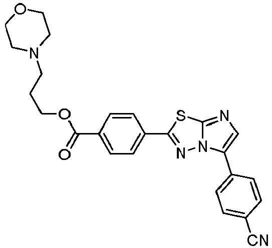 |
| 6 | 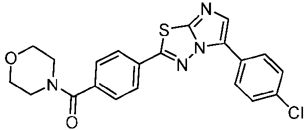 | 34 | 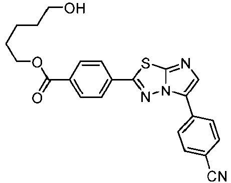 |
| 7 | 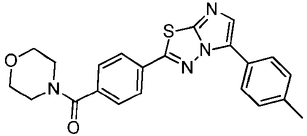 | 35 | 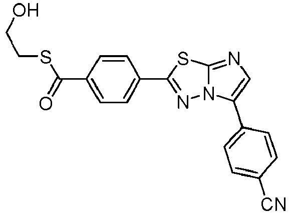 |
| 8 | 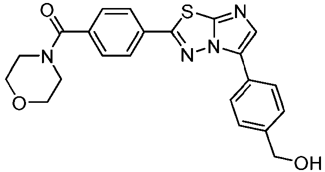 | 36 | 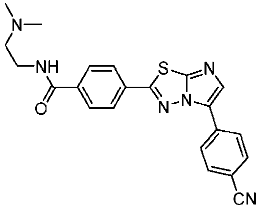 |
| 9 | 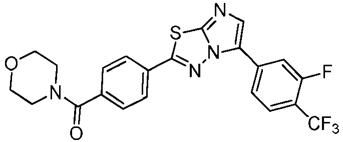 | 37 | 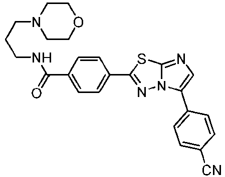 |
| 10 | 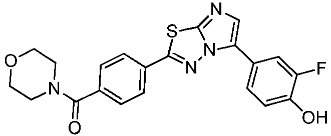 | 38 | 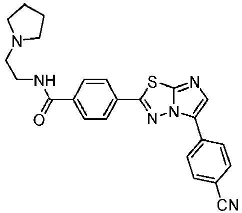 |
| 11 | 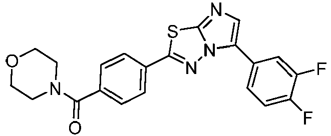 | 39 | 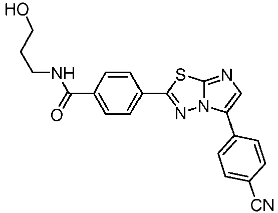 |
| 12 | 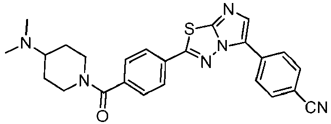 | 40 | 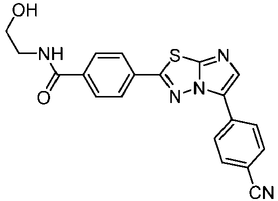 |
| 13 | 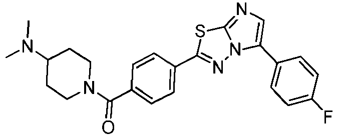 | 41 | 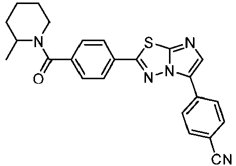 |
| 14 | 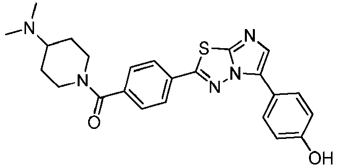 | 42 | 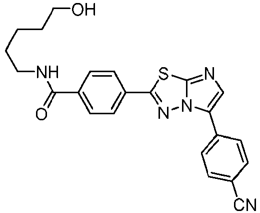 |
| 15 | 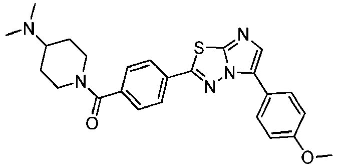 | 43 | 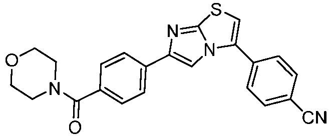 |
| 16 | 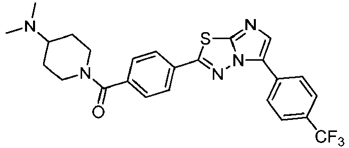 | 44 | 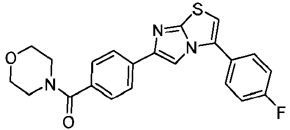 |
| 17 | 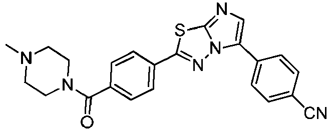 | 45 | 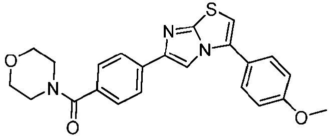 |
| 18 | 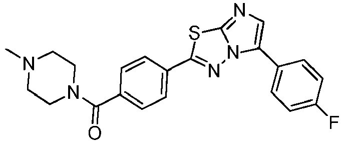 | 46 | 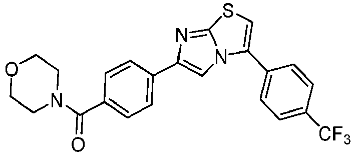 |
| 19 | 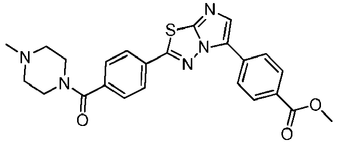 | 47 | 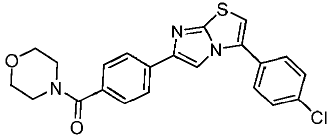 |
| 20 | 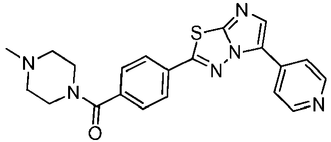 | 48 | 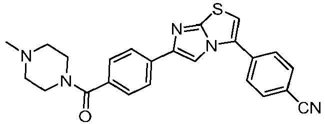 |
| 21 | 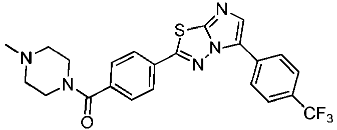 | 49 | 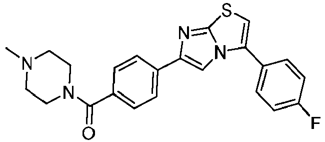 |
| 22 | 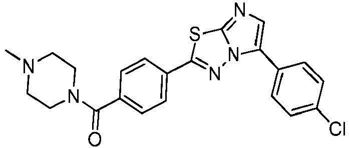 | 50 | 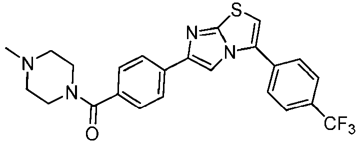 |
| 23 | 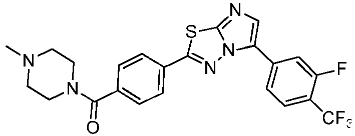 | 51 | 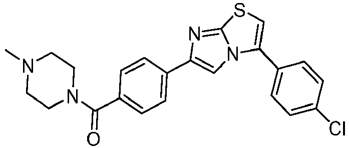 |
| 24 | 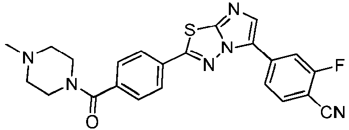 | 52 | 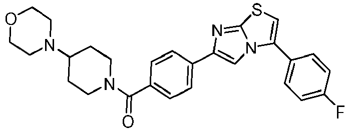 |
| 25 | 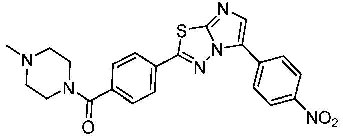 | 53 | 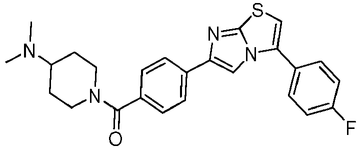 |
| 26 | 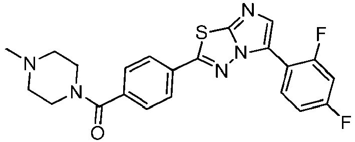 | 54 | 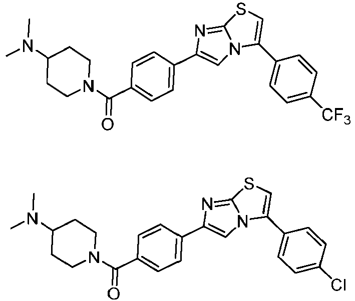 |
| 27 | 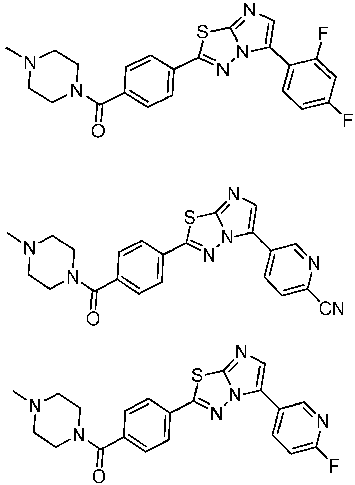 | 55 | 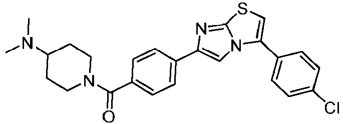 |
| 28 | 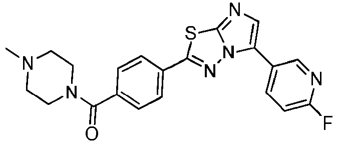 | | |
oder Stereoisomer, Tautomer oder geometrisches Isomer davon oder pharmazeutisch annehmbares Salz davon.

6. Herstellungsverfahren des Imidazothiazol-Derivats nach einem der Ansprüche 1-5, wobei das Herstellungsverfahren ein Syntheseverfahren von allgemeiner Formel 1 oder ein Syntheseverfahren von allgemeiner Formel 2 ist; wobei
das Syntheseverfahren von allgemeiner Formel 1 einen folgenden Schritt umfasst: 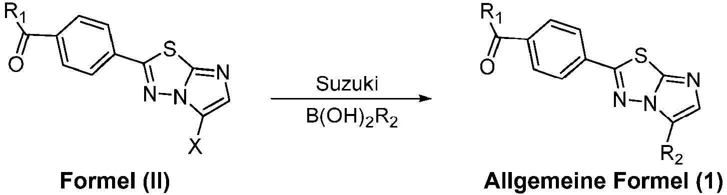
Unterziehen einer Verbindung von Formel (II) einer Suzuki-Kondensationsreaktion mit dem entsprechenden Borsäurederivat (B(OH)₂R₂), wodurch eine Verbindung von allgemeiner Formel 1 erlangt wird, wobei die Definitionen von R₁ und R₂ gleich sind wie in einem der Ansprüche 1-5, X ein Halogen ist, vorzugsweise X Chlor, Brom oder Jod ist;
und das Syntheseverfahren von allgemeiner Formel 2 einen folgenden Schritt umfasst: 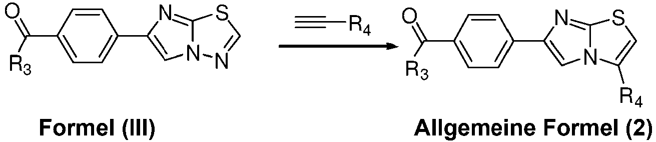
Reagieren einer Verbindung von Formel (III) mit einem entsprechenden Acetylenderivat (≡-R₄) unter alkalischen Bedingungen, wodurch die Verbindung von allgemeiner Formel 2 erlangt wird, wobei die Definitionen von R₃ und R₄ gleich sind wie in einem der Ansprüche 1-5.

7. Herstellungsverfahren nach Anspruch 6, wobei:
das Syntheseverfahren von allgemeiner Formel 1 ferner einen Schritt eines Herstellens einer Verbindung von Formel (II) aus einer Verbindung von Formel (IV) umfasst: 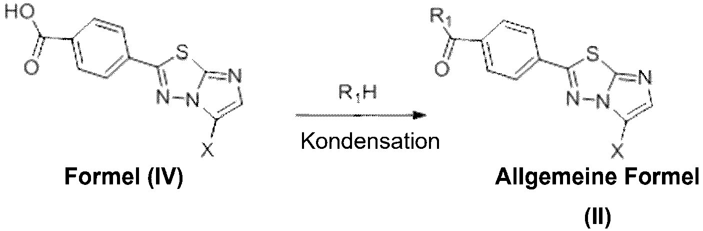
das Syntheseverfahren von allgemeiner Formel 2 ferner einen Schritt eines Herstellens einer Verbindung von Formel (III) aus einer Verbindung von Formel (V) umfasst:

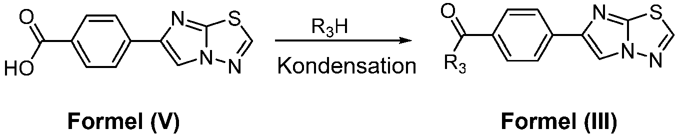

8. Zwischenprodukt zum Herstellen des Imidazothiazolderivats nach einem der Ansprüche 1-5, wobei das Zwischenprodukt eine Struktur von Formel (II), Formel (III), Formel (IV) oder Formel (V) aufweist: 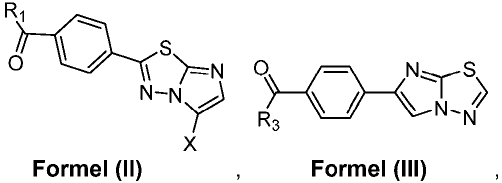 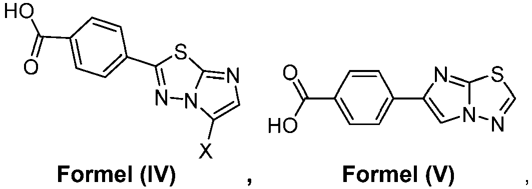
wobei die Definitionen von R₁ und R₃ gleich sind wie in Anspruch 1, und X ein Halogen ist;
vorzugsweise
das Zwischenprodukt ausgewählt ist aus der Gruppe, bestehend aus:

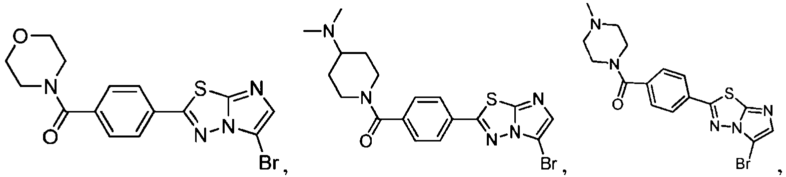

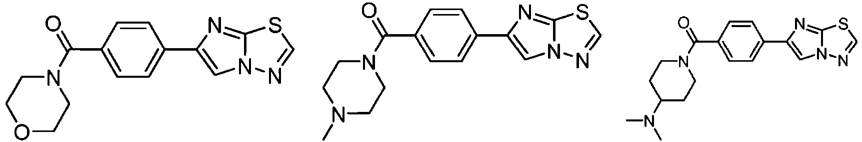

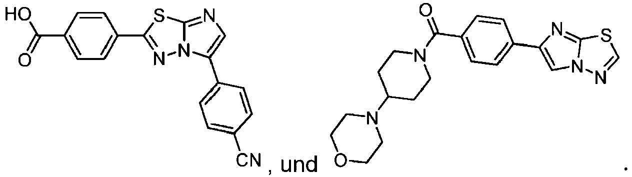

9. Zwischenprodukt nach Anspruch 8
zur Verwendung bei der Herstellung des Imidazothiazol-Derivats nach einem der Ansprüche 1-5.

10. Imidazothiazol-Derivat nach einem der Ansprüche 1-5 oder Stereoisomer, Tautomer, geometrisches Isomer davon oder pharmazeutisch annehmbares Salz davon, zur Verwendung für:
(a) Hemmen der Kinaseaktivität von MNK1 oder MNK2 oder einer Variante davon; und/oder
(b) Vorbeugen und/oder Behandeln von Stoffwechselkrankheiten, die mit MNK-Aktivität assoziiert sind; und/oder
(c) Vorbeugen und/oder Behandeln von Krebs, der durch abnormale MNK1- und/oder MNK2-Spiegel verursacht wird, und/oder
(d) Hemmen der Kinaseaktivität von MNK1 und/oder MNK2.

11. Verbindung zur Verwendung nach Anspruch 10, wobei die Stoffwechselerkrankung, die mit der MNK-Aktivität assoziiert ist, ausgewählt ist aus der Gruppe, bestehend aus Typ-1-Diabetes mellitus, Typ-2-Diabetes mellitus, Hyperlipämie, Fettleibigkeit und Fettlebererkrankung oder Komplikationen davon oder damit verbundenen Störungen.

12. Verbindung zur Verwendung nach Anspruch 11, wobei
Diabetes mellitus und die Komplikationen davon ausgewählt sind aus der Gruppe, bestehend aus gestörter Glukosetoleranz, diabetischer Gangrän, diabetischer Arthropathie, diabetischer Osteopenie, diabetischer Glomerulosklerose, diabetischer Nephropathie, diabetischer Dermatopathie, diabetischer Neuropathie, diabetischem Katarakt, diabetischer Retinopathie, diabetischer Makuladegeneration, diabetischem Fußsyndrom, diabetischem Koma, diabetischem hyperosmolares Koma, hypoglykämischem Koma, hyperglykämischem Koma, diabetischer Azidose, diabetischer Ketoazidose, intrakapillärer glomerulärer Nephropathie, diabetischer Muskelatrophie, diabetischer autonomer Neuropathie, diabetischer Mononeuropathie, diabetischer Polyneuropathie, diabetischer Gefäßerkrankung, diabetischer peripherer Gefäßerkrankung, diabetischem Ulkus, diabetischer Arthropathie und diabetischer Adipositas;
Hyperlipidämie und die Komplikationen davon ausgewählt sind aus der Gruppe, bestehend aus Hypercholesterinämie, familiärer Hypercholesterinämie, Verde-Hyperlipoproteinämie, Hyperß-Lipoproteinämie, Hyperlipidämie, Lipoproteinämie niedriger Dichte, reiner Hypertriglyceridämie, endogener Hypertriglyceridämie, einfacher Hypercholesterinämie, einfacher Hypertriglyceridämie und kardiovaskulärer Erkrankungen. wobei Herz-Kreislauf-Erkrankungen Folgendes beinhalten: Bluthochdruck, Ischämie, Krampfadern, Netzhautvenenverschluss, Atherosklerose, Angina pectoris, Myokardinfarkt, Stenokardie, pulmonale Hypertonie, kongestive Herzinsuffizienz, glomeruläre Erkrankungen, tubuläre interstitielle Erkrankungen der Niere, Nierenversagen, Gefäßverengungen und zerebrovaskuläre Erkrankungen (Schlaganfall); und
Fettlebererkrankung ausgewählt ist aus nichtalkoholischer Fettlebererkrankung (NAFLD) oder nichtalkoholischer Steatohepatitis (NASH) und der daraus resultierenden chronischen Entzündung, fortschreitenden Fibrose oder Zirrhose.

13. Pharmazeutische Zusammensetzung, umfassend das Imidazothiazol-Derivat nach einem der Ansprüche 1-5 oder Stereoisomer, Tautomer oder geometrisches Isomer davon oder pharmazeutisch annehmbares Salz davon als Wirkstoff; vorzugsweise wobei die pharmazeutische Zusammensetzung ferner pharmazeutisch annehmbare Hilfsstoffe umfasst.

14. Pharmazeutische Zusammensetzung nach Anspruch 13, wobei die pharmazeutische Zusammensetzung ferner einen anderen MNK1- und/oder MNK2-Hemmer umfasst.

15. Pharmazeutische Zusammensetzung nach einem der Ansprüche 13-14, wobei die Darreichungsform der pharmazeutischen Zusammensetzung ein Feststoffpräparat, ein flüssiges Präparat oder ein halbfestes Präparat ist, vorzugsweise wobei die Darreichungsform der pharmazeutischen Zusammensetzung eine Tablette, Kapsel oder Injektion ist.

## Revendications

1. Dérivé d'imidazothiazole, ou stéréoisomère, tautomère ou isomère géométrique de celui-ci, ou sel pharmaceutiquement acceptable de celui-ci, dans lequel le dérivé d'imidazothiazole a une structure de formule (I) : 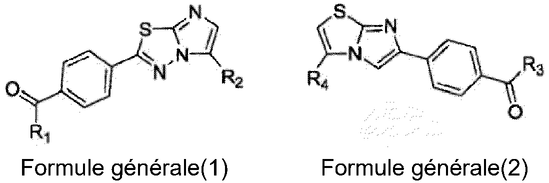 dans lequel
la formule (I) est la formule générale (1) ou la formule générale (2) ;
R₁ et R₃ sont indépendamment choisis parmi un hétérocycle de 5 à 6 chaînons contenant 1 à 2 atomes d'azote facultativement substitués par un ou plusieurs groupes choisis dans le groupe constitué par un alkyle en C1-C6, un haloalkyle en C1-C6, un alkylamino en C1-C6, un alcoxy en C1-C6, un cycle hétérocyclique de 5 à 6 chaînons, un halogène, un groupe hydroxyle, un groupe cyano, un groupe nitro, un groupe amino et un carbonyle ; ou
R₁ est un alcoxy en C1-C6 facultativement substitué par un ou plusieurs groupes choisis dans le groupe constitué par un hydroxyle, un halogène, un groupe amino, un groupe diméthylamino et un cycle hétérocyclique de 5 à 6 chaînons, ou R₁ est un alkylthio en C1-C6 facultativement substitué par un ou plusieurs groupes choisis dans le groupe constitué par un hydroxyle, un halogène, un groupe amino, un groupe diméthylamino et un cycle hétérocyclique de 5 à 6 chaînons, ou R₁ est un alkylamino en C1-C6 facultativement substitué par un ou plusieurs groupes choisis dans le groupe constitué par un hydroxyle, un halogène, un groupe amino, un groupe diméthylamino et des cycles hétérocycliques de 5 à 6 chaînons, fournis au choix ; et
R₂ et R₄ sont chacun indépendamment un aryle ou un hétéroaryle, dans lequel l'aryle et l'hétéroaryle sont facultativement substitués par un ou plusieurs groupes choisis dans le groupe constitué par un alkyle en C1-C6, un haloalkyle en C1-C6, un alkylamino en C1-C6, un alcoxy en C1-C6, un alcoxy-carbonyle en C1-C6, un hydroxyalkyle en C1-C6, un halogène, un hydroxyle, un groupe cyano, un groupe nitro, un groupe amino et un groupe carbonyle.

2. Dérivé d'imidazothiazole selon la revendication 1, ou le stéréoisomère, le tautomère ou l'isomère géométrique de celui-ci, ou le sel pharmaceutiquement acceptable de celui-ci, dans lequel l'hétérocyclyle de 5 à 6 chaînons est choisi dans le groupe constitué par le pipérazinyle, le morpholinyle, le pipéridinyle, l'hexahydropyranyle, le tétrahydrofuranyle, le tétrahydrothiophényle, le pyrrolyle et le pyrrolidinyle.

3. Dérivé d'imidazothiazole selon l'une quelconque des revendications 1 et 2, ou le stéréoisomère, le tautomère ou l'isomère géométrique de celui-ci, ou le sel pharmaceutiquement acceptable de celui-ci, dans lequel l'aryle est le phényle ou le naphtyle, et l'hétéroaryle est choisi dans le groupe constitué par le furyle, le thiophényle, le pyridyle, le thiazolyle et l'imidazolyle.

4. Dérivé d'imidazothiazole selon l'une quelconque revendications 1 à 3, ou le stéréoisomère, le tautomère ou l'isomère géométrique de celui-ci, ou le sel pharmaceutiquement acceptable de celui-ci, dans lequel
l'alkylamino en C1-C6 est -NR¹R², dans lequel R¹ et R² sont chacun indépendamment H ou un alkyle en C1-C6, à condition que R¹ et R² ne soient pas H en même temps.

5. Dérivé d'imidazothiazole selon l'une quelconque revendications 1 à 4, dans lequel le composé est choisi parmi les composés 1 à 55 présentés dans le tableau ci-dessous.
| Numér o | Structure | Numér o | Structure |
|---|---|---|---|
| 1 | 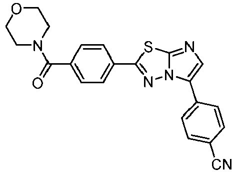 | 29 | 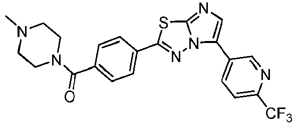 |
| 2 | 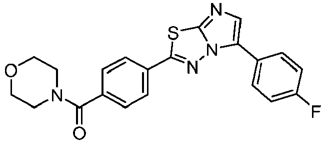 | 30 | 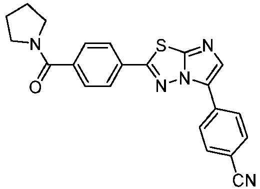 |
| 3 | 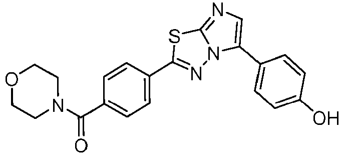 | 31 | 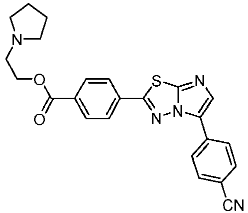 |
| 4 | 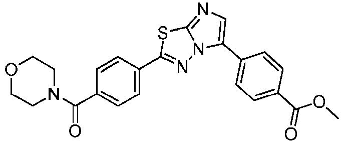 | 32 | 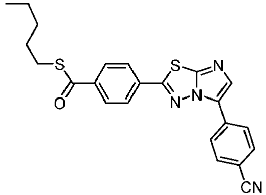 |
| 5 | 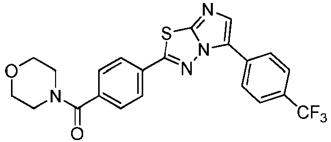 | 33 | 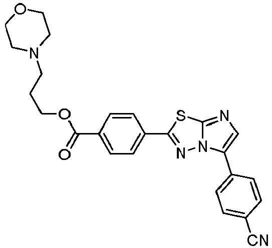 |
| 6 | 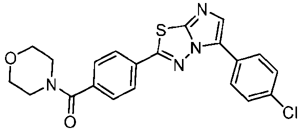 | 34 | 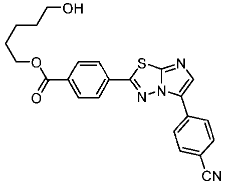 |
| 7 | 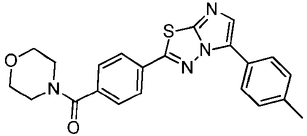 | 35 | 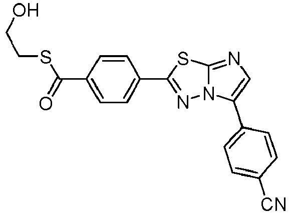 |
| 8 | 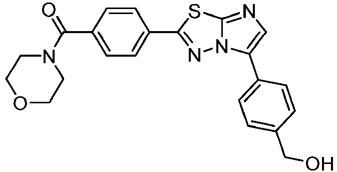 | 36 | 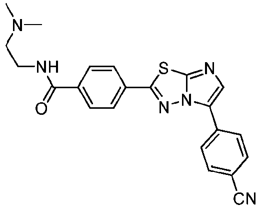 |
| 9 | 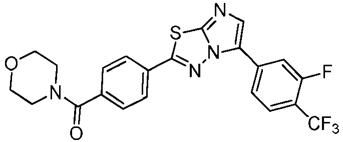 | 37 | 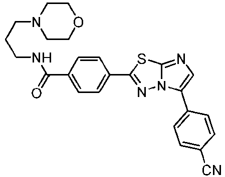 |
| 10 | 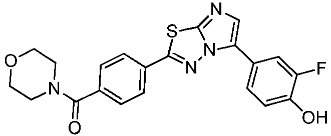 | 38 | 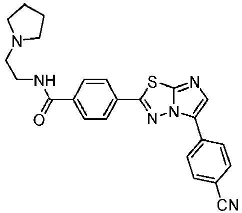 |
| 11 | 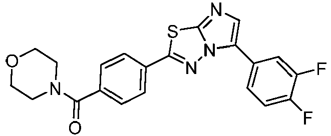 | 39 | 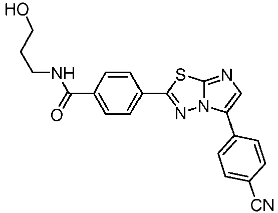 |
| 12 | 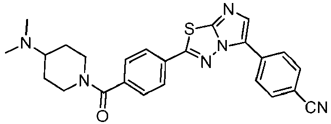 | 40 | 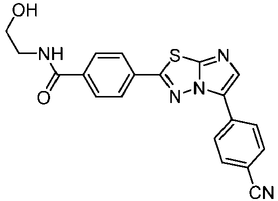 |
| 13 | 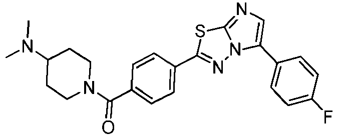 | 41 | 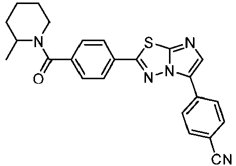 |
| 14 | 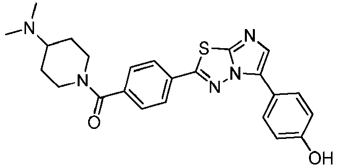 | 42 | 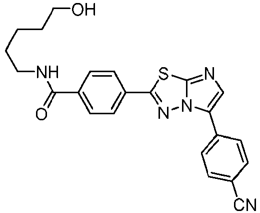 |
| 15 | 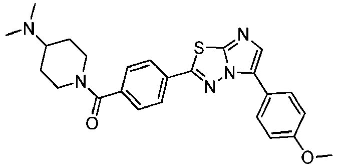 | 43 | 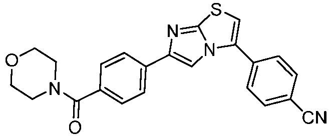 |
| 16 | 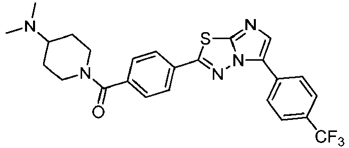 | 44 | 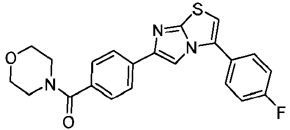 |
| 17 | 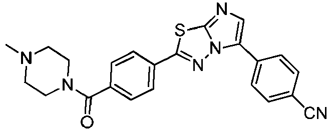 | 45 | 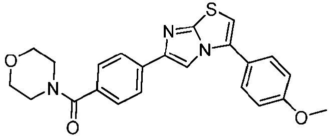 |
| 18 | 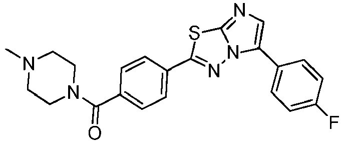 | 46 | 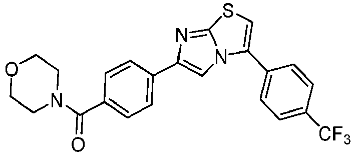 |
| 19 | 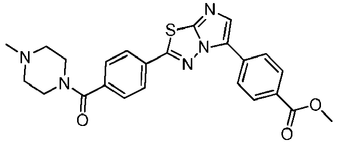 | 47 | 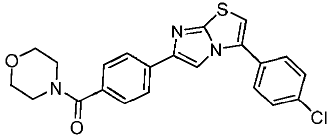 |
| 20 | 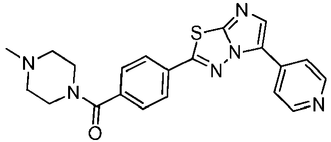 | 48 | 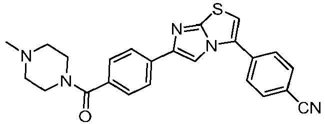 |
| 21 | 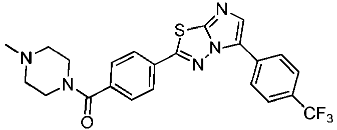 | 49 | 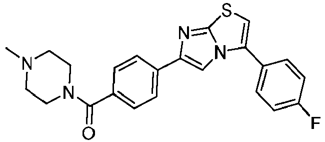 |
| 22 | 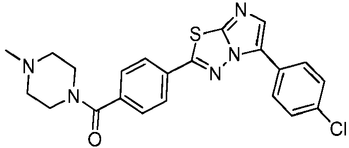 | 50 | 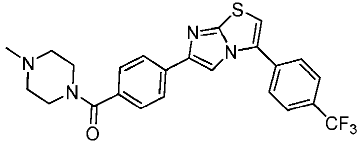 |
| 23 | 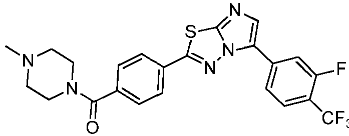 | 51 | 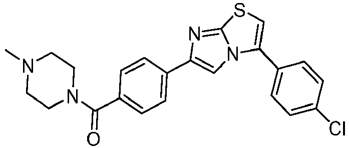 |
| 24 | 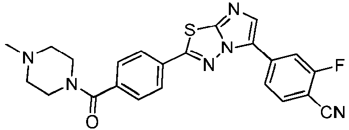 | 52 | 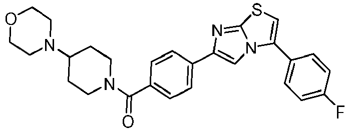 |
| 25 | 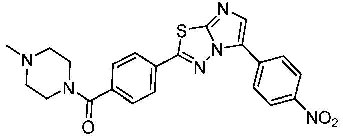 | 53 | 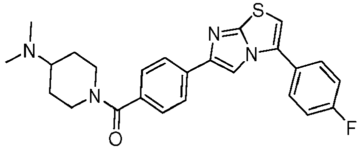 |
| 26 | 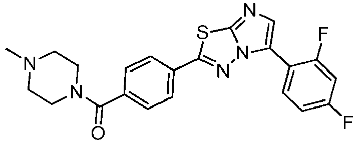 | 54 | 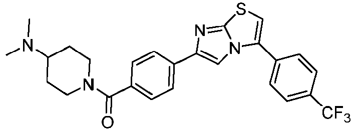 |
| 27 | 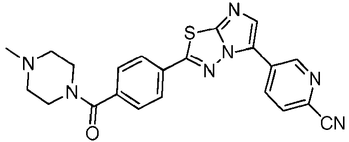 | 55 | 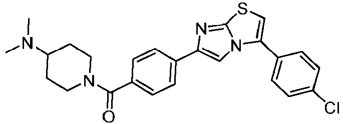 |
| 28 | 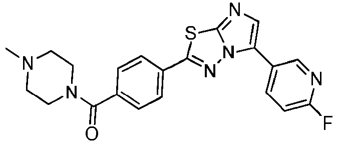 | | |
ou le stéréoisomère, le tautomère ou l'isomère géométrique de celui-ci, ou le sel pharmaceutiquement acceptable de celui-ci.

6. Procédé de préparation du dérivé d'imidazothiazole selon l'une quelconque des revendications 1 à 5, dans lequel le procédé de préparation est un procédé de synthèse de la formule générale 1 ou un procédé de synthèse de la formule générale 2 ; dans lequel
le procédé de synthèse de la formule générale 1 comprend une étape consistant à : 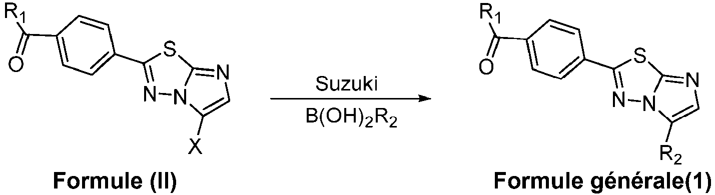
faire subir à un composé de formule (II) une réaction de condensation de Suzuki avec le dérivé d'acide borique (B(OH)2R2) correspondant, et obtenir ainsi un composé de formule générale 1, dans lequel les définitions de R₁ et R₂ sont les mêmes que dans l'une quelconque des revendications 1 à 5, X est un halogène, de préférence X est le chlore, le brome ou l'iode ;
et, le procédé de synthèse de la formule générale 2 comprend une étape consistant à : 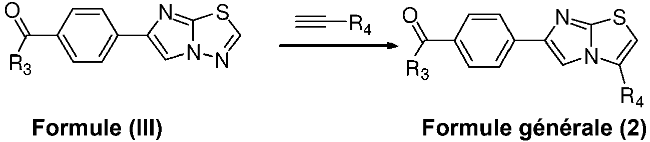
faire réagir un composé de formule (III) avec un dérivé d'acétylène ( 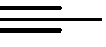 R₄ ) correspondant en condition alcaline, et obtenir ainsi le composé de formule générale 2, dans lequel les définitions de R₃ et R₄ sont les mêmes que dans l'une quelconque des revendications 1 à 5.

7. Procédé de préparation selon la revendication 6, dans lequel :
le procédé de synthèse de la formule générale 1 comprend en outre une étape de préparation d'un composé de formule (II) à partir d'un composé de formule (IV) : 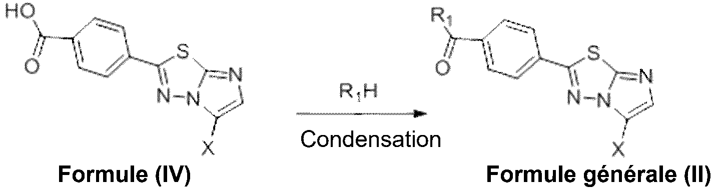
le procédé de synthèse de la formule générale 2 comprend en outre une étape de préparation d'un composé de formule (III) à partir d'un composé de formule (V) :

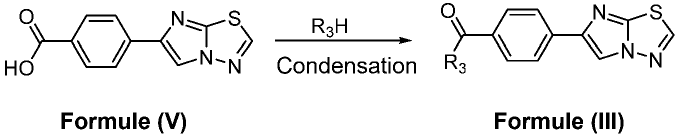

8. Intermédiaire pour la préparation du dérivé d'imidazothiazole selon l'une quelconque des revendications 1 à 5, dans lequel l'intermédiaire a une structure de formule (II), de formule (III), de formule (IV) ou de formule (V) : 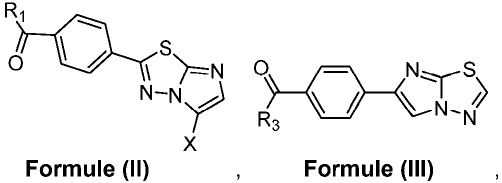 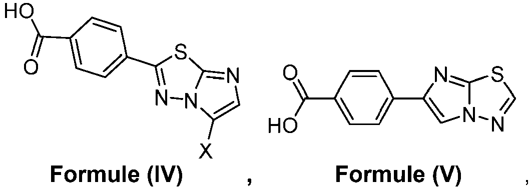
dans lequel les définitions de R₁ et R₃ sont les mêmes que dans la revendication 1, et X est un halogène ;
de préférence,
l'intermédiaire est choisi dans le groupe constitué par :

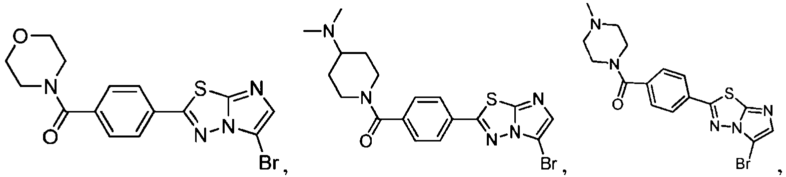

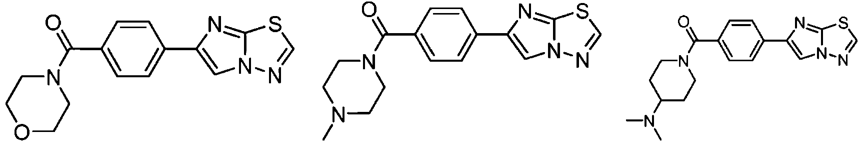

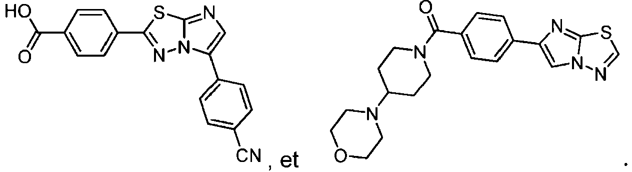

9. Intermédiaire selon la revendication 8
pour une utilisation dans la préparation du
dérivé d'imidazothiazole selon l'une quelconque des revendications 1 à 5.

10. Dérivé d'imidazothiazole selon l'une quelconque des revendications 1 à 5, ou le stéréoisomère, le tautomère, l'isomère géométrique de celui-ci, ou le sel pharmaceutiquement acceptable de celui-ci pour une utilisation visant à :
(a) inhiber l'activité kinase de MNK1 ou MNK2 ou une variante de ceux-ci ; et/ou
(b) prévenir et/ou traiter les maladies métaboliques associées à l'activité des MNK ; et/ou
(c) prévenir et/ou traiter les cancers causés par des niveaux anormaux de MNK1 et/ou MNK2, et/ou
(d) inhiber l'activité kinase de MNK1 et/ou MNK2.

11. Composé pour une utilisation selon la revendication 10, dans lequel la maladie métabolique associée à l'activité des MNK est choisie dans le groupe constitué par le diabète sucré de type 1, le diabète sucré de type 2, l'hyperlipémie, l'obésité et la stéatose hépatique, ou des complications de ceux-ci ou des troubles qui y sont liés.

12. Composé pour une utilisation selon la revendication 11, dans lequel
le diabète sucré et les complications de celui-ci sont choisis dans le groupe constitué par l'intolérance au glucose, la gangrène diabétique, l'arthropathie diabétique, l'ostéopénie diabétique, la glomérulosclérose diabétique, la néphropathie diabétique, la dermatopathie diabétique, la neuropathie diabétique, la cataracte diabétique, la rétinopathie diabétique, la dégénérescence maculaire diabétique, le syndrome du pied diabétique, le coma diabétique, le coma hyperosmolaire diabétique, le coma hypoglycémique, le coma hyperglycémique, l'acidose diabétique, l'acidocétose diabétique, la néphropathie glomérulaire intracapillaire, l'atrophie musculaire diabétique, la neuropathie autonome diabétique, la mononeuropathie diabétique, la polyneuropathie diabétique, la maladie vasculaire diabétique, la maladie vasculaire périphérique diabétique, l'ulcère diabétique, l'arthropathie diabétique et l'obésité diabétique ;
l'hyperlipidémie et les complications de celle-ci sont choisies dans le groupe constitué par l'hypercholestérolémie, l'hypercholestérolémie familiale, l'hyperlipoprotéinémie de Verde, l'hyperβ-lipoprotéinémie, l'hyperlipidémie, la lipoprotéinémie de faible densité, l'hypertriglycéridémie pure, l'hypertriglycéridémie endogène, l'hypercholestérolémie simple, l'hypertriglycéridémie simple, les maladies cardio-vasculaires, dans lequel les maladies cardiovasculaires comprennent : l'hypertension, l'ischémie, les varices, l'occlusion de la veine rétinienne, l'athérosclérose, l'angine de poitrine, l'infarctus du myocarde, la sténocardie, l'hypertension pulmonaire, l'insuffisance cardiaque congestive, la maladie glomérulaire, les troubles tubulaires interstitiels du rein, l'insuffisance rénale, la sténose vasculaire et les maladies cérébrovasculaires (accidents vasculaires cérébraux) ; et
la stéatose hépatique est choisie parmi la stéatose hépatique non alcoolique (NAFLD) ou la stéatohépatite non alcoolique (NASH), et l'inflammation chronique, la fibrose progressive ou la cirrhose qui en résultent.

13. Composition pharmaceutique comprenant le dérivé d'imidazothiazole selon l'une quelconque des revendications 1 à 5, ou le stéréoisomère, le tautomère ou l'isomère géométrique de celui-ci, ou le sel pharmaceutiquement acceptable de celui-ci en tant qu'ingrédient actif ; de préférence, la composition pharmaceutique comprend en outre des excipients pharmaceutiquement acceptables.

14. Composition pharmaceutique selon la revendication 13, dans laquelle la composition pharmaceutique comprend en outre un autre inhibiteur de **MNK1** et/ou MNK2.

15. Composition pharmaceutique selon l'une quelconque des revendications 13 et 14, dans laquelle la forme de dosage de la composition pharmaceutique est une préparation solide, une préparation liquide ou une préparation semi-solide, de préférence, la forme de dosage de la composition pharmaceutique est un comprimé, une capsule ou une injection.
